# EUROPEAN PATENT APPLICATION

(11) **EP 3 483 266 A1**
(43) Date of publication of application: **15.05.2019**
(21) Application number: 17827146.6
(22) Date of filing: 26.01.2017
(51) Int. Cl.: C12N 15/09, C12Q 1/68, G01N 33/53, G01N 33/569

(54) **INTRAORAL EXAMINATION METHOD**

(30) Priority: 11.07.2016 JP 2016136579
(71) Applicant: Mitsubishi Chemical Corporation, Tokyo 100-8251 (JP)
(72) Inventor: HARA Ai, Tokyo 100-8251 (JP); MURAKAMI Shinya, Suita-shi Osaka 565-0871 (JP); NOZAKI Takenori, Suita-shi Osaka 565-0871 (JP)
(74) Representative: TBK
(86) International application number: PCT/JP2017/002784
(87) International publication number: WO 2018/012011

(57) **Abstract**

Provided are a method with which it is possible to assess the state of periodontal disease and caries by a simple method, a method with which it is possible to assess the severity of periodontal disease, therapeutic effect, and risk of exacerbation by detection of intraoral bacteria, and the like. The present invention is an intraoral examination method that, for example, assesses the state of periodontal disease and/or caries by measuring the levels of bacteria present in an intraoral sample from an intraoral sample collected from a subject.

## Description

### FIELD OF THE INVENTION

The present invention relates to an intraoral examination method for assessing the status of periodontal disease and/or dental caries, and the like.

### BACKGROUND ART

Periodontal disease and dental caries, the two major oral diseases, are infectious bacterial diseases involving multiple bacteria.

While periodontal disease is a progressive multifactorial disease which involves causative bacteria (bacterial factors), immune (host factors) and lifestyle habits, its onset is always associated with periodontal disease-causing bacteria.

As such periodontal disease-causing bacteria, for example, *Porphyromonas gingivalis, Tannerella forsythensis, Treponema denticola, Campylobacter rectus, Fusobacterium nucleatum, Prevotella intermedia, Aggregatibacter actinomycetemcomitans* and the like have been reported (Non-patent documents 1 and 2). Among which, three bacteria, *Porphyromonas gingivalis, Tannerella forsythensis* and *Treponema denticola,* are called "Red Complex" and regarded crucial as causative bacteria of chronic periodontitis. The presence of "Red Complex" is known to increase the malignancy of the periodontal disease, and thus the bacteria categorized as "Red Complex" are considered to be clinically crucial bacteria.

In addition, *Aggregatibacter actinomycetemcomitans* has been reported as a causative bacterium of invasive periodontitis, and *Prevotella intermedia* has been reported as a causative bacterium of juvenile and gestational periodontitis (Non-patent document 1 and 2).

As a bacterial examination for periodontal disease, relevance between the sum of the three kinds of "Red Complex" bacteria in plaque or saliva and the status (progression) of periodontal disease has been reported. Specifically, it is assessed to be "low" when the sum of the bacterial count of at least one of *Porphyromonas gingivalis, Tannerella forsythensis* and *Treponema denticola* is less than 0.5% to the total bacterial count, "moderate" if it is 0.5% or more but less than 5% to the total bacterial count, and "high" if it is 0.5% or more to the total bacterial count (Non-patent document 3).

*Streptococcus mutans* is known as a causative bacterium of dental caries. This causative bacterium is known to metabolize sugars to produce lactic acid, as a result of which oral environment becomes acidic and enamel decalcification occurs.

For a bacterial examination for dental caries, a kit is commercially available which examines by culturing *Mutans streptococci* and *Lactobacillus,* and is utilized as one of the materials for diagnosis. The resulting cultures are visually observed for assessment by the approximate colony counts.

According to culture assessment, the colony counts are classified as "Class 0" when less than 100,000 CFU/mL, "Class 1" when 100,000 CFU/mL or higher but less than 500,000 CFU/mL, "Class 2" when 500,000 CFU/mL or higher but less than 1,000,000 CFU/mL, and "Class 3" when 1,000,000 CFU/mL or higher.

Examples of the bacterial examination method for the causative bacteria of periodontal disease or dental caries include observation with a microscope, a culture method, an enzymatic activity method, an immunological technique, a DNA probe method, a PCR method and a real-time PCR method.

Numbers of methods for determining a bacterial count in relation to either of periodontal disease or dental caries have been reported. For example, in one exemplary method, a bacterial count of *Porphyromonas gingivalis* and/or *Bacteroides forsythus* in the saliva is calculated by real-time PCR method (Patent document 1). In another exemplary method, a ratio of the bacterial count of *Mutans streptococci* to the bacterial count of the total *streptococci* is determined by real-time PCR method (Patent document 2).

Meanwhile, as the method for counting the number of periodontal disease-causing bacteria, for example, methods employing a culture method, real-time PCR, a next-generation sequencer or a DNA microarray have been reported. More specifically, there are reports of using real-time PCR method to individually detect the bacterial counts of *Porphyromonas gingivalis* and/or *Bacteroides forsythus* in saliva (Patent documents 3 and 4).

T-RFLP technique is also reported in which genomic DNA of bacterial flora is collected and treated with a restriction enzyme to identify the bacterial flora from the information of the fragmented DNA using similarity of the pattern as an index (Patent document 5). According to this report, a bacterial flora-derived pattern correlated with a dental clinical index was specified. However, it does not contain specific information of the counts of the individual bacteria, and thus no further understanding was reached. Although T-RFLP technique is capable of expressing a bacterial community structure as peak patterns and capable of easily analyzing multiple specimens through comparison, it has difficulty in understanding the bacterial community structure since each peak does not necessarily originates from a single bacterial species (Non-patent document 4).

Moreover, as a method for detecting a total bacterial count, there are reports of utilizing universal primers selected for a region that is highly conserved among different microbacteria (Patent documents 6-8).

In a case of a DNA microarray, there is a report that 20 kinds of intraoral bacteria were detected when a set of universal primers were set in a PCR step for preparing a specimen (Non-patent documents 5-8).

So far, there have also been cases where the number of at least one of the bacteria of "Red Complex" was counted and used as an index of severity of periodontal disease. Since periodontal disease, however, is a disease caused by multiple bacteria, determination of limited bacteria is not only insufficient as an evidence but also the severity of periodontal disease or the causative bacteria may be overlooked.

Furthermore, the index for judging the effect of treating periodontal disease has fundamentally been dependent on clinical information acquired by dentist's experiences, and in most cases, removal of the bacteria has not been confirmed.

Moreover, there has been no index for exacerbation of periodontal disease at an early stage of periodontal disease. Therefore, periodontal disease is already progressing when many patients notice that they have periodontal disease, and a treatment only after noticing the symptoms of periodontal disease often results in no therapeutic effect. Thus, there is also a need for a method for effectively predicting exacerbation of periodontal disease.

### PRIOR ART DOCUMENTS

### Patent documents

Patent document 1: Japanese Patent No. 4252812
Patent document 2: Japanese Unexamined Patent Application Publication No. 2008-206516
Patent document 3: Japanese Unexamined Patent Application Publication No. 2004-229537
Patent document 4: International Patent Application Publication No. WO2002/010444
Patent document 5: Japanese Unexamined Patent Application Publication No. 2011-193810
Patent document 6: International Patent Application Publication No. WO03/106676
Patent document 7: Japanese Unexamined Patent Application Publication (translation of PCT) No. 2004-504069
Patent document 8: Japanese Unexamined Patent Application Publication No. 2007-068431

### Non-patent documents

Non-patent document 1: Socransky, S.S. et al. J Clin Microbiol, 37, 1426-30, 1999
Non-patent document 2: Concept of periodontal treatment utilizing bacterial examination, written and edited by Masato MINABE, Toshiaki YOSHINO, p.3, published June 2005, Igaku Joho-sha
Non-patent document 3: Medical guideline for antimicrobial therapy for patients with periodontal disease, Japanese Society of Periodontology
Non-patent document 4: Human health and disease controlled by indigenous bacterial flora: Hiroshi OHNO, Shohei HATTORI, p.97, published March 2014, Yodosha
Non-patent document 5: Eberhard, J. et al. Oral Microbiol Immunol 2008; 23: 21-8
Non-patent document 6: Topcuoglu, N. et al. J Clin Pediatr Dent 2013; 38: 155-60
Non-patent document 7: Topcuoglu, N. et al. Anaerobe 2015; 35: 35-40
Non-patent document 8: Henne, K. et al. J Oral Microbiol 2014; 6: 25874

### SUMMARY OF INVENTION

### Problem to be Solved by Invention

Thus, the present invention has an objective of providing a method that is capable of assessing the status of periodontal disease and/or dental caries by a simple method.

More particularly, the present invention has an objective of providing a method for assessing the severity of periodontal disease, the effect of treating periodontal disease or the risk of exacerbation of periodontal disease by specifically detecting the intraoral bacteria.

### Means for Solving Problem

The present inventors have gone through extensive investigation to solve the above-described problems, and as a result of which found that the status of periodontal disease and/or dental caries can be assessed by determining a proportion of a specific bacterial count with respect to the total bacterial count of bacteria present in an intraoral sample, thereby accomplishing the present invention. In addition, the present inventors also found that the severity of periodontal disease, the effect of treating periodontal disease, the risk of exacerbation of periodontal disease and the like can be assessed by counting specific bacteria present in an intraoral sample, thereby accomplishing the present invention.

Thus, the present invention is as follows.
(1) An intraoral examination method, comprising measuring levels of bacteria present in an intraoral sample collected from a subject to assess the status of periodontal disease and/or dental caries.
(2) The method according to (1) above, comprising determining a proportion of a bacterial count of a specific bacterium with respect to the total count of the bacteria present in the intraoral sample to assess the status of periodontal disease and/or dental caries.
(3) The method according to (2) above, which uses saliva as the intraoral sample, wherein if the sum of bacterial counts of at least one bacterium selected from the group consisting of *Porphyromonas gingivalis, Tannerella forsythensis* and *Treponema denticola* among the bacteria present in the saliva is:
   less than 0.01% to the total bacterial count, then the symptom of periodontal disease is assessed to be "mild";
   0.01% or more but less than 0.5% to the total bacterial count, then the symptom of periodontal disease is assessed to be "moderate"; and
   0.5% or more to the total bacterial count, then the symptom of periodontal disease is assessed to be "severe".
(4) The method according to (2) above, which uses saliva as the intraoral sample, wherein if the bacterial count of *Streptococcus mutans* among the bacteria present in the saliva is:
   less than 0.05% to the total bacterial count, then the symptom of dental caries is assessed to be "mild";
   0.05% or more but less than 2.5% to the total bacterial count, then the symptom of dental caries is assessed to be "moderate"; and
   2.5% or more to the total bacterial count, then the symptom of dental caries is assessed to be "severe".
(5) The method according to (2) above, which uses plaque as the intraoral sample, wherein if the sum of bacterial counts of at least one bacterium selected from the group consisting of *Porphyromonas gingivalis, Tannerella forsythensis* and *Treponema denticola* among the bacteria present in the plaque is:
   less than 0.1% to the total bacterial count, then the symptom of periodontal disease is assessed to be "mild";
   0.1% or more but less than 5% to the total bacterial count, then the symptom of periodontal disease is assessed to be "moderate"; and
   5% or more to the total bacterial count, then the symptom of periodontal disease is assessed to be "severe".
(6) The method according to (1) above, comprising the steps of:
   measuring a bacterial level of a specific bacterium in the intraoral sample collected from the subject, a total level of the bacteria present in the intraoral sample, or both of a bacterial level of a specific bacterium and a total level of the bacteria present in the intraoral sample; and
   statistically analyzing the acquired measurement results to assess the severity of periodontal disease.
(7) The method according to (1) above, comprising the steps of:
   measuring a bacterial level of a specific bacterium in the intraoral sample collected from the subject, a total level of the bacteria present in the intraoral sample, or both of a bacterial level of a specific bacterium and a total level of the bacteria present in the intraoral sample, before and after a treatment of periodontal disease; and
   assessing the effect of treating periodontal disease based on the results from comparison between the bacterial level before the treatment and the bacterial level after the treatment.
(8) The method according to (1) above, comprising the steps of:
   measuring a bacterial level of a specific bacterium in the intraoral sample collected from the subject, a total level of the bacteria present in the intraoral sample, or both of a bacterial level of a specific bacterium and a total level of the bacteria present in the intraoral sample; and
   assessing the risk of exacerbation of periodontal disease based on the ratio of the acquired bacterial levels.
(9) The method according to either one of (6) and (7) above, wherein the specific bacterium in the intraoral sample is at least one selected from the group consisting of bacteria belonging to genus *Porphyromonas,* genus *Tannerella,* genus *Treponema,* genus *Prevotella,* genus *Campylobacter,* genus *Fusobacterium,* genus *Parvimonas,* genus *Streptococcus,* genus *Aggregatibacter,* genus *Capnocytophaga,* genus *Eikenella,* genus *Actinomyces,* genus *Veillonella,* genus *Selenomonas,* genus *Lactobacillus,* genus *Pseudomonas,* genus *Haemophilus,* genus *Klebsiella,* genus *Serratia,* genus *Moraxella* and genus *Candida.*
(10) The method according to either one of (6) and (7) above, wherein the specific bacterium in the intraoral sample is at least one selected from the group consisting of *Porphyromonas gingivalis, Tannerella forsythia, Treponema denticola, Prevotella intermedia, Aggregatibacter actinomycetemcomitans, Fusobacterium nucleatum* subsp. *vincentii, Fusobacterium nucleatum* subsp. *polymorphum, Fusobacterium nucleatum* subsp. *animalis, Fusobacterium nucleatum* subsp. *nucleatum, Streptococcus sanguis, Streptococcus mitis, Actinomyces viscosus, Streptococcus aureus, Pseudomonas aeruginosa, Streptococcus pyogenes, Streptococcus pneumoniae, Campylobacter gracilis, Campylobacter rectus, Campylobacter showae, Fusobacterium periodonticum, Parvimonas micra, Prevotella nigrescens, Streptococcus constellatus, Campylobacter concisus, Capnocytophaga gingivalis, Capnocytophaga ochracea, Capnocytophaga sputigena, Eikenella corrodens, Streptococcus gordonii, Streptococcus intermedius, Streptococcus mitis* bv. 2, *Actinomyces odontolyticus, Veillonella parvula, Actinomyces naeslundii II* and *Selenomonas noxia.*
(11) The method according to either one of (6) and (7) above, wherein the specific bacterium in the intraoral sample is at least one selected from the group consisting of *Porphyromonas gingivalis, Tannerella forsythia, Treponema denticola, Fusobacterium nucleatum, Campylobacter rectus, Streptococcus constellatus, Streptococcus gordonii, Streptococcus mitis, Streptococcus oralis, Streptococcus sanguinis* and *Veillonella parvula.*
(12) The method according to (8) above, wherein the specific bacterium in the intraoral sample is at least one selected from the group consisting of *Porphyromonas gingivalis, Tannerella forsythia* and *Treponema denticola,* and *Fusobacterium nucleatum.*
(13) The method according to (1) above, wherein the count of bacterium present in the intraoral sample is determined by a method of calculating the bacterial count which comprises the steps (1)-(3) below:
   (1) comparing data acquired from a test sample with a signal intensity of an absolute level index probe to correct the data;
   (2) for each of pre-isolated bacteria targeted for detection, determining a bacterial count calculation formula from a signal intensity ratio of a probe for detecting the bacterium targeted for detection; and
   (3) by using the calculation formula obtained in step (2) above, determining a bacterial count of each bacterial species targeted for detection from the signal intensity corrected in step (1) above.
(14) A primer or a primer set comprising one or more of DNAs having the nucleotide sequences represented by SEQ ID NOS:193-198 and 201-204 or nucleotide sequences complementary to said nucleotide sequences.
(15) An oligonucleotide probe comprising:
   DNA having the nucleotide sequence represented by SEQ ID NO:97, 100, 120, 121, 122, 129, 130, 152 or 155 or a nucleotide sequence complementary to said nucleotide sequence; or
   DNA that hybridizes with DNA having a nucleotide sequence complementary to said DNA under stringent conditions, and that has a function of detecting at least a part of a nucleotide sequence of nucleic acids derived from an intraoral bacterium.

### EFFECT OF THE INVENTION

The present invention is capable of assessing the status of periodontal disease and/or dental caries by a simple method.

In addition, the present invention is capable of assessing the severity of periodontal disease, the effect of treating periodontal disease, and the risk of exacerbation of periodontal disease by a simple method by detecting intraoral bacteria. More particularly, the present invention can carry out said assessments with higher accuracy than conventional culture methods, and with better work efficiency and less expensive than real-time PCR and a sequencer.

### BRIEF DESCRIPTION OF THE DRAWINGS

[Figure 1] A diagram showing results of cluster classification.
[Figure 2] A diagram showing results of cluster classification.
[Figure 3] A diagram showing a heatmap.

### MODE FOR CARRYING OUT THE INVENTION

Hereinafter, the present invention will be described in detail. The scope of the present invention is not limited to these descriptions and, besides the following examples, they can appropriately be modified and carried out without departing from the spirit of the present invention. This specification incorporates the entire content of the specification of Japanese Patent Application No. 2016-136579 (filed on July 11, 2016) based on which the present application claims priority. Furthermore, all of the publications cited herein such as prior art documents and publications, patent publications and other patent documents are incorporated herein by reference.

As described above, an intraoral examination method of the present invention is a method for assessing the status of periodontal disease and/or dental caries by measuring the levels of bacteria present in an intraoral sample.

Examples of specific aspects of such an intraoral examination method include, but not limited to:
A) an assessment method comprising determining a proportion of a bacterial count of a specific bacterium with respect to the total bacterial count of bacteria present in the intraoral sample and assessing the above; and B) an assessment method comprising measuring the levels of bacteria in an intraoral sample collected from a subject and assessing the above based on the acquired measurement results.

Hereinafter, Methods A) and B) will be described, respectively.

### I. Regarding Method A) above

While the method for counting the bacteria in the intraoral sample herein will be described mainly as a method using a DNA chip, a method other than the method using a DNA chip, for example, an invader assay, a real-time PCR method, an invader PCR assay or the like may also be employed for counting the bacteria in the intraoral sample.

### 1. Oligonucleotide probe used for measuring intraoral bacterial levels

According to the method of the present invention, a DNA chip can be used for measuring an intraoral bacterial level of an intraoral sample collected from a subject, where said DNA chip may, for example, be equipped with following probes (a) and at least one of probes (b) and (c). In general, a DNA chip is a generic term for a substrate arranged with probes. Herein, the terms DNA chip, DNA microarray and the like are not distinguished and are taken as synonyms.
(a) Probes of nucleic acids that specifically hybridize with genes of respective bacteria targeted for detection.
(b) Total level index probe of nucleic acids that hybridize with the genes of all of the bacteria.
(c) Probes of nucleic acids that specifically hybridize with one or more respective kinds of absolute level indices.

### (1) Intraoral bacteria targeted for measurement

In an examination method of the present invention, intraoral bacteria targeted for measurement, namely, bacterial species targeted for detection, may be, but not limited to, bacteria belonging to genus *Porphyromonas,* bacteria belonging to genus *Tannerella,* bacteria belonging to genus *Treponema,* bacteria belonging to genus *Prevotella,* bacteria belonging to genus *Campylobacter,* bacteria belonging to genus *Fusobacterium,* bacteria belonging to genus *Parvimonas,* bacteria belonging to genus *Streptococcus,* bacteria belonging to genus *Aggregatibacter,* bacteria belonging to genus *Capnocytophaga,* bacteria belonging to genus *Eikenella,* bacteria belonging to genus *Actinomyces,* bacteria belonging to genus *Veillonella,* bacteria belonging to genus *Selenomonas,* bacteria belonging to genus *Lactobacillus,* bacteria belonging to genus *Pseudomonas,* bacteria belonging to genus *Haemophilus,* bacteria belonging to genus *Klebsiella,* bacteria belonging to genus *Serratia,* bacteria belonging to genus *Moraxella,* and bacteria belonging to genus *Candida.*

More specifically, bacterial species targeted for detection are, for example, preferably at least one of *Porphyromonas gingivalis, Tannerella forsythia, Treponema denticola, Prevotella intermedia, Aggregatibacter actinomycetemcomitans, Fusobacterium nucleatum* subsp. *vincentii, Fusobacterium nucleatum* subsp. *polymorphum, Fusobacterium nucleatum* subsp. *animalis, Fusobacterium nucleatum* subsp. *nucleatum, Streptococcus sanguis, Streptococcus mitis, Actinomyces viscosus, Streptococcus aureus, Pseudomonas aeruginosa, Streptococcus pyogenes, Streptococcus pneumoniae, Campylobacter gracilis, Campylobacter rectus, Campylobacter showae, Fusobacterium periodonticum, Parvimonas micra, Prevotella nigrescens, Streptococcus constellatus, Campylobacter concisus, Capnocytophaga gingivalis, Capnocytophaga ochracea, Capnocytophaga sputigena, Eikenella corrodens, Streptococcus gordonii, Streptococcus intermedius, Streptococcus mitis* bv. 2, *Actinomyces odontolyticus, Veillonella parvula, Actinomyces naeslundii* II, *Selenomonas noxia, Streptococcus mutans* and the like which are currently considered to be relevant to periodontal disease and dental caries, and more preferably *Porphyromonas gingivalis, Tannerella forsythia, Treponema denticola* and *Streptococcus mutans.*

### (2) About probe (a)

According to the present invention, oligonucleotide DNA that may be used as the probe (a) is one that can hybridize with a nucleotide sequence of a specific region in a nucleotide sequence of nucleic acids derived from an intraoral bacterium. While said nucleic acids may be, without limitation, either DNA (including chromosomal DNA, plasmid DNA or the like) or RNA, it is preferably chromosomal DNA. Specifically, an oligonucleotide used as a probe of the present invention is one that can hybridize with a nucleotide sequence of 16S rRNA gene in the chromosomal DNA of the intraoral bacterium.

Probes that can be used for the present invention are preferably obtained by selecting regions that can serve as nucleotide sequences specific to the above-described respective intraoral bacteria to be detected, and designing nucleotide sequences of said regions. Generally, probe design requires, in addition to selection of specific regions, matching of melting temperatures (Tm) and minimization of formation of a secondary structure.

A nucleotide sequences that is specific to each species of intraoral bacteria may be found, for example, by providing a multiple alignment to design a probe located in a region unique to that species. More specifically, while the algorithm for providing the alignment is not particularly limited, a program such as ClustalX1.8 may be utilized as an analysis program. While default status can be employed to run the program, the parameters used for providing the alignment may suitably be adjusted according to the type of the program or the like.

Meanwhile, the specificity of the probe may be such that it allows collective detection of the bacteria belonging to the same genus based on specificity at a genus level, or such that it allows detection based on specificity at an individual species level, which can suitably be determined according to the purpose of the bacteria detection.

### (3) About probe (b)

The total level index probe is a probe intended to capture all of the bacteria in the specimen that were amplified with specific primer pairs. With respect to bacteria detection, it is crucial to detect the total bacterial level from the viewpoint of how much the bacteria targeted for detection exist among the entire bacteria including bacteria not targeted for detection, and also from the viewpoint of how much level of bacteria is present in the specimen in the first place.

The bacteria not targeted for detection is understood as a sum (total) of bacteria whose presence and kinds are known but not targeted for detection, and bacteria whose presence and kinds are unknown.

For the detection of the total bacterial level, for example, the total bacterial level may be measured independently from the DNA chip, but convenience of handling would be enhanced by providing a probe that can serve as an index of the total bacterial level on the DNA chip. The probe used may be a nucleotide sequence that is common to the various kinds of bacterial species among the nucleotide sequences amplified by the primer pairs. If no such sequence is found, a plurality of relatively common sequences may be designed so that they can be used for comprehensive judgement to find a total level index probe. The total level index probe is preferably a probe that hybridizes with nucleic acids derived from the bacteria contained in the specimen, specifically, a probe that contains a nucleotide sequence commonly included by the multiple kinds of bacteria targeted for detection among the nucleotide sequences amplified by the above-described specific primer pairs. An example of the total level index probe is shown in Table 1-1-1 (SEQ ID NO:60).

The total level index is generally high since it represents the total level of the amplified products specific to the individual bacterial species, and thus the signal intensity of interest may exceed the detectable signal intensity range.

In order to avoid such circumstance, the amount of the specimen subjected to hybridization is preferably limited. Alternatively, the Tm value of the probe may be lowered, for example, upon designing the probe. Specifically, a technique such as decreasing the GC content or shortening the length of the probe sequence itself may be contemplated.

Furthermore, nucleic acids that can competitively act against the hybridization between the amplified nucleic acids and the total level index probe may be added upon hybridization to reduce the signal intensity. Examples of such nucleic acids include nucleic acids having a sequence that is entirely or partially identical to the total level index probe, and nucleic acids having a sequence that is entirely or partially complementary to the sequence of the total level index probe.

### (4) About probe (c)

An absolute level index probe refers to a probe that hybridizes solely to the nucleic acids of the absolute level index.

Herein, an absolute level index refers to nucleic acids that are added to the specimen for a certain amount prior to amplification reaction and hybridization reaction. The absolute level index is nucleic acids that ensure actual amplification reaction upon usual amplification reaction, and plays a role as a so-called positive control.

Therefore, the DNA chip may be provided with a probe specific to the absolute level index so that whether or not amplification reaction, hybridization and the like have appropriately taken place can be confirmed by detecting said probe. Moreover, if one type of absolute level index is set, the signal intensity of said absolute level index acquired from multiple DNA chips should be constant, and thus the signal intensities of the absolute level index may be compared to calculate correction coefficients if there are some variations in the amplification efficiency or the hybridization efficiency. The corrected signal intensities can be used upon comparison among the multiple DNA chips.

Examples of probes specific to respective bacteria will be shown in Table 1-1-1 (SEQ ID NOS:3-59). In addition, examples of the absolute level index probes will be shown in Table 1-1-1 (SEQ ID NOS:61-75) while examples of absolute level indices will be shown in Table 1-1-2 (SEQ ID NOS:76-90).

If the absolute level index is added prior to amplification reaction, it should be nucleic acids that can be amplified with a specific primer pair, in other words, it should possess a nucleotide sequence complementary to the primer pair, and it should possess a nucleotide sequence that is not possessed by any of the bacteria targeted for detection or the bacteria not targeted for detection in order to be detected through hybridization.

**[Table 1-1-1]**

| SEQ ID NO | Role | Sequence (5' to 3') |
|---|---|---|
| 3 | *Porphyromonas gingivalis* (P.g.) probe | TTCAATGCAATACTCGTATC |
| 4 | *Porphyromonas gingivalis* (P.g.) probe | GTACATTCAATGCAATACTC |
| 5 | *Tannerella forsythia* (T.f.) probe | CACGTATCTCATTTTATTCC |
| 6 | *Tannerella forsythia* (T.f.) probe | AATACACGTATCTCATTTTATT |
| 7 | *Treponema denticola* (T.d.) probe | CTCTTCTTCTTATTCTTCAT |
| 8 | *Treponema denticola* (T.d.) probe | CCTCTTCTTCTTATTCTTCAT |
| 9 | *Campylobacter gracilis* (C.g.) probe | GCCTTCGCAATAGGTATT |
| 10 | *Campylobacter gracilis* (C.g.) probe | CGCCTTCGCAATAGGTAT |
| 11 | *Campylobacter rectus* (C.r.) probe | ATTCTTTCCCAAGAAAAGGA |
| 12 | *Campylobacter rectus* (C.r.) probe | GTCATAATTCTTTCCCAAGA |
| 13 | *Campylobacter showae* (C.s.) probe | CAATGGGTATTCTTCTTGAT |
| 14 | *Fusobacterium nucleatum* subsp. *vincentii* (F.n.) probe | TAGTTATACAGTTTCCAACG |
| 15 | *Fusobacterium nucleatum* subsp. *vincentii* (F.n.) probe | CTAGTTATACAGTTTCCAAC |
| 16 | *Fusobacterium nucleatum* subsp. *polymorphum* (F.n.) probe | TCCAGTACTCTAGTTACACA |
| 17 | *Fusobacterium nucleatum* subsp. *polymorphum* (F.n.) probe | CCAGTACTCTAGTTACACA |
| 18 | *Fusobacterium nucleatum* subsp. *animalis* (F.n.) probe | TTTCTTTCTTCCCAACTGAA |
| 19 | *Fusobacterium nucleatum* subsp. *animalis* (F.n.) probe | ATTTCTTTCTTCCCAACTGA |
| 20 | *Fusobacterium nucleatum* subsp. *nucleatum* (F.n.) probe | TACATTCCGAAAAACGTCAT |
| 21 | *Fusobacterium nucleatum* subsp. *nucleatum* (F.n.) probe | TTACATTCCGAAAAACGTCA |
| 22 | *Fusobacterium periodonticum* (F.p.) probe | TATGCAGTTTCCAACGCAA |
| 23 | *Fusobacterium periodonticum* (F.p.) probe | CTCTAGTTATGCAGTTTCC |
| 24 | *Parvimonas micra* (P.m.) probe | AAGTGCTTAATGAGGTTAAG |
| 25 | *Parvimonas micra* (P.m.) probe | TTTCAAGTGCTTAATGAGGT |
| 26 | *Prevotella intermedia* (P.i.) probe | GGGTAAATGCAAAAAGGCA |
| 27 | *Prevotella intermedia* (P.i.) probe | GCAAGGTAGATGTTGAGCA |
| 28 | *Prevotella nigrescens* (P.n.) probe | CTTTATTCCCACATAAAAGC |
| 29 | *Prevotella nigrescens* (P.n.) probe | TCCTTATTCATGAGGTACAT |
| 30 | *Streptococcus constellatus* (S.c.) probe | AAGTACCGTCACTGTGTG |
| 31 | *Streptococcus constellatus* (S.c.) probe | TTAAGTACCGTCACTGTGT |
| 32 | *Aggregatibacter actinomycetemcomitans* (A.a.) probe | GTCAATTTGGCATGCTATTA |
| 33 | *Aggregatibacter actinomycetemcomitans* (A.a.) probe | TTTAACGTCAATTTGGCATG |
| 34 | *Campylobacter concisus* (C.c.) probe | CCCAAGCAGTTCTATGGT |
| 35 | *Campylobacter concisus* (C.c.) probe | TCCCAAGCAGTTCTATGG |
| 36 | *Capnocytophaga gingivalis* (C.g.) probe | TACACGTACACCTTATTCTT |
| 37 | *Capnocytophaga gingivalis* (C.g.) probe | CATCAATGTACACGTACAC |
| 38 | *Capnocytophaga ochracea* (C.o.) probe | CATTCAAGACCAACAGTTT |
| 39 | *Capnocytophaga ochracea* (C.o.) probe | CAACCATTCAAGACCAACA |
| 40 | *Capnocytophaga sputigena* (C.s.) probe | GCTTAGTTGAGCTAAGCG |
| 41 | *Capnocytophaga sputigena* (C.s.) probe | TCAAAGGCAGTTGCTTAGT |
| 42 | *Eikenella corrodens* (E.c.) probe | CTAGCTATCCAGTTCAGAA |
| 43 | *Eikenella corrodens* (E.c.) probe | CTCTAGCTATCCAGTTCAG |
| 44 | *Streptococcus gordonii* (S.g.) probe | CACCCGTTCTTCTCTTACA |
| 45 | *Streptococcus gordonii* (S.g.) probe | CACCCGTTCTTCTCTTAC |
| 46 | *Streptococcus intermedius* (S.i.) probe | CAGTATGAACTTTCCATTCT |
| 47 | *Streptococcus intermedius* (S.i.) probe | ACAGTATGAACTTTCCATTCT |
| 48 | *Streptococcus mitis* (S.m.) probe | CCCCTCTTGCACTCAAGT |
| 49 | *Streptococcus mitis* (S.m.) probe | TCTCCCCTCTTGCACTCA |
| 50 | *Streptococcus mitis* bv 2 (S.m.) probe | TCCCCTCTTGCACTCAAGT |
| 51 | *Actinomyces odontolyticus* (A.o.) probe | AAGTCAGCCCGTACCCA |
| 52 | *Actinomyces odontolyticus* (A.o.) probe | CGCACTCAAGTCAGCCC |
| 53 | *Veillonella parvula* (V.p.) probe | CTATTCGCAAGAAGGCCTT |
| 54 | *Veillonella parvula* (V.p.) probe | TATTCGCAAGAAGGCCTT |
| 55 | *Actinomyces naeslundii* II (A.n.) probe | CACAAGGAGCAGGCCTG |
| 56 | *Actinomyces naeslundii* II (A.n.) probe | CCACCCACAAGGAGCAG |
| 57 | *Selenomonas noxia* (S.n.) probe | TTCGCATTAGGCACGTTC |
| 58 | *Selenomonas noxia* (S.n.) probe | CTATTCGCATTAGGCACGT |
| 59 | *Streptococcus mutans* (S.mu.) probe | CACACGTTCTTGACTTAC |
| 60 | Total level index probe | CGTATTACCGCGGCTGCTGGCAC |
| 61 | Absolute level index 1 probe | CGTGCATTGTCGTGTAGGTTCGACCCTAAT |
| 62 | Absolute level index 2 probe | GCAGCTACGTTCATACCTACGCAAGGCATT |
| 63 | Absolute level index 3 probe | GAGGAGATACCGAATCGGTCGACGACATTT |
| 64 | Absolute level index 4 probe | TGTTGCGTGAAGGTCGTGAACGATTGGCAA |
| 65 | Absolute level index 5 probe | CCCCTACTGAGCAAACGTTGCACTAATGGA |
| 66 | Absolute level index 6 probe | AACAACGACCGAGTGCATAGTCACGTACGA |
| 67 | Absolute level index 7 probe | AGGAGCCCTAAGGTATTGGCGAGAAAAGTC |
| 68 | Absolute level index 8 probe | CTGAGTATCCGCATATCTTCCGAGGTTGCA |
| 69 | Absolute level index 9 probe | ACTTAGCTGACCGAAGGACCATAACGCTGT |
| 70 | Absolute level index 10 probe | TGGAAGGGATCCGTAGTCAACCGTTGACTT |
| 71 | Absolute level index 11 probe | CGGATCGACATACGACGCCTACAGAATGTT |
| 72 | Absolute level index 12 probe | TAAACGTCTAGGCGAGACTATGAGTGCTCC |
| 73 | Absolute level index 13 probe | CGTATGGATCGATCCGACGTACCACATTAG |
| 74 | Absolute level index 14 probe | ACTGCGTATGATCGACACGGCTAATCGTAG |
| 75 | Absolute level index 15 probe | CTATTCGACCAGCGATATCACTACGTAGGC |

**[Table 1-1-2]**

| SEQ ID NO | Name | Sequence (5' to 3') |
|---|---|---|
| 76 | Absolute level index 1 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGAATGGTAAGGGTCGTATTAGGGTCGAACCTACACGACAATGCACGTCGAAGCGGTTGCTAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 77 | Absolute level index 2 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGTAGCCGATTGAACTAATGCCTTGCGTAGGTATGAACGTAGCTGCTAGTCGAGGCCTTGTATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 78 | Absolute level index 3 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGTCCTCGATACATACGAAATGTCGTCGACCGATTCGGTATCTCCTCTGGCACCGAAGACAAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 79 | Absolute level index 4 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGTGCTACGCTTTACGCTTGCCAATCGTTCACGACCTTCACGCAACACTTGATCGAACCGAAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 80 | Absolute level index 5 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGACAACAGACGGTACGTCCATTAGTGCAACGTTTGCTCAGTAGGGGGTCTAAGCGTCACTTATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 81 | Absolute level index 6 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGGAGCAGTTTTCTTCTCGTACGTGACTATGCACTCGGTCGTTGTTGGAATACCGGTCGTAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 82 | Absolute level index 7 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGAACGCAATACGGTGGGACTTTTCTCGCCAATACCTTAGGGCTCCTGTGTACCTAAGCGAAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 83 | Absolute level index 8 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGGATGACGGATAAGTTGCAACCTCGGAAGATATGCGGATACTCAGACGTGATATGCGCAGATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 84 | Absolute level index 9 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGGTGTACATCGGATGACAGCGTTATGGTCCTTCGGTCAGCTAAGTAAGTCCGTTTTCCACATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 85 | Absolute level index 10 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGTTCAGTCAACCGGAGAAGTCAACGGTTGACTACGGATCCCTTCCATGTAGAGCTCTCACAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 86 | Absolute level index 11 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGCGATTGTCGCGTTAAACATTCTGTAGGCGTCGTATGTCGATCCGGGACTTCGCTTCATAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 87 | Absolute level index 12 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGTCGTGTGTCGTGAGAGGAGCACTCATAGTCTCGCCTAGACGTTTATGACGAGATATCACGATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 88 | Absolute level index 13 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGTAGCGTGTAACGCACTAATGTGGTACGTCGGATCGATCCATACGCAACTTTGTACCGAGATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 89 | Absolute level index 14 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGAGCAACCATCTTGAGCTACGATTAGCCGTGTCGATCATACGCAGTCCGAGGCATTACTGAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 90 | Absolute level index 15 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGGCCTTCTCCGAATAGCCTACGTAGTGATATCGCTGGTCGAATAGGCGGATTGCTCATAAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |

A specific primer means that the sequence targeted for amplification can be limited, where the primer pair is not necessary a single pair. If necessary, a multiplex technique that uses two or more primer pairs may also be applied. Examples of the primer pairs are shown in Table 1-2. Primer pairs for bacterial amplification (SEQ ID NOS:1 and 2), and a primer pair for absolute level index (SEQ ID NOS:91 and 92) may be used.

**[Table 1-2]**

| SEQ ID NO | Role | Note | Sequence (5' to 3') |
|---|---|---|---|
| 1 | Forward primer (for amplifying bacteria) | Fluorescently labeled at 5' | TCCTACGGGAGGCAGCAGT |
| 2 | Reverse primer (for amplifying bacteria) | | CAGGGTATCTAATCCTGTTTGCTACC |
| 91 | Forward primer (for amplifying absolute level index) | Fluorescently labeled at 5' | GAGAAGCCTACACAAACGTAACGTC |
| 92 | Reverse primer (for amplifying absolute level index) | | CTCTAAAGACCGCTCTATCTCGG |

According to a method for designing a primer of the present invention, first, at least one variable region showing diversity of bacteria targeted for analysis is selected, and then highly conserved universal primer design regions are selected to precede and succeed the selected variable region, thereby designing a primer sequence. While the targeted variable region is not limited, it may be 16S rRNA gene among the genomic sequence, which exists in all bacteria. Either full-length or one or more of the variable regions V1-V9 of 16S rRNA gene may favorably be targeted. More preferably, variable regions V3-V4 are targeted.

In order to assess the coverage of the primer, database based on acquisition of genomic sequences of a wide range of bacteria is utilized. Specifically, examples of such database include RDP, NCBI, KEGG and MGDB.

For example, an input of the designed universal primer sequence into RDP database ProbeMatch will give a list of results that shows the number of complete matches among the total search. The closer the number of complete matches to the total search is, the higher the coverage of the primer is. As to the conditions in this case, *Type* may be selected for Strain while *Isolates* may be selected for Source.

The absolute level index may be a standard reference material characterized for nucleic acids used in a quantitative analysis, which is developed by the National Institute of Advanced Industrial Science and Technology (AIST), or it may be newly designed. If the absolute level index is to be designed, for example, the RANDBETWEEN function of software "EXCEL" (from MICROSOFT) may be used, which can randomly return X number (X is a given number) of integers of 1 to 4. The resulting random integers may be linked in a row to give a X-digit number consisting only of 1 to 4 so as to obtain a large number of sequences having X bases of random ATGC by converting 1 to A, 2 to T, 3 to C and 4 to G.

From these sequences, only sequences that have the sum of G and T equal to the sum of A and T are picked out. The picked sequences are subjected to Blast search against database such as GenBank at NCBI so as to select less homologous sequences among organism-derived nucleic acids. Primer sequences are allowed to flank both ends of the sequence, thereby designing a sequence. Moreover, the designed sequence may suitably be linked for extension or partially removed for shortening.

In order to keep the reaction efficiency upon amplification reaction as constant as possible, the difference between the amplified base length of the bacterium targeted for detection and the amplified base length of the absolute level index is preferably made small. For example, if the amplified product of the bacterium targeted for detection is about 500 bp, the amplified product of the absolute level index is preferably made to be about 300 bp to 1000 bp.

On the other hand, if the amplified chain length is to be confirmed by electrophoresis or the like after the amplification, the amplified product of the absolute level index is designed to have a length that differs from the length of that of the bacterium targeted for detection so that the absolute level index-derived amplified product can be detected at a location separated from the band for the bacterium targeted for detection, thereby confirming success/failure of the amplification reaction prior to hybridization.

Finally, if the concentration of the absolute level index contained in the specimen is too high, competition with the bacteria targeted for detection becomes intense upon the amplification reaction and the bacteria targeted for detection that are supposed to be detected may not be detected. Therefore, there is a need to suitably adjust the concentration according to the application.

In order to design a probe used for the present invention, stringency upon hybridization needs to be considered. The stringency can be made high to a certain level so that even if a similar nucleotide sequence region exists among certain regions of respective nucleic acids of various intraoral bacteria, hybridization can take place by distinguishing other regions. On the other hand, if the nucleotide sequences of said certain region differ from each other, the stringency may be set lower.

As such stringency conditions, for example, highly stringent conditions refer to hybridization under the conditions of 50-60°C while lowly stringent conditions refer to hybridization under the conditions of 30-40°C. Examples of stringent conditions for hybridization include conditions such as "0.24M Tris-HCl/0.24M NaCl/0.05% Tween-20, 40°C", "0.24M Tris-HCl/0.24M NaCl/0.05% Tween-20, 37°C" and "0.24M Tris-HCl/0.24M NaCl/0.05% Tween-20, 30°C". Examples of more stringent conditions include conditions such as "0.24M Tris-HCl/0.24M NaCl/0.05% Tween-20, 50°C", "0.24M Tris-HCl/0.24M NaCl/0.05% Tween-20, 55°C" and "0.06M Tris-HCl/0.06M NaCl/0.05% Tween-20, 60°C". More specifically, in one method, the probe may be added and kept at 50°C for an hour or longer to form a hybrid, which is subsequently washed with 0.24M Tris-HCl/0.24M

NaCl/0.05% Tween-20 at 50°C for 20 minutes for four times, and finally washed with 0.24M Tris-HCl/0.24M NaCl at 50°C for 10 minutes once. Stringent conditions can be set higher by increasing the temperature upon hybridization or washing. In addition to such conditions like salt concentration, temperature and the like of the buffer, those skilled in the art would also be capable of determining other conditions such as probe concentration, probe length, reaction time and the like. For a detailed procedure of the hybridization method, see "Molecular Cloning, A Laboratory Manual 4th ed." (Cold Spring Harbor Press (2012), "Current Protocols in Molecular Biology" (John Wiley & Sons (1987-1997)), and the like.

While the length of the probe used for the present invention is not limited, it is, for example, preferably 10 bases or longer, more preferably 16-50 bases, and still more preferably 18-35 bases. As long as the length of the probe is appropriate (as long as within the above-mentioned range), it can be used for specific detection while suppressing non-specific hybridization (mismatch).

For designing the probe used for the present invention, it is preferable to confirm Tm. Tm refers to the temperature at which 50% of a nucleic acid strand will form a hybrid with its complementary strand. Thus, the temperature for hybridization needs to be optimized so that template DNA or RNA forms a double strand with its probe for hybridization. If this temperature is too low, non-specific reaction is likely to result, and therefore the temperature is preferably as high as possible. Accordingly, Tm of the nucleic acid fragment to be designed is an important factor for hybridization. Tm can be confirmed by utilizing a known probe designing software, where exemplary software that can be used for the present invention includes Probe Quest (registered trademark; Dynacom). Alternatively, Tm can also be confirmed by calculating by own without using a software. In this case, calculation based on nearest neighbor base pair method (Nearest Neighbor Method), Wallance method, GC% method or the like can be utilized. Average Tm of the probe of the present invention is preferably, but not limited to, about 35-70°C or 45-60°C. Other conditions for allowing specific hybridization with a probe include the GC content and the like, which are well known to those skilled in the art.

Furthermore, nucleotides constituting a probe used for the present invention may be any of DNA, RNA or PNA, or a hybrid of two or more types of DNA, RNA and PNA.

Specifically, examples of the probe used for the present invention preferably include those containing the nucleotide sequence of DNA (d) or (e) below. For example, when the primers shown in Table 1-2 (SEQ ID NOS:1 and 2) are used for amplification, the sequences shown in Table 1-1-1 (SEQ ID NOS:3-59) above can be used as the probes, where at least two sequences selected from the nucleotide sequences represented by SEQ ID NOS:3-59 are preferably used. Alternatively, they may be complementary sequences of at least two sequences selected from the nucleotide sequences represented by SEQ ID NOS:3-59, sequences substantially identical to at least two sequences selected from the nucleotide sequences represented by SEQ ID NOS:3-59, or sequences substantially identical to complementary sequences of at least two sequences selected from the nucleotide sequences represented by SEQ ID NOS:3-59.

Herein, substantially identical means that the sequence specifically hybridizes with the sequence represented by any of SEQ ID NOS:3-59 or with the complementary sequence thereof under stringent conditions.
(d) DNA consisting of a nucleotide sequence represented by any of SEQ ID NOS:3-59.
(e) DNA that hybridizes with DNA consisting of a nucleotide sequence complementary to DNA (d) above under stringent conditions, and that has a function of detecting at least a part of a nucleotide sequence of nucleic acids derived from an intraoral bacterium.

For the respective DNAs (d) above, their specific nucleotide sequences, probe names and intraoral bacteria targeted for detection are shown in Table 1-1-1 above.

DNA (e) above can be obtained from a cDNA library or a genomic library by carrying out a known hybridization method such as colony hybridization, plaque hybridization or southern blotting, using any of DNAs (d) above, DNA consisting of a nucleotide sequence complementary thereto, or a fragment thereof as a probe. The library is not limited, and a library generated by a known method or a commercially available cDNA library or genomic library may be utilized. For a detailed procedure of the hybridization method, see references mentioned above. DNA to be hybridized is preferably a nucleotide sequence having at least 60% or more, more preferably 80% or more, still more preferably 90% or more, yet more preferably 95% or more, particularly preferably 98% or more and most preferably 99% or more homology to the nucleotide sequence of DNA (d) above.

The probe used for the present invention can be prepared, for example, through chemical synthesis employing a general oligonucleotide synthesis method (purification can be carried out by HPLC or the like). Such a probe can be designed, for example, by Probe Quest (registered trademark: from Dynacom). In addition, the probe of the present invention may also contain, for example, an additional sequence such as a tag sequence.

According to the method of the present invention, the nucleotide sequence of the nucleic acids of the above-described intraoral bacterium targeted for detection does not have to be the exact nucleotide sequence thereof, and it may have partial mutations such as deletion, substitution or insertion in the nucleotide sequence. Accordingly, the nucleotide sequence of the nucleic acids targeted for detection may also be a mutant gene that can hybridize with a sequence complementary to said nucleotide sequence under stringent conditions and that has functions and activity originating from said nucleotide sequence, and thus a probe can also be designed based on a nucleotide sequence of such mutant gene. Herein, "stringent conditions" may refer to the application of the conditions similar to those described above.

### 2. DNA chip for detecting genes of intraoral bacteria to measure levels of intraoral bacteria

As described above, a DNA chip can be used in the method of the present invention, where this DNA chip has a plurality of various oligonucleotide probes described in Item 1 above arranged on a substrate that serves as a support.

The substrate that serves as a support may be in any form of a flat plate (glass plate, resin plate, silicon plate, etc.), a stick, beads or the like. When a flat plate is used as the support, predetermined probes can be fixed thereon by types at predetermined intervals (spotting method, etc.; see Science 270, 467-470 (1995), etc.). Alternatively, predetermined probes can sequentially be synthesized thereon by types at specified positions (photolithography method, etc.; see Science 251, 767-773 (1991), etc.). Another preferable form of the support may be, for example, one that uses hollow fibers. When hollow fibers are used as a support, a preferable exemplary DNA chip (hereinafter, referred to as a "fiber-type DNA chip") can be obtained by fixing predetermined probes in respective hollow fibers by types, bundling and fixing all of the hollow fibers, and then repeatedly cutting the fibers with respect to the longitudinal direction of the fibers. This microarray may also be referred to as a microarray that has nucleic acids fixed in through holes of a substrate, which is also referred to as a so-called "through-hole-type DNA chip" (see Japanese Patent Publication No. 3510882).

The method for fixing probes onto a support is not limited, and any binding mode can be employed. Moreover, fixing is not limited to direct fixing onto the support. For example, a support may be pre-coated with a polymer such as polylysine so that probes may be fixed onto the treated support. Furthermore, when a tubular body such as hollow fibers is used as a support, the tubular body may be made to retain a gel-like substance so that probes can be fixed to the gel-like substance.

Hereinafter, a fiber-type DNA chip, one form of DNA chips, will be described in detail. This DNA chip can be prepared, for example, through Steps (i)-(iv) below.
(i) Step of three-dimensionally arranging a plurality of hollow fibers such that the hollow fibers are oriented in the same longitudinal direction to produce an arranged body.
(ii) Step of embedding the arranged body to produce a block body.
(iii) Step of introducing a gel-precursor polymerizable solution containing an oligonucleotide probe into a hollow of each hollow fiber of the block body to allow polymerization reaction, thereby making the hollow to retain the gel-like substance containing the probe.
(iv) Step of cutting the hollow fibers in a direction intersecting with the longitudinal direction thereof to slice the block body.

An example of the material used as the hollow fiber preferably include, but not limited to, a material described in Japanese Unexamined Patent Application Publication No. 2004-163211.

The hollow fibers are three-dimensionally arranged such that their lengths are equal in the longitudinal direction (Step (i)). The method employed for the arrangement may be, for example, a method in which a plurality of hollow fibers are arranged in parallel at predetermined intervals on a sheet-like material such as an adhesive sheet to form a sheet, which is thereafter wound up in spiral (see Japanese Unexamined Patent Application Publication No. *Heisei* 11-108928), or a method in which two porous plates provided with a plurality of pores at predetermined intervals are layered such that the pores meet each other, where hollow fibers are passed through the pores and then the two porous plates are temporary fixed at a distance and a curable resin material is charged and cured around the hollow fibers between the two porous plates (see Japanese Unexamined Patent Application Publication No. 2001-133453).

The produced arranged body is embedded so that the arrangement is not disordered (Step (ii)). The method of embedment may preferably be a method in which a polyurethane resin, an epoxy resin or the like is poured into the gap between the fibers, a method in which the fibers are adhered to each other by heat welding, or a method likewise.

The hollow of each hollow fiber of the embedded arranged body is filled with a gel-precursor polymerizable solution (gel-forming solution) containing the oligonucleotide probe to allow polymerization reaction in the hollow (Step (iii)). As a result, the hollow of each hollow fiber can retain the gel-like substance having the probe fixed thereto.

The gel-precursor polymerizable solution refers to a solution containing a reactive substance such as a gel-forming polymerizable monomer, where said monomer or the like can be polymerized/crosslinked so that the solution becomes a gel-like substance. Examples of such monomer include acrylamide, dimethylacrylamide, vinylpyrrolidone and methylene-bis-acrylamide. In this case, the solution may contain a polymerization initiator and the like.

After fixing the probes in the hollow fibers, the block body is cut into slices in a direction intersecting with (preferably perpendicular to) the longitudinal direction of the hollow fibers (Step (iv)). The resulting slice can be used as a DNA chip. The thickness of this DNA chip is preferably about 0.01 mm-1 mm. The block body can be cut, for example, with a microtome, a laser or the like.

Preferable examples of the above-described fiber-type DNA chip include DNA chip (Genopal™) from Mitsubishi Rayon and the like.

In the fiber-type DNA chip, the probes are three-dimensionally arranged in the gel as described above so as to maintain a three-dimensional structure. Accordingly, the detection efficiency is enhanced as compared to a flat DNA chip that has probes bound onto a coated surface of a glass slide, and thus a highly sensitive and highly reproducible examination can be realized.

The number of types of probes arranged on the DNA chip is 500 types or less, preferably 250 types or less and more preferably 100 types or less per DNA chip. By limiting the number (types) of the arranged probes to some extent, the intraoral bacteria of interest can be detected with high sensitivity. The types of the probes are distinguished by the nucleotide sequences. Therefore, usually, even probes that are derived from the same gene are specified as different types of probes even if a single difference exists between the nucleotide sequences.

### 3. Detection of gene of intraoral bacterium

According to the method of the present invention, the method of detecting a gene of an intraoral bacterium for measuring the level thereof comprises the following steps.
(i) Step of extracting nucleic acids from a specimen, namely, an intraoral sample collected from a subject.
(ii) Step of allowing the extracted nucleic acids to make contact with the oligonucleotide probe of the present invention or the DNA chip of the present invention described above.
(iii) Step of calculating the level of the bacterium based on the signal intensity obtained from the DNA chip.

Hereinafter, the steps of the detection method will be described one by one in detail.

### (1) About Step (i)

In this step, an intraoral sample collected from a subject human or organism is used as a specimen, where the nucleic acids of the bacterium contained in the specimen are extracted. The type of the intraoral sample collected is not particularly limited. For example, saliva, plaque (subgingival plaque, supragingival plaque), tongue fur, mousewash or the like may be used, among which plaque is preferable, and subgingival plaque collected from a habitat of the largest number of periodontal disease bacteria is more preferable.

The method of collecting the intraoral sample is not particularly limited, and it may suitably be selected according to the type of the sample. For example, in a case where saliva is used as the intraoral sample, a method that utilizes a commercially available saliva collection kit, a method in which a cotton swab is placed in the mouse to collect saliva, a method in which saliva is directly collected in a container, or the like may be employed.

In a case where plaque is used as the intraoral sample, brushing of the tooth surface or the interdental area with a tooth brush, scratching of the tooth surface with a cotton swab, scratching of the interdental area with an interdental brush, a paper point technique, or the like may be employed. The tooth brush, the cotton swab, the interdental brush or the paper point used for collecting the plaque is immersed in sterilized water and agitated if necessary to dissolve or suspend the plaque. The resulting solution or suspension may also be used as the specimen. The amount of plaque to be collected is not particularly limited, and it may, for example, be an amount that can be collected with a single paper point.

In a case where tongue fur is used as the intraoral sample, scratching of the tongue surface with a cotton swab may be employed. The cotton swab used for collecting the plaque is dissolved or suspended so as to use the resulting solution or suspension as the specimen. The amount of tongue fur to be collected is not particularly limited, and it may, for example, be an amount that can be collected with a single cotton swab.

In a case where a mousewash is used as the intraoral sample, the mousewash or water is kept in the mouse to collect saliva in a container together with the mousewash or water to use the obtained solution as the specimen. The mousewash may, for example, be sterilized physiological saline or the like.

Next, nucleic acids are extracted from the bacteria present in the obtained intraoral sample. The extraction method is not limited and any known method can be employed. For example, the method may be an automatic extraction method using an instrument, a method utilizing a commercially available nucleic acid extraction kit, a method in which a treatment with proteinase K is followed by phenol extraction, a method utilizing chloroform, a simple extraction method by heating or dissolving the sample, or the like. Alternatively, one may proceed to the next step without extracting nucleic acids from the specimen.

Without limitation, the nucleic acids obtained from the specimen may be allowed to make direct contact with the DNA chip or the like, or a nucleotide sequence region desired may be amplified by PCR or the like so as to allow the amplified fragments thereof to make contact with the DNA chip or the like. The region to be amplified using the obtained nucleic acids as a template is a site coding for a nucleic acid region containing the nucleotide sequence of the probe or the oligonucleotide arranged on the DNA chip used in the present invention. The region desired to be amplified is not limited and a nucleotide sequence of a highly conserved region for all kinds of intraoral bacteria can be utilized to amplify a mixture of various kinds of intraoral bacteria at once. The sequence used for such amplification can be determined based on analyses of nucleotide sequences of an experimentally isolated and purified polynucleotide, or can be determined *in Silico* by searching and aligning a nucleotide sequence known from database of nucleotide sequences or the like. The database of nucleic acids or amino acids may be, for example, without limitation, DDBJ (DNA Data Bank of Japan), EMBL (European Molecular Biology Laboratory, EMBL nucleic acid sequence data library), GenBank (Genetic sequence data bank), Taxonomy database from NCBI (National Center for Biotechnology Information), or the like.

Specifically, the site desired to be amplified is preferably ribosome RNA (16S rRNA) gene in chromosomal DNAs of intraoral bacteria. PCR primers that can be used for amplification of this region may preferably be, for example, SEQ ID NOS:1 and 2 shown in Table 1-2. The amplification of the nucleic acids by PCR method can be carried out by a common method.

The extracted nucleic acids and the amplified fragments thereof in this step may suitably be labeled so as to be used in the detection process following hybridization. Specifically, a method in which the terminal of the PCR primer is labeled with a reporter dye, a method in which a reactive nucleotide analog is incorporated upon reverse transcription reaction, a method in which biotin-labeled nucleotides are incorporated, and the like may be contemplated. Furthermore, labeling can be carried out through reaction with a fluorescently labeled reagent after preparation. As the fluorescent reagent, for example, various kinds of reporter dyes (e.g., Cy5, Cy3, VIC, FAM, HEX, TET, fluorescein, FITC, TAMRA, Texas red, Yakima Yellow, etc.) can be used.

### (2) About Step (ii)

In this step, the nucleic acids or the amplified fragments thereof obtained in Step (i) are allowed to make contact with the probes or the DNA chip used for the present invention. Specifically, a hybridization solution containing said nucleic acids or the like is prepared so as to allow the nucleic acids or the like in said solution to bind (hybridize) with the oligonucleotide probe mounted on the DNA chip. The hybridization solution can suitably be prepared using a buffer such as SDS or SSC according to a common method.

The hybridization reaction can be carried out by suitably the setting reaction conditions (type, pH, temperature and the like of the buffer) such that the nucleic acids or the like in the hybridization solution can hybridize with the oligonucleotide probe mounted on the DNA chip under stringent conditions. The term "stringent conditions" as used herein refers to conditions that are less likely to result cross-hybridization induced by similar sequences, or that can dissociate any nucleic acids cross-hybridized with similar sequences. Specifically, it refers to the conditions for washing the DNA chip upon or after the hybridization reaction.

For example, the conditions upon hybridization reaction may be a reaction temperature of preferably 35-70°C, more preferably 40-65°C and hybridization time of preferably about 1 minutes to 16 hours.

Moreover, as the conditions for washing the DNA chip after the hybridization, washing liquid composition is preferably 0.24M Tris-HCl/0.24M NaCl/0.05% Tween-20, and a temperature upon washing is preferably 35-80°C or 40-65°C, more preferably 45-60°C. More specifically, the conditions preferably include a salt (sodium) concentration of 48-780 mM and a temperature of 37-80°C, and more preferably include a salt concentration of 97.5-390 mM and a temperature of 45-60°C.

After washing, the detected intensity of each spot is measured with a device that can detect the label of the nucleic acids or the like bound to the probe. For example, if the above-described nucleic acids or the like are fluorescently labeled, a fluorescence detector such as CRBIO (from Hitachi Software Engineering), arrayWoRx (from GE Healthcare), Affymetrix 428 Array Scanner (from Affymetrix), GenePix (from Axon Instruments), ScanArray (from PerkinElmer) or Genopal Reader (from Mitsubishi Rayon) can be used to measure the fluorescence intensity. In a case where this device is a fluorescence scanner, scanning can be performed by suitably adjusting, for example, the laser output and sensitivity of the detection section, whereas in a case where this device is a CCD camera type scanner, scanning can be performed by suitably adjusting the exposure time. A quantitative method based on the scan results can be performed with a quantification software. The quantification software is not particularly limited, and the average value, the median or the like of the fluorescence intensity of the spot can be used for quantification. Furthermore, considering the dimensional accuracy of the spot area of the DNA fragment and the like, adjustment is preferably performed upon quantification, for example, using the fluorescence intensity of the spot having no probe as background.

### (3) About Step (iii)

In this step, the bacterial level of the bacterial species targeted for detection is calculated from the signal intensity acquired by the above-described procedure. For example, it can be expressed as a signal-to-noise ratio based on the signal intensity of the probe for detecting the bacterium targeted for detection and the background signal intensity. Alternatively, it may be preferable to conduct detections for each bacterium under a plurality of conditions by varying the bacterial chromosomal DNA concentration so as to acquire a conversion factor (standard curve) for each bacterium in advance for calculating the chromosomal DNA concentration based on the signal intensity obtained under each concentration condition, by which chromosomal DNA concentrations can be calculated from signal intensities obtained under the respective conditions.

In either case, a correction coefficient may be calculated for each DNA chip so that signal intensities of an absolute level index probe acquired by detection of a plurality of DNA chips will be constant. Accordingly, comparison can be made among the DNA chips by taking the correction coefficient into account for a signal intensity of a bacterium targeted for detection from each DNA chip.

### 4. Assessments of status of periodontal disease and/or dental caries

### (1) Assessment of status of periodontal disease (Assessment criteria 1)

If the total bacterial count of at least one of *Porphyromonas gingivalis, Tannerella forsythensis* and *Treponema denticola* among the bacteria contained in saliva as the intraoral sample is less than 0.01% to the total bacterial count, it is assessed to be "mild", "moderate" if it is 0.01% or more but less than 0.5% to the total bacterial count, and "severe" if it is 0.5% or more to the total bacterial count.

In a case where plaque is used as the intraoral sample, the status of periodontal disease is assessed to be "mild" if the total bacterial count of at least one of *Porphyromonas gingivalis, Tannerella forsythensis* and *Treponema denticola* is less than 0.1% to the total bacterial count, "moderate" if it is 0.1% or more but less than 5% to the total bacterial count, and "severe" if it is 5% or more to the total bacterial count.

This bacterial examination for periodontal disease relies on the report of classifying the status of periodontal disease into 4 stages according to the total count of the three types of "Red Complex" bacteria in plaque or saliva. The classification uses the counts of three types of bacteria as criteria, where the reference values are derived from multiple clinical research reports and general descriptions, are recognized as a guideline by the Japanese Society of Periodontology, and thus are found to be sufficiently reasonable values. Assuming the case of saliva, the reference values were set to 1/10 the values in the case of plaque. The two stages in the middle of the 4 stages of the classification are combined together as a single stage to give three-stage classification, which would be easier for the person who takes the examination to understand (Non-patent document 3).

### (2) Assessment of status of dental caries (Assessment criteria 2)

Moreover, the status of dental caries is assessed to be "mild" if the *Streptococcus mutans* bacterial count among the bacteria contained in saliva as the intraoral sample is less than 0.05% to the total bacterial count, the status of dental caries is assessed "moderate" if it is 0.05% or more but less than 2.5% to the total bacterial count, and the status of dental caries is assessed "severe" if it is 2.5% or more to the total bacterial count.

These assessment criteria were determined, for example, based on four-stage assessment of dental caries risk with *Streptococcus mutans* cultures using a caries risk test kit from Ivoclar Vivadent or the like (J Health Care Dent.2000, 2, 4-17).

According to this culture assessment, colonies resulting from *Streptococcus mutans* culture are classified as follows by visual observation.
"Class 0" when less than 100,000 CFU/mL
"Class 1" when 100,000 CFU/mL or more but less than 500,000 CFU/mL
"Class 2" when 500,000 CFU/mL or more but less than 1,000,000 CFU/mL
"Class 3" when 1,000,000 CFU/mL or more

CFU refers to a colony-forming unit upon culture, where approximately 1 CFU is known to be 100 genome copies. Furthermore, about 1,000,000,000 genome copies of bacteria are contained in 1 mL of human saliva.

Therefore, based on these values, the above-mentioned criteria, i.e., "low" for "Class 0", "moderate" for "Class 1", and "high" for "Classes 2 and 3", were set herein.

### (3) Others

The assessments of the status of periodontal disease and dental caries obtained with the present invention gives the status predicted just from the bacterial counts, and do not indicate accurate disease condition. Therefore, diagnosis by a dentist is required for diagnosis of accurate disease condition.

According to the present invention, however, the status of periodontal disease and dental caries can conveniently be assessed, which allows early discovery and early treatment as well as prevention of periodontal disease and dental caries.

### II. Regarding Method B) above

While Method B) above is not limited, it may specifically include, for example, as a first aspect, the steps of:
measuring i) a bacterial level of a specific bacterium, ii) a total level of bacteria present in an intraoral sample collected from a subject, or iii) both the level of a specific bacterium and the total level of bacteria present in the intraoral sample; and
performing a statistical analysis based on the acquired measurement results to assess the severity of periodontal disease.

The method may include, as a second aspect, the steps of:
measuring i) a bacterial level of a specific bacterium, ii) a total level of bacteria present in an intraoral sample collected from a subject before and after a treatment of periodontal disease, or iii) both the level of a specific bacterium and the total bacteria level present in the intraoral sample; and
assessing the effect of treating periodontal disease based on the results from comparison between the bacterial level before the treatment and the bacterial level after the treatment.

The method may include, as a third aspect, the steps of:
measuring i) a bacterial level of a specific bacterium, ii) a total level of bacteria present in an intraoral sample collected from a subject, or iii) both the level of a specific bacterium and the total level of bacteria present in the intraoral sample; and
assessing the risk of exacerbation of periodontal disease based on the ratio of the acquired bacterial levels.

### 1. Oligonucleotide probe used for measuring intraoral bacterial levels

According to the method of the present invention, a DNA chip can be used for measuring an intraoral bacterial level of an intraoral sample collected from a subject, where said DNA chip may, for example, be equipped with following probes (a) and at least either one of probes (b) and (c). In general, a DNA chip is a generic term for a substrate arranged with probes. Herein, the term DNA chip, DNA microarray and the like are not distinguished and are taken as synonyms.
(a) Probes of nucleic acids that specifically hybridize with genes of respective bacteria targeted for detection.
(b) Total level index probe of nucleic acids that hybridize with the genes of all of the bacteria.
(c) Probes of nucleic acids that specifically hybridize with one or more respective kinds of absolute level indices.

### (1) Intraoral bacteria targeted for measurement

In an examination method of the present invention, intraoral bacteria targeted for measurement, namely, bacterial species targeted for detection, may be, but not limited to, bacteria belonging to genus *Porphyromonas,* bacteria belonging to genus *Tannerella,* bacteria belonging to genus *Treponema,* bacteria belonging to genus *Prevotella,* bacteria belonging to genus *Campylobacter,* bacteria belonging to genus *Fusobacterium,* bacteria belonging to genus *Parvimonas,* bacteria belonging to genus *Streptococcus,* bacteria belonging to genus *Aggregatibacter,* bacteria belonging to genus *Capnocytophaga,* bacteria belonging to genus *Eikenella,* bacteria belonging to genus *Actinomyces,* bacteria belonging to genus *Veillonella,* bacteria belonging to genus *Selenomonas,* bacteria belonging to genus *Lactobacillus,* bacteria belonging to genus *Pseudomonas,* bacteria belonging to genus *Haemophilus,* bacteria belonging to genus *Klebsiella,* bacteria belonging to genus *Serratia,* bacteria belonging to genus *Moraxella,* and bacteria belonging to genus *Candida.*

More specifically, bacterial species targeted for detection are, for example, preferably at least one of *Porphyromonas gingivalis, Tannerella forsythia, Treponema denticola, Prevotella intermedia, Aggregatibacter actinomycetemcomitans, Fusobacterium nucleatum* subsp. *vincentii, Fusobacterium nucleatum* subsp. *polymorphum, Fusobacterium nucleatum* subsp. *animalis, Fusobacterium nucleatum* subsp. *nucleatum, Streptococcus sanguis, Streptococcus mitis, Actinomyces viscosus, Streptococcus aureus, Pseudomonas aeruginosa, Streptococcus pyogenes, Streptococcus pneumoniae, Campylobacter gracilis, Campylobacter rectus, Campylobacter showae, Fusobacterium periodonticum, Parvimonas micra, Prevotella nigrescens, Streptococcus constellatus, Campylobacter concisus, Capnocytophaga gingivalis, Capnocytophaga ochracea, Capnocytophaga sputigena, Eikenella corrodens, Streptococcus gordonii, Streptococcus intermedius, Streptococcus mitis* bv. 2, *Actinomyces odontolyticus, Veillonella parvula, Actinomyces naeslundii* II, *Selenomonas noxia* and the like, which are currently considered to be relevant to periodontal disease.

According to the present invention, *Porphyromonas gingivalis, Tannerella forsythia, Treponema denticola, Fusobacterium nucleatum, Campylobacter rectus, Streptococcus constellatus, Streptococcus gordonii, Streptococcus mitis, Streptococcus oralis, Streptococcus sanguinis, Veillonella parvula* and the like are preferably targeted for detection. More preferably, at least one, preferably two of them are targeted for detection. By measuring and comparing the levels of bacteria before and after a treatment of periodontal disease, the effect of treating periodontal disease can objectively be assessed. Sufficiently decreased bacterial count indicates the presence of therapeutic effect, whereas no decrease in the bacterial count indicates the absence of therapeutic effect and thus the need of alternative therapy.

In addition, since the bacterial levels are information that has a strong correlation or inverse correlation with clinical information, they can be indices for indicating severity of periodontal disease. Detailed information related to severity of periodontal disease can be obtained, which shows which of highly malignant bacteria or lowly malignant bacteria is higher among the total bacterial count.

When assessing risk of exacerbation of periodontal disease, intraoral bacteria targeted for measurement are preferably at least one selected from the group consisting of *Porphyromonas gingivalis, Tannerella forsythia* and *Treponema denticola,* and *Fusobacterium nucleatum.* Since an increase in bacterium *Fusobacterium nucleatum* precedes an increase in the periodontal pocket depth PD, i.e., clinical information of exacerbation of the periodontal disease, or an increase in the "Red Complex", i.e., an even earlier stage, detection of *Fusobacterium nucleatum* can be an early index of periodontal disease. In other words, the bacterial level of *Fusobacterium nucleatum* to the total bacterial levels of the three kinds of "Red Complex" bacteria, namely, *Porphyromonas gingivalis, Tannerella forsythia* and *Treponema denticola* can be the index for assessing risk of exacerbation.

### (2) About probe (a)

According to the present invention, oligonucleotide DNA that may be used as the probe (a) is one that can hybridize with a nucleotide sequence of a specific region in a nucleotide sequence of nucleic acids derived from an intraoral bacterium. While said nucleic acids may be, without limitation, either DNA (including chromosomal DNA, plasmid DNA or the like) or RNA, it is preferably chromosomal DNA. Specifically, an oligonucleotide used as a probe of the present invention is one that can hybridize with a nucleotide sequence of 16S rRNA gene in the chromosomal DNA of the intraoral bacterium.

Probes that can be used for the present invention are preferably obtained by selecting regions that can serve as nucleotide sequences specific to the above-described respective intraoral bacteria to be detected, and designing nucleotide sequences of said regions. Generally, probe design requires, in addition to selection of specific regions, matching of melting temperatures (Tm) and minimization of formation of a secondary structure.

A nucleotide sequences that is specific to each species of intraoral bacteria may be found, for example, by providing a multiple alignment to design a probe located in a region unique to that species. More specifically, while the algorithm for providing the alignment is not particularly limited, a program such as ClustalX1.8 may be utilized as an analysis program. While default status can be employed to run the program, the parameters used for providing the alignment may suitably be adjusted according to the type of the program or the like.

Meanwhile, the specificity of the probe may be such that it allows collective detection of the bacteria belonging to the same genus based on specificity at a genus level, or such that it allows detection based on specificity at an individual species level, which can suitably be determined according to the purpose of the bacteria detection.

### (3) About probe (b)

The total level index probe is a probe intended to capture all of the bacteria in the specimen that were amplified with specific primer pairs. With respect to bacteria detection, it is crucial to detect the total bacterial level from the viewpoint of how much the bacteria targeted for detection exist among the entire bacteria including bacteria not targeted for detection, and also from the viewpoint of how much level of bacteria is present in the specimen in the first place.

The bacteria not targeted for detection is understood as a sum (total) of bacteria whose presence and kinds are known but not targeted for detection, and bacteria whose presence and kinds are unknown.

For the detection of the total bacterial level, for example, the total bacterial level may be measured independently from the DNA chip, but convenience of handling would be enhanced by providing a probe that can serve as an index of the total bacterial level on the DNA chip. The probe used may be a nucleotide sequence that is common to the various kinds of bacterial species among the nucleotide sequences amplified by the primer pairs. If no such sequence is found, a plurality of relatively common sequences may be designed so that they can be used for comprehensive judgement to find a total level index probe. The total level index probe is preferably a probe that hybridizes with nucleic acids derived from the bacteria contained in the specimen, specifically, a probe that contains a nucleotide sequence commonly included by the multiple kinds of bacteria targeted for detection among the nucleotide sequences amplified by the above-described specific primer pairs. An example of the total level index probe is shown in Table 2-1 (SEQ ID NO:159).

The total level index is generally high since it represents the total level of the amplified products specific to the individual bacterial species, and thus the signal intensity of interest may exceed the detectable signal intensity range.

In order to avoid such circumstance, the amount of the specimen subjected to hybridization is preferably limited. Alternatively, the Tm value of the probe may be lowered, for example, upon designing the probe. Specifically, a technique such as decreasing the GC content or shortening the length of the probe sequence itself may be contemplated.

Furthermore, nucleic acids that can competitively act against the hybridization between the amplified nucleic acids and the total level index probe may be added upon hybridization to reduce the signal intensity. Examples of such nucleic acids include nucleic acids having a sequence that is entirely or partially identical to the total level index probe, and nucleic acids having a sequence that is entirely or partially complementary to the sequence of the total level index probe.

### (4) About probe (c)

An absolute level index probe refers to a probe that hybridizes solely to the nucleic acids of the absolute level index.

Herein, an absolute level index refers to nucleic acids that are added to the specimen for a certain amount prior to amplification reaction and hybridization reaction. The absolute level index is nucleic acids that ensure actual amplification reaction upon usual amplification reaction, and plays a role as a so-called positive control.

Therefore, the DNA chip may be provided with a probe specific to the absolute level index so that whether or not amplification reaction, hybridization and the like have appropriately taken place can be confirmed by detecting said probe. Moreover, if one type of absolute level index is set, the signal intensity of said absolute level index acquired from multiple DNA chips should be constant, and thus the signal intensities of the absolute level index may be compared to calculate correction coefficients if there are some variations in the amplification efficiency or the hybridization efficiency. The corrected signal intensities can be used upon comparison among multiple DNA chips.

Examples of probes specific to respective bacteria will be shown in Table 2-1 (SEQ ID NOS:93-158). In addition, examples of the absolute level index probes will be shown in Table 2-1 (SEQ ID NOS:160-174) while examples of absolute level indices will be shown in Table 2-2 (SEQ ID NOS:175-189).

If the absolute level index is added prior to amplification reaction, it should be nucleic acids that can be amplified with a specific primer pair, in other words, it should possess a nucleotide sequence complementary to the primer pair, and it should possess a nucleotide sequence that is not possessed by any of the bacteria targeted for detection or the bacteria not targeted for detection in order to be detected through hybridization.

**[Table 2-1]**

| SEQ ID NO | Name of probe | Sequence (5' to 3') |
|---|---|---|
| 93 | *Porphyromonas gingivalis* probe 1 | TTCAATGCAATACTCGTATC |
| 94 | *Porphyromonas gingivalis* probe 2 | GTACATTCAATGCAATACTC |
| 95 | *Tannerella forsythia* probe 1 | CACGTATCTCATTTTATTCC |
| 96 | *Tannerella forsythia* probe 2 | AATACACGTATCTCATTTTATT |
| 97 | *Tannerella forsythia* probe 3 | CACGTATCTCATTTTATTCCCCTGT |
| 98 | *Treponema denticola* probe 1 | CTCTTCTTCTTATTCTTCAT |
| 99 | *Treponema denticola* probe 2 | CCTCTTCTTCTTATTCTTCAT |
| 100 | *Treponema denticola* probe 3 | CCTCTTCTTCTTATTCTTCATCTGC |
| 101 | *Campylobacter gracilis* probe 1 | GCCTTCGCAATAGGTATT |
| 102 | *Campylobacter gracilis* probe 2 | CGCCTTCGCAATAGGTAT |
| 103 | *Campylobacter* spp. probe 1 | ATTCTTTCCCAAGAAAAGGA |
| 104 | *Campylobacter* spp. probe 2 | GTCATAATTCTTTCCCAAGA |
| 105 | *Campylobacter* spp. probe 3 | CAATGGGTATTCTTCTTGAT |
| 106 | *Fusobacterium nucleatum* probe 1 | TAGTTATACAGTTTCCAACG |
| 107 | *Fusobacterium nucleatum* probe 2 | CTAGTTATACAGTTTCCAAC |
| 108 | *Fusobacterium nucleatum* probe 3 | TCCAGTACTCTAGTTACACA |
| 109 | *Fusobacterium nucleatum* probe 4 | CCAGTACTCTAGTTACACA |
| 110 | *Fusobacterium nucleatum* probe 5 | TTTCTTTCTTCCCAACTGAA |
| 111 | *Fusobacterium nucleatum* probe 6 | ATTTCTTTCTTCCCAACTGA |
| 112 | *Fusobacterium nucleatum* probe 7 | TACATTCCGAAAAACGTCAT |
| 113 | *Fusobacterium nucleatum* probe 8 | TTACATTCCGAAAAACGTCA |
| 114 | *Fusobacterium nucleatum* probe 9 | TATGCAGTTTCCAACGCAA |
| 115 | *Fusobacterium nucleatum* probe 10 | CTCTAGTTATGCAGTTTCC |
| 116 | *Parvimonas micra* probe 1 | AAGTGCTTAATGAGGTTAAG |
| 117 | *Parvimonas micra* probe 2 | TTTCAAGTGCTTAATGAGGT |
| 118 | *Prevotella intermedia* probe 1 | GGGTAAATGCAAAAAGGCA |
| 119 | *Prevotella intermedia* probe 2 | GCAAGGTAGATGTTGAGCA |
| 120 | *Prevotella intermedia* probe 3 | CGAAGGGTAAATGCAAA |
| 121 | *Prevotella intermedia* probe 4 | CGAAGGGTAAATGCAAAGGGGC |
| 122 | *Prevotella intermedia* probe 5 | CGAAGGGTAAATGCAAAAAGGC |
| 123 | *Prevotella nigrescens* probe 1 | CTTTATTCCCACATAAAAGC |
| 124 | *Prevotella nigrescens* probe 2 | TCCTTATTCATGAGGTACAT |
| 125 | *Streptococcus* spp. probe 1 | AAGTACCGTCACTGTGTG |
| 126 | *Streptococcus* spp. probe 2 | TTAAGTACCGTCACTGTGT |
| 127 | *Aggregatibacter actinomycetemcomitans* probe 1 | GTCAATTTGGCATGCTATTA |
| 128 | *Aggregatibacter actinomycetemcomitans* probe 2 | TTTAACGTCAATTTGGCATG |
| 129 | *Aggregatibacter actinomycetemcomitans* probe 3 | GTCAATTTGGCATGCTATTAACACACC |
| 130 | *Aggregatibacter actinomycetemcomitans* probe 4 | GTCAAGTTGGCATGCTATTAACACACC |
| 131 | *Campylobacter concisus* probe 1 | CCCAAGCAGTTCTATGGT |
| 132 | *Campylobacter concisus* probe 2 | TCCCAAGCAGTTCTATGG |
| 133 | *Capnocytophaga* spp. probe 1 | TACACGTACACCTTATTCTT |
| 134 | *Capnocytophaga* spp. probe 2 | CATCAATGTACACGTACAC |
| 135 | *Capnocytophaga* spp. probe 3 | CATTCAAGACCAACAGTTT |
| 136 | *Capnocytophaga* spp. probe 4 | CAACCATTCAAGACCAACA |
| 137 | *Capnocytophaga* spp. probe 5 | GCTTAGTTGAGCTAAGCG |
| 138 | *Capnocytophaga* spp. probe 6 | TCAAAGGCAGTTGCTTAGT |
| 139 | *Eikenella corrodens* probe 1 | CTAGCTATCCAGTTCAGAA |
| 140 | *Eikenella corrodens* probe 2 | CTCTAGCTATCCAGTTCAG |
| 141 | *Streptococcus* spp. probe 3 | CACCCGTTCTTCTCTTACA |
| 142 | *Streptococcus* spp. probe 4 | CACCCGTTCTTCTCTTAC |
| 143 | *Streptococcus intermedius* probe 1 | CAGTATGAACTTTCCATTCT |
| 144 | *Streptococcus intermedius* probe 2 | ACAGTATGAACTTTCCATTCT |
| 145 | *Streptococcus* spp. probe 5 | CCCCTCTTGCACTCAAGT |
| 146 | *Streptococcus* spp. probe 6 | TCTCCCCTCTTGCACTCA |
| 147 | *Streptococcus* spp. probe 7 | TCCCCTCTTGCACTCAAGT |
| 148 | *Actinomyces odontolyticus* probe 1 | AAGTCAGCCCGTACCCA |
| 149 | *Actinomyces odontolyticus* probe 2 | CGCACTCAAGTCAGCCC |
| 150 | *Veillonella parvula* probe 1 | CTATTCGCAAGAAGGCCTT |
| 151 | *Veillonella parvula* probe 2 | TATTCGCAAGAAGGCCTT |
| 152 | *Veillonella parvula* probe 3 | TCCTTCTAACTGTTCGC |
| 153 | *Actinomyces naeslundii* II probe 1 | CACAAGGAGCAGGCCTG |
| 154 | *Actinomyces naeslundii* II probe 2 | CCACCCACAAGGAGCAG |
| 155 | *Actinomyces naeslundii* II probe 3 | AACCCACCCACAAACGA |
| 156 | *Selenomonas noxia* probe 1 | TTCGCATTAGGCACGTTC |
| 157 | *Selenomonas noxia* probe 2 | CTATTCGCATTAGGCACGT |
| 158 | *Streptococcus mutans* probe | CACACGTTCTTGACTTAC |
| 159 | Total level index probe | CGTATTACCGCGGCTGCTGGCAC |
| 160 | Absolute level index 1 probe | CGTGCATTGTCGTGTAGGTTCGACCCTAAT |
| 161 | Absolute level index 2 probe | GCAGCTACGTTCATACCTACGCAAGGCATT |
| 162 | Absolute level index 3 probe | GAGGAGATACCGAATCGGTCGACGACATTT |
| 163 | Absolute level index 4 probe | TGTTGCGTGAAGGTCGTGAACGATTGGCAA |
| 164 | Absolute level index 5 probe | CCCCTACTGAGCAAACGTTGCACTAATGGA |
| 165 | Absolute level index 6 probe | AACAACGACCGAGTGCATAGTCACGTACGA |
| 166 | Absolute level index 7 probe | AGGAGCCCTAAGGTATTGGCGAGAAAAGTC |
| 167 | Absolute level index 8 probe | CTGAGTATCCGCATATCTTCCGAGGTTGCA |
| 168 | Absolute level index 9 probe | ACTTAGCTGACCGAAGGACCATAACGCTGT |
| 169 | Absolute level index 10 probe | TGGAAGGGATCCGTAGTCAACCGTTGACTT |
| 170 | Absolute level index 11 probe | CGGATCGACATACGACGCCTACAGAATGTT |
| 171 | Absolute level index 12 probe | TAAACGTCTAGGCGAGACTATGAGTGCTCC |
| 172 | Absolute level index 13 probe | CGTATGGATCGATCCGACGTACCACATTAG |
| 173 | Absolute level index 14 probe | ACTGCGTATGATCGACACGGCTAATCGTAG |
| 174 | Absolute level index 15 probe | CTATTCGACCAGCGATATCACTACGTAGGC |

**[Table 2-2]**

| SEQ ID NO | Name | Sequence (5' to 3') |
|---|---|---|
| 175 | Absolute level index 1 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGAATGGTAAGGGTCGTATTAGGGTCGAACCTACACGACAATGCACGTCGAAGCGGTTGCTAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 176 | Absolute level index 2 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGTAGCCGATTGAACTAATGCCTTGCGTAGGTATGAACGTAGCTGCTAGTCGAGGCCTTGTATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 177 | Absolute level index 3 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGTCCTCGATACATACGAAATGTCGTCGACCGATTCGGTATCTCCTCTGGCACCGAAGACAAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 178 | Absolute level index 4 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGTGCTACGCTTTACGCTTGCCAATCGTTCACGACCTTCACGCAACACTTGATCGAACCGAAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 179 | Absolute level index 5 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGACAACAGACGGTACGTCCATTAGTGCAACGTTTGCTCAGTAGGGGGTCTAAGCGTCACTTATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 180 | Absolute level index 6 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGGAGCAGTTTTCTTCTCGTACGTGACTATGCACTCGGTCGTTGTTGGAATACCGGTCGTAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 181 | Absolute level index 7 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGAACGCAATACGGTGGGACTTTTCTCGCCAATACCTTAGGGCTCCTGTGTACCTAAGCGAAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 182 | Absolute level index 8 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGGATGACGGATAAGTTGCAACCTCGGAAGATATGCGGATACTCAGACGTGATATGCGCAGATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 183 | Absolute level index 9 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGGTGTACATCGGATGACAGCGTTATGGTCCTTCGGTCAGCTAAGTAAGTCCGTTTTCCACATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 184 | Absolute level index 10 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGTTCAGTCAACCGGAGAAGTCAACGGTTGACTACGGATCCCTTCCATGTAGAGCTCTCACAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 185 | Absolute level index 11 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGCGATTGTCGCGTTAAACATTCTGTAGGCGTCGTATGTCGATCCGGGACTTCGCTTCATAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 186 | Absolute level index 12 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGTCGTGTGTCGTGAGAGGAGCACTCATAGTCTCGCCTAGACGTTTATGACGAGATATCACGATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 187 | Absolute level index 13 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGTAGCGTGTAACGCACTAATGTGGTACGTCGGATCGATCCATACGCAACTTTGTACCGAGATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 188 | Absolute level index 14 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGAGCAACCATCTTGAGCTACGATTAGCCGTGTCGATCATACGCAGTCCGAGGCATTACTGAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |
| 189 | Absolute level index 15 | GTGAGAAGCCTACACAAACGTAACGTCAGGGCTAAGACAAACGCTAACGGTACACCCTAGATGGGAGCTTGTAGCTAGATCGCTAAGTCCTA CCGACATGTAGGCATACTCACGAAGGCAATTCCCTGAAAGCCTCGTCTTATCCCGAACTTGGCATCTGCTGATACGTCAGGTTGAACGCGTA CATTTACCTGTCATGCGTGGGCCTTCTCCGAATAGCCTACGTAGTGATATCGCTGGTCGAATAGGCGGATTGCTCATAAATGCACATTGGCT AAGGCCCACGGAACACGAATCACGTGAGATCACTTACTATTCGACGGAACTACTATACGCACCGGGACATGCAAGTAGCGTCCCACAAGCAT AAGGAACTCTATACTCGCCATCTACGCAGCTACAGGGGATACACGTATGAGCGGTTACGAAGTAAAGCCGAGATAGAGCGGTCTTTAGAGA |

A specific primer means that the sequence targeted for amplification can be limited, where the primer pair is not necessary a single pair. If necessary, a multiplex technique that uses two or more primer pairs may also be applied. Examples of the primer pairs are shown in Table 2-3. Primer pairs for bacterial amplification (SEQ ID NOS:192-204), and a primer pair for absolute level index (SEQ ID NOS:190 and 191) may be used.

According to a method for designing a primer of the present invention, first, at least one variable region showing diversity of bacteria targeted for analysis is selected, and then highly conserved universal primer design regions are selected to precede and succeed the selected variable region, thereby designing a primer sequence. While the targeted variable region is not limited, it may be 16S rRNA gene among the genomic sequence, which exists in all bacteria. Either full-length or one or more of the variable regions V1-V9 of 16S rRNA gene may favorably be targeted. More preferably, variable regions V3-V4 are targeted.

In order to assess the coverage of the primer, database based on acquisition of genomic sequences of a wide range of bacteria is utilized. Specifically, examples of such database include RDP, NCBI, KEGG and MGDB.

For example, an input of the designed universal primer sequence into RDP database ProbeMatch will give a list of results that shows the number of complete matches among the total search. The closer the number of complete matches to the total search is, the higher the coverage of the primer is. As to the conditions in this case, *Type* may be selected for Strain while *Isolates* may be selected for Source.

Specifically, the primers are preferably those having DNA of the nucleotide sequences represented by SEQ ID NOS:193-198 and SEQ ID NOS:201-204 (or nucleotide sequences complementary to said nucleotide sequences). These primers are preferable for having higher sequence homology to the periodontal disease bacteria group than the existing primers.

Specifically, the primers are those having DNA of the nucleotide sequences represented more preferably by SEQ ID NOS:193-198 and 203-204, still more preferably by SEQ ID NOS:197-198 and 203-204 and particularly preferably by SEQ ID NOS:198 and 204 (or nucleotide sequences complementary to said nucleotide sequences).

According to the above-described assessment, DNA of the nucleotide sequences represented by SEQ ID NOS:193-198 and 203-204 (or nucleotide sequences complementary to said nucleotide sequences) have been confirmed to have 100% sequence homology to the periodontal disease bacteria group.

In particular, a primer pair having DNAs of the nucleotide sequences represented by SEQ ID NOS:198 and 204 (or nucleotide sequences complementary to said nucleotide sequences) is preferable since homology between the primer sequences and the genomic DNA sequences of the periodontal disease bacteria group is 100%, and since the conditions, namely, the melting temperature (Tm) and the GC content match between the primer pair. Generally, designing of a probe pair requires close melting temperature (Tm) to minimize formation of a secondary structure.

**[Table 2-3]**

| SEQ ID NO | Primer name | Sequence (5' to 3') |
|---|---|---|
| 190 | Forward primer (for amplifying absolute level index) | GAGAAGCCTACACAAACGTAACGTC |
| 191 | Reverse primer (for amplifying absolute level index) | CTCTAAAGACCGCTCTATCTCGG |
| 192 | Cy5-Universal16S-FWD | TCCTACGGGAGGCAGCAGT |
| 193 | Cy5-Universal16S-FWD1 | TCCTACGGGAGGCAGCAG |
| 194 | Cy5-Universal16S-FWD2 | TCCTACGGGAGGCAGCA |
| 195 | Cy5-Universal16S-FWD3 | TCCTACGGGAGGCAGC |
| 196 | Cy5-Universal16S-FWD4 | CCTACGGGAGGCAGC |
| 197 | Cy5-Universal16S-FWD5 | CTACGGGAGGCAGCAG |
| 198 | Cy5-Universal16S-FWD6 | TACGGGAGGCAGCAG |
| 199 | Universal RVS | GGACTACCAGGGTATCTAATCCTGTT |
| 200 | Universal RVS2 2014 | CAGGGTATCTAATCCTGTTTGCTACC |
| 201 | Universal RVS1 2016 | CAGGGTATCTAATCCTGTTYG |
| 202 | Universal RVS2 2016 | CAGGGTATCTAATCCTGTT |
| 203 | Universal RVS3 2016 | GGGTATCTAATCCYGTT |
| 204 | Universal RVS4 2016 | CRGGGTATCTAATCCYGTT |

The absolute level index may be a standard reference material characterized for nucleic acids used in a quantitative analysis, which is developed by the National Institute of Advanced Industrial Science and Technology (AIST), or it may be newly designed. If the absolute level index is to be designed, for example, the RANDBETWEEN function of software "EXCEL" (from MICROSOFT) may be used, which can randomly return X number (X is a given number) of integers of 1 to 4. The resulting random integers may be linked in a row to give a X-digit number consisting only of 1 to 4 so as to obtain a large number of sequences having X bases of random ATGC by converting 1 to A, 2 to T, 3 to C and 4 to G.

From these sequences, only sequences that have the sum of G and T equal to the sum of A and T are picked out. The picked sequences are subjected to Blast search against database such as GenBank at NCBI so as to select less homologous sequences among organism-derived nucleic acids. Primer sequences are allowed to flank both ends of the sequence, thereby designing a sequence. Moreover, the designed sequence may suitably be linked for extension or partially removed for shortening.

In order to keep the reaction efficiency upon amplification reaction as constant as possible, the difference between the amplified base length of the bacterium targeted for detection and the amplified base length of the absolute level index is preferably made small. For example, if the amplified product of the bacterium targeted for detection is about 500 bp, the amplified product of the absolute level index is preferably made to be about 300 bp to 1000 bp.

On the other hand, if the amplified chain length is to be confirmed by electrophoresis or the like after the amplification, the amplified product of the absolute level index is designed to have a length that differs from the length of that of the bacterium targeted for detection so that the absolute level index-derived amplified product can be detected at a location separated from the band for the bacterium targeted for detection, thereby confirming success/failure of the amplification reaction prior to hybridization.

Finally, if the concentration of the absolute level index contained in the specimen is too high, competition with the bacteria targeted for detection becomes intense upon the amplification reaction and the bacteria targeted for detection that are supposed to be detected may not be detected. Therefore, there is a need to suitably adjust the concentration according to the application.

In order to design a probe used for the present invention, stringency upon hybridization needs to be considered. The stringency can be made high to a certain level so that even if a similar nucleotide sequence region exists among certain regions of respective nucleic acids of various intraoral bacteria, hybridization can take place by distinguishing other regions. On the other hand, if the nucleotide sequences of said certain region differ from each other, the stringency may be set lower.

As such stringency conditions, for example, highly stringent conditions refer to hybridization under the conditions of 50-60°C while lowly stringent conditions refer to hybridization under the conditions of 30-40°C. Examples of stringent conditions for hybridization include conditions such as "0.24M Tris-HCl/0.24M NaCl/0.05% Tween-20, 40°C", "0.24M Tris-HCl/0.24M NaCl/0.05% Tween-20, 37°C" and "0.24M Tris-HCl/0.24M NaCl/0.05% Tween-20, 30°C". Examples of more stringent conditions include conditions such as "0.24M Tris-HCl/0.24M NaCl/0.05% Tween-20, 50°C", "0.24M Tris-HCl/0.24M NaCl/0.05% Tween-20, 55°C" and "0.06M Tris-HCl/0.06M NaCl/0.05% Tween-20, 60°C". More specifically, in one method, the probe may be added and kept at 50°C for an hour or longer to form a hybrid, which is subsequently washed with 0.24M Tris-HCl/0.24M NaCl/0.05% Tween-20 at 50°C for 20 minutes for four times, and finally washed with 0.24M Tris-HCl/0.24M NaCl at 50°C for 10 minutes once. Stringent conditions can be set higher by increasing the temperature upon hybridization or washing. In addition to such conditions like salt concentration, temperature and the like of the buffer, those skilled in the art would also be capable of determining other conditions such as probe concentration, probe length, reaction time and the like. For a detailed procedure of the hybridization method, see "Molecular Cloning, A Laboratory Manual 4th ed." (Cold Spring Harbor Press (2012), "Current Protocols in Molecular Biology" (John Wiley & Sons (1987-1997)), and the like.

While the length of the probe used for the present invention is not limited, it is, for example, preferably 10 bases or longer, more preferably 16-50 bases, and still more preferably 18-35 bases. As long as the length of the probe is appropriate (as long as within the above-mentioned range), it can be used for specific detection while suppressing non-specific hybridization (mismatch).

For designing the probe used for the present invention, it is preferable to confirm Tm. Tm refers to the temperature at which 50% of a nucleic acid strand will form a hybrid with its complementary strand. Thus, the temperature for hybridization needs to be optimized so that template DNA or RNA forms a double strand with its probe for hybridization. If this temperature is too low, non-specific reaction is likely to result, and therefore the temperature is preferably as high as possible. Accordingly, Tm of the nucleic acid fragment to be designed is an important factor for hybridization. Tm can be confirmed by utilizing a known probe designing software, where exemplary software that can be used for the present invention includes Probe Quest (registered trademark; Dynacom). Alternatively, Tm can also be confirmed by calculating by own without using a software. In this case, calculation based on nearest neighbor base pair method (Nearest Neighbor Method), Wallance method, GC% method or the like can be utilized. Average Tm of the probe of the present invention is preferably, but not limited to, about 35-70°C or 45-60°C. Other conditions for allowing specific hybridization with a probe include the GC content and the like, which are well known to those skilled in the art.

Furthermore, nucleotides constituting a probe used for the present invention may be any of DNA, RNA or PNA, or a hybrid of two or more types of DNA, RNA and PNA.

Specifically, examples of the probe used for the present invention preferably include those containing the nucleotide sequence of DNA (d) or (e) below. For example, when the primers shown in Table 2-3 (SEQ ID NOS:190-204) are used for amplification, the sequences shown in Table 2-1 (SEQ ID NOS:93-174) above can be used as the probes, where at least two sequences selected from the nucleotide sequences represented by SEQ ID NOS:1-82 are preferably used. Alternatively, they may be complementary sequences of at least two sequences selected from the nucleotide sequences represented by SEQ ID NOS:93-174, sequences substantially identical to at least two sequences selected from the nucleotide sequences represented by SEQ ID NOS:93-174, or sequences substantially identical to complementary sequences of at least two sequences selected from the nucleotide sequences represented by SEQ ID NOS:93-174.

Herein, substantially identical means that the sequence specifically hybridizes with the sequence represented by any of SEQ ID NOS:93-174 or with the complementary sequence thereof under stringent conditions.
(d) DNA consisting of a nucleotide sequence represented by any of SEQ ID NOS:93-174.
(e) DNA that hybridizes with DNA consisting of a nucleotide sequence complementary to DNA (d) above under stringent conditions, and that has a function of detecting at least a part of a nucleotide sequence of nucleic acids derived from an intraoral bacterium

As the probe, those represented by SEQ ID NOS:97, 100, 120, 121, 122, 129, 130, 152 and 155 are preferable. Those represented by SEQ ID NOS:97, 100, 121, 122, 129 and 130 are preferable in that hydrogen bonding with specimen-derived DNA can be promoted by extending the base length of the probe sequence and enhancing the melting temperature (Tm).

Among them, those represented by SEQ ID NOS:121, 122, 129 and 130 are preferable in that multiple nucleotide sequences are established to accommodate single nucleotide polymorphisms with reference to the genomic DNA sequences of multiple strains of the bacteria targeted for detection.

Moreover, those represented by SEQ ID NOS:120, 152 and 155 are preferable in that regions with higher specificity are further selected with reference to the genomic DNA sequences of the bacteria targeted for detection. These primers are preferable for having higher sequence homology to the periodontal disease bacteria group than the existing primers.

For the respective DNAs (d) above, their specific nucleotide sequences, probe names and intraoral bacteria targeted for detection are shown in Table 2-1 above. Here, probe for *Streptococcus* spp. indicates the sum of the bacterial levels of *Streptococcus* *constellatus, Streptococcus gordonii, Streptococcus mitis, Streptococcus oralis, Streptococcus sanguinis* and the like.

Furthermore, probe 1 for *Capnocytophaga* spp. indicates the sum of the bacterial levels of *Capnocytophaga gingivalis, Capnocytophaga sputigena* and the like, probe 2 for *Capnocytophaga* spp. indicates the sum of the bacterial levels of *Capnocytophaga gingivalis, Capnocytophaga ochracea* and the like. Probe for *Fusobacterium nucleatum* indicates the sum of the bacterial levels of *Fusobacterium nucleatum* subsp. *vincentii, Fusobacterium nucleatum* subsp. *polymorphum, Fusobacterium nucleatum* subsp. *animalis, Fusobacterium nucleatum* subsp. *nucleatum* and the like. Probes 2 and 3 for *Campylobacter* spp. indicate a sum of the bacterial levels of *Campylobacter rectus, Campylobacter showae* and the like.

DNA (e) above can be obtained from a cDNA library or a genomic library by carrying out a known hybridization method such as colony hybridization, plaque hybridization or southern blotting, using any of DNAs (d) above, DNA consisting of a nucleotide sequence complementary thereto, or a fragment thereof as a probe. The library is not limited, and a library generated by a known method or a commercially available cDNA library or genomic library may be utilized. For a detailed procedure of the hybridization method, see references mentioned above. DNA to be hybridized is preferably a nucleotide sequence having at least 60% or more, more preferably 80% or more, still more preferably 90% or more, yet more preferably 95% or more, particularly preferably 98% or more and most preferably 99% or more homology to the nucleotide sequence of DNA (d) above.

The probe used for the present invention can be prepared, for example, through chemical synthesis employing a general oligonucleotide synthesis method (purification can be carried out by HPLC or the like). Such a probe can be designed, for example, by Probe Quest (registered trademark: from Dynacom). In addition, the probe of the present invention may also contain, for example, an additional sequence such as a tag sequence.

According to the method of the present invention, the nucleotide sequence of the nucleic acids of the above-described intraoral bacterium targeted for detection does not have to be the exact nucleotide sequence thereof, and it may have partial mutations such as deletion, substitution or insertion in the nucleotide sequence. Accordingly, the nucleotide sequence of the nucleic acids targeted for detection may also be a mutant gene that can hybridize with a sequence complementary to said nucleotide sequence under stringent conditions and that has functions and activity originating from said nucleotide sequence, and thus a probe can also be designed based on a nucleotide sequence of such mutant gene. Herein, "stringent conditions" may refer to the application of the conditions similar to those described above.

### 2. DNA chip for detecting genes of intraoral bacteria to measure levels of intraoral bacteria

As described above, a DNA chip can be used in the method of the present invention, where this DNA chip has a plurality of various oligonucleotide probes described in Item 1 above arranged on a substrate that serves as a support.

The substrate that serves as a support may be in any form of a flat plate (glass plate, resin plate, silicon plate, etc.), a stick, beads or the like. When a flat plate is used as the support, predetermined probes can be fixed thereon by types at predetermined intervals (spotting method, etc.; see Science 270, 467-470 (1995), etc.). Alternatively, predetermined probes can sequentially be synthesized thereon by types at specified positions (photolithography method, etc.; see Science 251, 767-773 (1991), etc.). Another preferable form of the support may be, for example, one that uses hollow fibers. When hollow fibers are used as a support, a preferable exemplary DNA chip (hereinafter, referred to as a "fiber-type DNA chip") can be obtained by fixing predetermined probes in respective hollow fibers by types, bundling and fixing all of the hollow fibers, and then repeatedly cutting the fibers with respect to the longitudinal direction of the fibers. This microarray may also be referred to as a microarray that has nucleic acids fixed in through holes of a substrate, which is also referred to as a so-called "through-hole-type DNA chip" (see Japanese Patent Publication No. 3510882).

The method for fixing probes onto a support is not limited, and any binding mode can be employed. Moreover, fixing is not limited to direct fixing onto the support. For example, a support may be pre-coated with a polymer such as polylysine so that probes may be fixed onto the treated support. Furthermore, when a tubular body such as hollow fibers is used as a support, the tubular body may be made to retain a gel-like substance so that probes can be fixed to the gel-like substance.

Hereinafter, a fiber-type DNA chip, one form of DNA chips, will be described in detail. This DNA chip can be prepared, for example, through Steps (i)-(iv) below.
(i) Step of three-dimensionally arranging a plurality of hollow fibers such that the hollow fibers are oriented in the same longitudinal direction to produce an arranged body.
(ii) Step of embedding the arranged body to produce a block body.
(iii) Step of introducing a gel-precursor polymerizable solution containing an oligonucleotide probe into a hollow of each hollow fiber of the block body to allow polymerization reaction, thereby making the hollow to retain the gel-like substance containing the probe.
(iv) Step of cutting the hollow fibers in a direction intersecting with the longitudinal direction thereof to slice the block body.

An example of the material used as the hollow fiber preferably include, but not limited to, a material described in Japanese Unexamined Patent Application Publication No. 2004-163211.

The hollow fibers are three-dimensionally arranged such that their lengths are equal in the longitudinal direction (Step (i)). The method employed for the arrangement may be, for example, a method in which a plurality of hollow fibers are arranged in parallel at predetermined intervals on a sheet-like material such as an adhesive sheet to form a sheet, which is thereafter wound up in spiral (see Japanese Unexamined Patent Application Publication No. *Heisei* 11-108928), or a method in which two porous plates provided with a plurality of pores at predetermined intervals are layered such that the pores meet each other, where hollow fibers are passed through the pores and then the two porous plates are temporary fixed at a distance and a curable resin material is charged and cured around the hollow fibers between the two porous plates (see Japanese Unexamined Patent Application Publication No. 2001-133453).

The produced arranged body is embedded so that the arrangement is not disordered (Step (ii)). The method of embedment may preferably be a method in which a polyurethane resin, an epoxy resin or the like is poured into the gap between the fibers, a method in which the fibers are adhered to each other by heat welding, or a method likewise.

The hollow of each hollow fiber of the embedded arranged body is filled with a gel-precursor polymerizable solution (gel-forming solution) containing the oligonucleotide probe to allow polymerization reaction in the hollow (Step (iii)). As a result, the hollow of each hollow fiber can retain the gel-like substance having the probe fixed thereto.

The gel-precursor polymerizable solution refers to a solution containing a reactive substance such as a gel-forming polymerizable monomer, where said monomer or the like can be polymerized/crosslinked so that the solution becomes a gel-like substance. Examples of such monomer include acrylamide, dimethylacrylamide, vinylpyrrolidone and methylene-bis-acrylamide. In this case, the solution may contain a polymerization initiator and the like.

After fixing the probes in the hollow fibers, the block body is cut into slices in a direction intersecting with (preferably perpendicular to) the longitudinal direction of the hollow fibers (Step (iv)). The resulting slice can be used as a DNA chip. The thickness of this DNA chip is preferably about 0.01 mm-1 mm. The block body can be cut, for example, with a microtome, a laser or the like.

Preferable examples of the above-described fiber-type DNA chip include DNA chip (Genopal™) from Mitsubishi Rayon and the like.

In the fiber-type DNA chip, the probes are three-dimensionally arranged in the gel as described above so as to maintain a three-dimensional structure. Accordingly, the detection efficiency is enhanced as compared to a flat DNA chip that has probes bound onto a coated surface of a glass slide, and thus a highly sensitive and highly reproducible examination can be realized.

The number of types of probes arranged on the DNA chip is 500 types or less, preferably 250 types or less and more preferably 100 types or less per DNA chip. By limiting the number (types) of the arranged probes to some extent, the intraoral bacteria of interest can be detected with high sensitivity. The types of the probes are distinguished by the nucleotide sequences. Therefore, usually, even probes that are derived from the same gene are specified as different types of probes even if a single difference exists between the nucleotide sequences.

### 3. Detection of gene of intraoral bacterium

According to the method of the present invention, the method of detecting a gene of an intraoral bacterium for measuring the level thereof comprises the following steps.
(i) Step of extracting nucleic acids from a specimen, namely, an intraoral sample collected from a subject.
(ii) Step of allowing the extracted nucleic acids to make contact with the oligonucleotide probe of the present invention or the DNA chip of the present invention described above.
(iii) Step of calculating the level of the bacterium based on the signal intensity obtained from the DNA chip.

Hereinafter, the steps of the detection method will be described one by one in detail.

### (1) About Step (i)

In this step, an intraoral sample collected from a subject human or organism is used as a specimen, where the nucleic acids of the bacterium contained in the specimen are extracted. The type of the intraoral sample collected is not particularly limited. For example, saliva, plaque (subgingival plaque, supragingival plaque), tongue fur, mousewash or the like may be used, among which plaque is preferable, and subgingival plaque collected from a habitat of the largest number of periodontal disease bacteria is more preferable.

The method of collecting the intraoral sample is not particularly limited, and it may suitably be selected according to the type of the sample. For example, in a case where saliva is used as the intraoral sample, a method that utilizes a commercially available saliva collection kit, a method in which a cotton swab is placed in the mouse to collect saliva, a method in which saliva is directly collected in a container, or the like may be employed.

In a case where plaque is used as the intraoral sample, brushing of the tooth surface or the interdental area with a tooth brush, scratching of the tooth surface with a cotton swab, scratching of the interdental area with an interdental brush, a paper point technique, or the like may be employed. The tooth brush, the cotton swab, the interdental brush or the paper point used for collecting the plaque is immersed in sterilized water and agitated if necessary to dissolve or suspend the plaque. The resulting solution or suspension may also be used as the specimen. The amount of plaque to be collected is not particularly limited, and it may, for example, be an amount that can be collected with a single paper point.

In a case where tongue fur is used as the intraoral sample, scratching of the tongue surface with a cotton swab may be employed. The cotton swab used for collecting the plaque is dissolved or suspended so as to use the resulting solution or suspension as the specimen. The amount of tongue fur to be collected is not particularly limited, and it may, for example, be an amount that can be collected with a single cotton swab.

In a case where a mousewash is used as the intraoral sample, the mousewash or water is kept in the mouse to collect saliva in a container together with the mousewash or water to use the obtained solution as the specimen. The mousewash may, for example, be sterilized physiological saline or the like.

Next, nucleic acids are extracted from the bacteria present in the obtained intraoral sample. The extraction method is not limited and any known method can be employed. For example, the method may be an automatic extraction method using an instrument, a method utilizing a commercially available nucleic acid extraction kit, a method in which a treatment with proteinase K is followed by phenol extraction, a method utilizing chloroform, a simple extraction method by heating or dissolving the sample, or the like. Alternatively, one may proceed to the next step without extracting nucleic acids from the specimen.

Without limitation, the nucleic acids obtained from the specimen may be allowed to make direct contact with the DNA chip or the like, or a nucleotide sequence region desired may be amplified by PCR or the like so as to allow the amplified fragments thereof to make contact with the DNA chip or the like. The region to be amplified using the obtained nucleic acids as a template is a site coding for a nucleic acid region containing the nucleotide sequence of the probe or the oligonucleotide arranged on the DNA chip used in the present invention. The region desired to be amplified is not limited and a nucleotide sequence of a highly conserved region for all kinds of intraoral bacteria can be utilized to amplify a mixture of various kinds of intraoral bacteria at once. The sequence used for such amplification can be determined based on analyses of nucleotide sequences of an experimentally isolated and purified polynucleotide, or can be determined *in Silico* by searching and aligning a nucleotide sequence known from database of nucleotide sequences or the like. The database of nucleic acids or amino acids may be, for example, without limitation, DDBJ (DNA Data Bank of Japan), EMBL (European Molecular Biology Laboratory, EMBL nucleic acid sequence data library), GenBank (Genetic sequence data bank), Taxonomy database from NCBI (National Center for Biotechnology Information), or the like.

Specifically, the site desired to be amplified is preferably ribosome RNA (16S rRNA) gene in chromosomal DNAs of intraoral bacteria. PCR primers that can be used for amplification of this region may preferably be, for example, SEQ ID NOS:190-204 shown in Table 2-3. The amplification of the nucleic acids by PCR method can be carried out by a common method.

The extracted nucleic acids and the amplified fragments thereof in this step may suitably be labeled so as to be used in the detection process following hybridization. Specifically, a method in which the terminal of the PCR primer is labeled with a reporter dye, a method in which a reactive nucleotide analog is incorporated upon reverse transcription reaction, a method in which biotin-labeled nucleotides are incorporated, and the like may be contemplated. Furthermore, labeling can be carried out through reaction with a fluorescently labeled reagent after preparation. As the fluorescent reagent, for example, various kinds of reporter dyes (e.g., Cy5, Cy3, VIC, FAM, HEX, TET, fluorescein, FITC, TAMRA, Texas red, Yakima Yellow, etc.) can be used.

### (2) About Step (ii)

In this step, the nucleic acids or the amplified fragments thereof obtained in Step (i) are allowed to make contact with the probes or the DNA chip used for the present invention. Specifically, a hybridization solution containing said nucleic acids or the like is prepared so as to allow the nucleic acids or the like in said solution to bind (hybridize) with the oligonucleotide probe mounted on the DNA chip. The hybridization solution can suitably be prepared using a buffer such as SDS or SSC according to a common method.

The hybridization reaction can be carried out by suitably the setting reaction conditions (type, pH, temperature and the like of the buffer) such that the nucleic acids or the like in the hybridization solution can hybridize with the oligonucleotide probe mounted on the DNA chip under stringent conditions. The term "stringent conditions" as used herein refers to conditions that are less likely to result cross-hybridization induced by similar sequences, or that can dissociate any nucleic acids cross-hybridized with similar sequences. Specifically, it refers to the conditions for washing the DNA chip upon or after the hybridization reaction.

For example, the conditions upon hybridization reaction may be a reaction temperature of preferably 35-70°C, more preferably 40-65°C and hybridization time of preferably about 1 minutes to 16 hours.

Moreover, as the conditions for washing the DNA chip after the hybridization, washing liquid composition is preferably 0.24M Tris-HCl/0.24M NaCl/0.05% Tween-20, and a temperature upon washing is preferably 35-80°C or 40-65°C, more preferably 45-60°C. More specifically, the conditions preferably include a salt (sodium) concentration of 48-780 mM and a temperature of 37-80°C, and more preferably include a salt concentration of 97.5-390 mM and a temperature of 45-60°C.

After washing, the detected intensity of each spot is measured with a device that can detect the label of the nucleic acids or the like bound to the probe. For example, if the above-described nucleic acids or the like are fluorescently labeled, a fluorescence detector such as CRBIO (from Hitachi Software Engineering), arrayWoRx (from GE Healthcare), Affymetrix 428 Array Scanner (from Affymetrix), GenePix (from Axon Instruments), ScanArray (from PerkinElmer) or Genopal Reader (from Mitsubishi Rayon) can be used to measure the fluorescence intensity. In a case where this device is a fluorescence scanner, scanning can be performed by suitably adjusting, for example, the laser output and sensitivity of the detection section, whereas in a case where this device is a CCD camera type scanner, scanning can be performed by suitably adjusting the exposure time. A quantitative method based on the scan results can be performed with a quantification software. The quantification software is not particularly limited, and the average value, the median or the like of the fluorescence intensity of the spot can be used for quantification. Furthermore, considering the dimensional accuracy of the spot area of the DNA fragment and the like, adjustment is preferably performed upon quantification, for example, using the fluorescence intensity of the spot having no probe as background.

### (3) About Step (iii)

In this step, the bacterial level of the bacterial species targeted for detection is calculated from the signal intensity acquired by the above-described procedure. For example, it can be expressed as a signal-to-noise ratio based on the signal intensity of the probe for detecting the bacterium targeted for detection and the background signal intensity. Alternatively, it may be preferable to conduct detections for each bacterium under a plurality of conditions by varying the bacterial chromosomal DNA concentration so as to acquire a conversion factor (standard curve) for each bacterium in advance for calculating the chromosomal DNA concentration based on the signal intensity obtained under each concentration condition, by which chromosomal DNA concentrations can be calculated from signal intensities obtained under the respective conditions.

In either case, a correction coefficient may be calculated for each DNA chip so that signal intensities of an absolute level index probe acquired by detection of a plurality of DNA chips will be constant. Accordingly, comparison can be made among the DNA chips by taking the correction coefficient into account for a signal intensity of a bacterium targeted for detection from each DNA chip.

### 4. Assessment of severity of periodontal disease

Besides periodontal disease patients, severity of periodontal disease can also be assessed based on the bacterial levels of respective intraoral bacteria for those unaware of having periodontal disease, for patients with systemic diseases suggested to be relative to periodontal disease such as heart disease, or for pregnant women.

Specifically, according to this assessment, the pattern of the detected labeling substance on the DNA chip used for detection is considered to indicate the characteristics of the intraoral bacteria of the subject. According to another aspect of assessment, a statistical analysis of the patterns of the detected labeling substance on the DNA chip used for detection can be conducted for multiple specimens.

Examples of the statistical analysis include correlation analysis, hierarchical clustering and non-hierarchical clustering. More specifically, a distance function used in the correlation analysis may be derived from a technique such as Pearson correlation or Cosine coefficient. Meanwhile, the hierarchical clustering may be a technique such as UPGMA (unweighted-pair group method using arithmetic averages). Such an analysis technique can be used for a cluster analysis so that subjects can be grouped, and analogy of the subjects can further be assessed.

According to the present invention, the results of the detected intraoral bacteria in the specimen are subjected to a statistical analysis together with the detection results that have been accumulated, so that they can be classified based on similarity into any of severity groups corresponding to periodontal disease specimens, early periodontal disease specimens or normal specimens. The results of this classification, that is, the bacterial levels of the specimen, may suggest latent disease activity. In addition, based on these results, the bacterial levels of specified multiple kinds of bacteria can be used as independent variables in a regression analysis so as to calculate prediction accuracy for the prediction of grouping by severity of periodontal disease and for the prediction of periodontal pocket depth.

Since the severity of periodontal disease can accurately be assessed from information of multiple bacteria according to the first aspect of the method of the present invention, it can be an objective index for indicating the severity of periodontal disease.

### 5. Assessment of effect of treating periodontal disease

The above-described second aspect of the method of the present invention, namely, a method for assessing the effect of treating periodontal disease, uses the results obtained by the method for detecting genes of intraoral bacteria described in Item 3 above as an index.

When specimens are collected before and after the periodontal disease treatment for comparative examination, the therapeutic effect can objectively be assessed by finding out the bacterium that increased or decreased before and after the treatment. Moreover, the bacterium that cannot easily be reduced by the treatment can be revealed from the bacterial data after the treatment, so that it can be treated specifically.

A treatment refers to treatments generally practiced by dentists and dental hygienists on the site. Examples of such treatments include plaque control (tooth brushing guidance), removal of dental calculus (scaling and root planing) and occlusal adjustment as basic periodontal therapies. Furthermore, a surgical treatment can be carried out when the dental calculus stays deep in the pocket and cannot be removed as found by a re-evaluation examination following the basic periodontal therapy, which may be flap surgery, periodontal tissue regeneration therapy, plastic surgery (periodontal plastic surgery), or the like.

Specifically, according to the assessment, the pattern of the detected labeling substance on the DNA chip used for detection is considered to indicate the characteristics of the intraoral bacteria of the subject. In addition, another aspect of assessment may be to perform a statistical analysis of the patterns of the detected labeling substance on the DNA chips used for detection, for multiple specimens.

A method of the statistical analysis may, for example, be a t-test or a non-parametric correlation analysis. More specifically, there are also techniques such as Student's t-test and Welch's t-test for unequal variances. Such an analysis technique can be used for significance tests of the clinical information and the bacterial levels of the specimen, thereby assessing therapeutic effects.

The bacterial level before the treatment can be compared with the bacterial level after the treatment so that the effect of treating periodontal disease can be assessed to be present when the comparison results shows that the total bacteria level or at least one of the bacterial levels of *Porphyromonas gingivalis, Tannerella forsythia, Treponema denticola, Campylobacter rectus, Fusobacterium nucleatum, Prevotella intermedia, Prevotella nigrescens, Aggregatibacter actinomycetemcomitans* and *Capnocytophaga gingivalis* is decreased.

Moreover, the therapeutic effect is also assessed to be present when the proportion of the *Streptococcus* spp. to the total bacteria level is increased as compared to the proportion of the same before the treatment.

On the other hand, the effect of treating periodontal disease is assessed to be absent when the comparison results before and after the treatment show that the total bacteria level and any bacterial level of *Porphyromonas gingivalis, Tannerella forsythia, Treponema denticola, Campylobacter rectus, Fusobacterium nucleatum, Prevotella intermedia, Prevotella nigrescens, Aggregatibacter actinomycetemcomitans,* and *Capnocytophaga gingivalis* are not decreased.

According to the second aspect of the method of the present invention, the effect of treating periodontal disease can be assessed from information of a plurality of bacteria, which can serve as an objective index of the effect of treating periodontal disease.

### 6. Assessment of risk of exacerbation of periodontal disease

According to the above-described third aspect of the method of the present invention, a method for assessing the risk of exacerbation of periodontal disease uses the results obtained by the method for detecting genes of intraoral bacteria described in Item 3 above as an index.

For example, risk of exacerbation can be assessed and monitored on a regular basis for a single subject. Besides periodontal disease patients, severity of periodontal disease can also be assessed based on the bacterial levels of respective intraoral bacteria for those unaware of having periodontal disease, for patients with systemic diseases suggested to be relative to periodontal disease such as heart disease, or for pregnant women.

If a specimen has few or none of highly malignant periodontal disease bacteria detected but has a significantly high bacterial level of a specific bacterium, it is judged to have a risk of exacerbation. Exacerbation means that the periodontal pocket depth will become deeper, or the bacterial level of a highly malignant periodontal disease bacterium will become higher.

Specifically, according to this assessment, the pattern of the detected labeling substance on the DNA chip used for detection is considered to indicate the characteristics of the intraoral bacteria of the subject. According to another aspect of assessment, a statistical analysis of the patterns of the detected labeling substance on the DNA chip used for detection can be conducted for multiple specimens.

According to the present invention, the risk of exacerbation of periodontal disease could be assessed to be high if few or none of *Porphyromonas gingivalis, Tannerella forsythensis* and *Treponema denticola* is detected while a significant bacterial level of *Fusobacterium nucleatum* is detected upon intraoral bacteria detection, for example, if the ratio of the bacterial level of *Fusobacterium nucleatum* to the total bacterial level of *Porphyromonas gingivalis, Tannerella forsythia* and *Treponema denticola* is large.

On the other hand, the risk of exacerbation of periodontal disease could be assessed to be low if none of *Porphyromonas gingivalis, Tannerella forsythensis* or *Treponema denticola* is detected and a bacterial level of *Fusobacterium nucleatum* is not significant, or if the ratio of the bacterial level of *Fusobacterium nucleatum* to the total bacterial level of *Porphyromonas gingivalis, Tannerella forsythia* and *Treponema denticola* is small.

Since the third aspect of the method of the present invention is capable of accurately assessing the risk of exacerbation of periodontal disease from the information of multiple bacteria, it can be utilized for a prophylactic purpose.

Hereinafter, the present invention will be described more specifically by way of examples, although the present invention should not be limited thereto.

### [Example 1-1]

Assessment of status of periodontal disease/dental caries using saliva specimen

### <Preparation of DNA>

With the cooperation of five healthy adults (males and females in their 20s to 50s), a saliva assessment test was conducted. The saliva of the five adults, i.e., Subjects A, B, C, D and E, were assessed.

Saliva was collected by placing γ-collection swab RI (Eiken Chemical) in the mouse for a minute. The γ-collection swab RI was immersed in water in a tube and left at room temperature for 5 minutes to elute the bacterial components. Thereafter, the γ-collection swab RI was removed and the tube was placed in a centrifuge. DNAs were extracted from the resulting pellets using DNeasy Blood & Tissue Kit (QIAGEN).

### <Evaluation of DNA>

The DNAs were diluted to about 10 pg and subjected to PCR using GeneAmp 9700 (Applied Biosystems) for amplification and labeling. As primers, SEQ ID NOS:1, 2, 91 and 92 were used. Composition of the PCR liquid (20 µL system) was as shown in Table 1-3.

**[Table 1-3]**

| | Final concentration |
|---|---|
| Water | - |
| Control DNA | 0.5 pg |
| Primer for control DNA (F) | 0.25 µM |
| Primer for control DNA (R) | 0.25 µM |
| Primer for bacterium (F) | 0.25 µM |
| Primer for bacterium (R) | 0.25 µM |
| Saliva-derived DNA | 10 pg |
| Ex taq | - |

PCR conditions were as shown in Table 1-4.

**[Table 1-4]**

| Ramp max, liquid amount 20 ul | | | | | |
|---|---|---|---|---|---|
| 95°C | 95°C | 56°C | 72°C | 40°C | 4°C |
| 1 min | 10 sec | 30 sec | 20 sec | 8 min | Unlimited |
| | 40 Cycles | | | | |

At the end of PCR, 180 µL of a hybridization reaction solution (48 µL of 1M Tris-HCl, 48 µL of 1M NaCl, 20 µL of 0.5% Tween 20, 65 µL of water) was added respectively. As a hybridization pretreatment, the resultant was allowed to react in GeneAmp 9700 at 94°C for a minute and left at 4°C.

In an automatic hybridization/washing machine (Mitsubishi Rayon), hybridization took place at 50°C for 2 hours followed by washing.

Probes mounted on the DNA chip used for measurement were as indicated in Table 1-5.

**[Table 1-5]**

| SEQ ID NO | Role | Other bacterium targeted for detection | Sequence (5' to 3') |
|---|---|---|---|
| 3 | *Porphyromonas gingivalis* (P.g.) probe | | TTCAATGCAATACTCGTATC |
| 5 | *Tannerella forsythia* (T.f.) probe | | CACGTATCTCATTTTATTCC |
| 8 | *Treponema denticola* (T.d.) probe | | CCTCTTCTTCTTATTCTTCAT |
| 12 | *Campylobacter rectus* (C.r.) probe | C.s. | GTCATAATTCTTTCCCAAGA |
| 20 | *Fusobacterium nucleatum* subsp. *nucleatum* (F.n.) probe | | TACATTCCGAAAAACGTCAT |
| 26 | *Prevotella intermedia* (P.i.) probe | | GGGTAAATGCAAAAAGGCA |
| 28 | *Prevotella nigrescens* (P.n.) probe | | CTTTATTCCCACATAAAAGC |
| 32 | *Aggregatibacter actinomycetemcomitans* (A.a.) probe | | GTCAATTTGGCATGCTATTA |
| 34 | *Campylobacter concisus* (C.c.) probe | | CCCAAGCAGTTCTATGGT |
| 44 | *Streptococcus gordonii* (S.g.) probe | S.s. | CACCCGTTCTTCTCTTACA |
| 47 | *Streptococcus intermedius* (S.i.) probe | | ACAGTATGAACTTTCCATTCT |
| 59 | *Streptococcus mutans* (S.mu.) probe | | CACACGTTCTTGACTTAC |
| 60 | Total bacterial level probe (16S rRNA consensus seq.) | | CGTATTACCGCGGCTGCTGGCAC |
| 75 | Control DNA 15 probe | | CTATTCGACCAGCGATATCACTA CGTAGGC |

For detection, Genopal Reader (Mitsubishi Rayon) was used with exposure times of 0.1, 1, 4 and 40 seconds.

### <Results>

The results of the bacterial counts in 1 mL of saliva are shown in Table 1-6.

**[Table 1-6]**

| Bacterial count per 1 ml of saliva | | | | | | |
|---|---|---|---|---|---|---|
| | | Subject A | Subject B | Subject C | Subject D | Subject E |
| Probe name | *Porphyromonas gingivalis* | 0 | 0 | 3577812 | 0 | 339241 |
| | *Tannerella forsythensis* | 0 | 0 | 0 | 0 | 0 |
| | *Treponema denticola* | 0 | 0 | 0 | 0 | 0 |
| | *Campylobacter rectus* | 0 | 0 | 0 | 0 | 0 |
| | Genus *Fusobacterium* | 9035535 | 4287560 | 3604766 | 0 | 0 |
| | *Prevotella intermedia* | 0 | 0 | 0 | 0 | 0 |
| | *Prevotella nigrescens* | 0 | 0 | 0 | 0 | 0 |
| | *Aggregatibacter actinomycetemcomitans* | 0 | 0 | 0 | 0 | 0 |
| | *Campylobacter concisus* | 0 | 5008244 | 0 | 0 | 0 |
| | *Streptococcus gordonii* | 905281 | 564191 | 366040 | 0 | 106759 |
| | *Streptococcus intermedius* | 27894569 | 0 | 0 | 0 | 0 |
| | *Streptococcus mutans* | 0 | 18862 | 0 | 17736 | 18808 |
| | Total bacterial level | 358280399 | 288932869 | 206229745 | 34420486 | 81926337 |

### <Assessments>

### (1) Assessment of status of periodontal disease

For Subject A, the total bacterial count of the three kinds of bacteria, namely, *Porphyromonas gingivalis, Tannerella forsythensis* and *Treponema denticola* was 0, and their proportion to the total bacteria level was 0%.

According to the criteria where periodontal disease is assessed to be "mild" if the total bacterial count of at least one of *Porphyromonas gingivalis, Tannerella forsythensis* and *Treponema denticola* among the bacteria contained in the intraoral sample is less than 0.05% to the total bacterial count, "moderate" if it is 0.05% or more but less than 0.5% to the total bacterial count, and "severe" if it is 0.5% or more to the total bacterial count (assessment criteria 2 described herein), Subject A was assessed with "mild" periodontal disease.

For Subject B, the total bacterial count of the three kinds of bacteria, namely, *Porphyromonas gingivalis, Tannerella forsythensis* and *Treponema denticola* was 0, and their proportion to the total bacteria level was 0%.

According to the same criteria as Subject A, Subject B was assessed with "mild" periodontal disease.

For Subject C, the total bacterial count of the three kinds of bacteria, namely, *Porphyromonas gingivalis, Tannerella forsythensis* and *Treponema denticola* was 3,577,812, and their proportion to the total bacteria level was 1.7%.

According to the same criteria as Subject A, Subject C was assessed with "severe" periodontal disease.

For Subject D, the total bacterial count of the three kinds of bacteria, namely, *Porphyromonas gingivalis, Tannerella forsythensis* and *Treponema denticola* was 0, and their proportion to the total bacteria level was 0%.

According to the same criteria as Subject A, Subject D was classified as "mild".

For Subject E, the total bacterial count of the three kinds of bacteria, namely, *Porphyromonas gingivalis, Tannerella forsythensis* and *Treponema denticola* was 339241, and their proportion to the total bacteria level was 0.4%.

According to the same criteria as Subject A, Subject E was assessed with "moderate" periodontal disease.

### (2) Assessment of status of dental caries

For Subject A, the bacterial count of *Streptococcus mutans* was 0, and its proportion to the total bacteria level was 0%.

According to the criteria where dental caries is assessed to be "mild" if the bacterial count of *Streptococcus mutans* among the bacteria contained in the intraoral sample is less than 0.05% to the total bacterial count, "moderate" if it is 0.05% or more but less than 2.5% to the total bacterial count, and "severe" if it is 2.5% or more to the total bacterial count (assessment criteria 5 described herein), Subject A was assessed with "mild" dental caries.

For Subject B, the bacterial count of *Streptococcus mutans* was 18,862, and its proportion to the total bacteria level was 0.01%. According to the same criteria as Subject A, Subject B was assessed with "mild" dental caries.

For Subject C, the bacterial count of *Streptococcus mutans* was 0, and its proportion to the total bacteria level was 0%. According to the same criteria as Subject A, Subject C was assessed with "mild" dental caries.

For Subject D, the bacterial count of *Streptococcus mutans* was 17,736, and its proportion to the total bacteria level was 0.05%. According to the same criteria as Subject A, Subject D was assessed with "moderate" dental caries.

For Subject E, the bacterial count of *Streptococcus mutans* was 18,808, and its proportion to the total bacteria level was 0.02%. According to the same criteria as Subject A, Subject E was assessed with "mild" dental caries.

### With a single sampling, information of the bacteria related to periodontal disease and dental caries was comprehensively acquired, and the statuses of periodontal disease and dental caries were simultaneously assessed from the bacterial counts (Table 1-7).

For Subjects A and B, both of the status of periodontal disease and the status of dental caries were found to be mild. For Subject C, the status of periodontal disease was found to be severe while the status of dental caries was mild. For Subject D, the status of periodontal disease was found to be mild while the status of dental caries was moderate. For Subject E, the status of periodontal disease was found to be moderate while the status of dental caries was mild.

**[Table 1-7]**

| Subject A | | | |
|---|---|---|---|
| | Mild | Moderate | Severe |
| Periodontal bacteria | * | | |
| Cariogenic bacterium | * | | |
| | | | |

| Subject B | | | |
|---|---|---|---|
| | Mild | Moderate | Severe |
| Periodontal bacteria | * | | |
| Cariogenic bacterium | * | | |
| | | | |

| Subject C | | | |
|---|---|---|---|
| | Mild | Moderate | Severe |
| Periodontal bacteria | | | * |
| Cariogenic bacterium | * | | |
| | | | |

| Subject D | | | |
|---|---|---|---|
| | Mild | Moderate | Severe |
| Periodontal bacteria | * | | |
| Cariogenic bacterium | * | | |
| | | | |

| Subject E | | | |
|---|---|---|---|
| | Mild | Moderate | Severe |
| Periodontal bacteria | | | * |
| Cariogenic bacterium | * | | |

### [Example 1-2]

Assessment of statuses of periodontal disease/dental caries using plaque specimen

### <Preparation of subgingival plaque specimen>

With the cooperation of five subjects at the Osaka University Dental Hospital, a subgingival plaque assessment test was conducted. The subgingival plaques from these five subjects, i.e., Subjects F, G, H, I and J, were assessed.

The subgingival plaque was collected by inserting two Absorbent paper points (ISO Color-Coded) #40 (from DENTSPLY MAILLEFER) into periodontal pocket for 30 seconds. Thereafter, the paper points were placed in a microtube containing 0.15 mL of sterilized distilled water and vortexed for 20 seconds. The paper points were removed with sterilized tweezers and the resultant was frozen and stored at -20°C until detection.

The stored frozen specimen was melted to be used as a PCR template. PCR was conducted using GeneAmp 9700 (Applied Biosystems) for amplification and labeling. As primers, SEQ ID NOS:1, 2, 91 and 92 were used. Composition of the PCR liquid (20 µL system) was as shown in Table 1-8.

**[Table 1-8]**

| | Composition |
|---|---|
| Water | - |
| Control DNA | 0.5 pg |
| Primer for control DNA (F) | 0.25 µM |
| Primer for control DNA (R) | 0.25 µM |
| Primer for bacterium (F) | 0.25 µM |
| Primer for bacterium (R) | 0.25 µM |
| Plaque lysate | 1 µL |
| Ex taq | - |

PCR conditions were as shown in Table 1-9.

**[Table 1-9]**

| Ramp max, liquid amount 20 ul | | | | | |
|---|---|---|---|---|---|
| 95°C | 95°C | 56°C | 72°C | 40°C | 4°C |
| 1 min | 10 sec | 30 sec | 20 sec | 8 min | Unlimited |
| | 40 Cycles | | | | |

At the end of PCR, 180 µL of a hybridization reaction solution (48 µL of 1M Tris-HCl, 48 µL of 1M NaCl, 20 µL of 0.5% Tween 20, 65 µL of water) was added respectively. As a hybridization pretreatment, the resultant was allowed to react in GeneAmp 9700 at 94°C for a minute and left at 4°C.

In an automatic hybridization/washing machine (Mitsubishi Rayon), hybridization took place at 50°C for 2 hours followed by washing.

Probes mounted on the DNA chip used for measurement were as indicated in Table 1-10.

**[Table 1-10]**

| SEQ ID NO | Role | Other bacterium targeted for detection | Sequence (5' to 3') |
|---|---|---|---|
| 3 | *Porphyromonas gingivalis* (P.g.) probe | | TTCAATGCAATACTCGTATC |
| 5 | *Tannerella forsythia* (T.f.) probe | | CACGTATCTCATTTTATTCC |
| 8 | *Treponema denticola* (T.d.) probe | | CCTCTTCTTCTTATTCTTCAT |
| 12 | *Campylobacter rectus* (C.r.) probe | C.s. | GTCATAATTCTTTCCCAAGA |
| 20 | *Fusobacterium nucleatum* subsp. *nucleatum* (F.n.) probe | | TACATTCCGAAAAACGTCAT |
| 26 | *Prevotella intermedia* (P.i.) probe | | GGGTAAATGCAAAAAGGCA |
| 28 | *Prevotella nigrescens* (P.n.) probe | | CTTTATTCCCACATAAAAGC |
| 32 | *Aggregatibacter actinomycetemcomitans* (A.a.) probe | | GTCAATTTGGCATGCTATTA |
| 34 | *Campylobacter concisus* (C.c.) probe | | CCCAAGCAGTTCTATGGT |
| 44 | *Streptococcus gordonii* (S.g.) probe | S.s. | CACCCGTTCTTCTCTTACA |
| 47 | *Streptococcus intermedius* (S.i.) probe | | ACAGTATGAACTTTCCATTCT |
| 59 | *Streptococcus mutans* (S.mu.) probe | | CACACGTTCTTGACTTAC |
| 60 | Total bacterial level probe (16S rRNA consensus seq.) | | CGTATTACCGCGGCTGCTGGCAC |
| 75 | Control DNA 15 probe | | CTATTCGACCAGCGATATCACTAC GTAGGC |

For detection, Genopal Reader (Mitsubishi Rayon) was used with exposure times of 0.1, 1, 4 and 40 seconds.

### <Results>

The results of the bacterial counts per paper point are shown in Table 1-11.

**[Table 1-11]**

| Bacterial count per paper point | | | | | | |
|---|---|---|---|---|---|---|
| | | Subject F | Subject G | Subject H | Subject I | Subject J |
| Probe name | *Porphyromonas gingivalis* | 72 | 0 | 0 | 0 | 0 |
| | *Tannerella forsythens is* | 2371 | 0 | 0 | 84 | 0 |
| | *Treponema denticola* | 15 | 0 | 0 | 0 | 0 |
| | *Campylobacter rectus* | 24 | 2 | 3 | 206 | 130 |
| | Genus *Fusobacterium* | 2045 | 26 | 520 | 1020 | 1964 |
| | *Prevotella intermedia* | 0 | 0 | 0 | 0 | 0 |
| | *Prevotella nigrescens* | 21 | 0 | 171 | 35 | 854 |
| | *Aggregatibacter actinomycetemcomitans* | 0 | 0 | 0 | 0 | 0 |
| | *Campylobacter concisus* | 0 | 0 | 0 | 9 | 0 |
| | *Streptococcus gordonii* | 17 | 1553 | 291 | 198 | 32 |
| | *Streptococcus intermedius* | 222 | 0 | 1169 | 220 | 438 |
| | *Streptococcus mutans* | 0 | 3 | 6 | 0 | 0 |
| | Total bacterial level | 37594 | 5872 | 38000 | 37090 | 37878 |

### <Assessments>

### (1) Assessment of status of periodontal disease

For Subject F, the total bacterial count of the three kinds of bacteria, namely, *Porphyromonas gingivalis, Tannerella forsythensis* and *Treponema denticola* was 2,458, and their proportion to the total bacteria level was 6.5%.

According to the criteria where periodontal disease is assessed to be "mild" if the total bacterial count of at least one of *Porphyromonas gingivalis, Tannerella forsythensis* and *Treponema denticola* among the bacteria contained in the intraoral sample is less than 0.05% to the total bacterial count, "moderate" if it is 0.05% or more but less than 0.5% to the total bacterial count, and "severe" if it is 0.5% or more to the total bacterial count (assessment criteria 1 described herein), Subject F was assessed with "severe" periodontal disease.

For Subject G, the total bacterial count of the three kinds of bacteria, namely, *Porphyromonas gingivalis, Tannerella forsythensis* and *Treponema denticola* was 0, and their proportion to the total bacteria level was 0%.

According to the same criteria as Subject F, Subject G was assessed with "mild" periodontal disease.

For Subject H, the total bacterial count of the three kinds of bacteria, namely, *Porphyromonas gingivalis, Tannerella forsythensis* and *Treponema denticola* was 0, and their proportion to the total bacteria level was 0%.

According to the same criteria as Subject F, Subject H was assessed with "mild" periodontal disease.

For Subject I, the total bacterial count of the three kinds of bacteria, namely, *Porphyromonas gingivalis, Tannerella forsythensis* and *Treponema denticola* was 84, and their proportion to the total bacteria level was 0.23%.

According to the same criteria as Subject F, Subject I was classified as "moderate".

For Subject J, the total bacterial count of the three kinds of bacteria, namely, *Porphyromonas gingivalis, Tannerella forsythensis* and *Treponema denticola* was 0, and their proportion to the total bacteria level was 0%.

According to the same criteria as Subject F, Subject J was assessed with "mild" periodontal disease.

### (2) Assessment of status of dental caries

For Subject F, the bacterial count of *Streptococcus mutans* was 0, and its proportion to the total bacteria level was 0%.

According to the criteria where dental caries is assessed to be "mild" if the bacterial count of *Streptococcus mutans* among the bacteria contained in the intraoral sample is less than 0.05% to the total bacterial count, "moderate" if it is 0.05% or more but less than 2.5% to the total bacterial count, and "severe" if it is 2.5% or more to the total bacterial count (assessment criteria 2 described herein), Subject F was assessed with "mild" dental caries.

For Subject G, the bacterial count of *Streptococcus mutans* was 3, and its proportion to the total bacteria level was 0.06%. According to the same criteria as Subject F, Subject G was assessed with "moderate" dental caries.

For Subject H, the bacterial count of *Streptococcus mutans* was 6, and its proportion to the total bacteria level was 0%. According to the same criteria as Subject F, Subject H was assessed with "mild" dental caries.

For Subject I, the bacterial count of *Streptococcus mutans* was 0, and its proportion to the total bacteria level was 0%. According to the same criteria as Subject F, Subject I was assessed with "mild" dental caries.

For Subject J, the bacterial count of *Streptococcus mutans* was 0, and its proportion to the total bacteria level was 0%. According to the same criteria as Subject F, Subject J was assessed with "mild" dental caries.

With a single sampling, information of the bacteria related to periodontal disease and dental caries was comprehensively acquired, and the statuses of periodontal disease and dental caries were simultaneously assessed from the bacterial counts (Table 1-12). For Subjects H and J, both of the status of periodontal disease and the status of dental caries were found to be mild. For Subject F, the status of periodontal disease was found to be severe while the status of dental caries was mild. For Subject G, the status of periodontal disease was found to be mild while the status of dental caries was moderate. For Subject I, the status of periodontal disease was found to be moderate while the status of dental caries was mild.

**[Table 1-12]**

| Subject F | | | |
|---|---|---|---|
| | Mild | Moderate | Severe |
| Periodontal bacteria | | | * |
| Cariogenic bacterium | * | | |
| | | | |

| Subject G | | | |
|---|---|---|---|
| | Mild | Moderate | Severe |
| Periodontal bacteria | * | | |
| Cariogenic bacterium | | * | |
| | | | |

| Subject H | | | |
|---|---|---|---|
| | Mild | Moderate | Severe |
| Periodontal bacteria | * | | |
| Cariogenic bacterium | * | | |
| | | | |

| Subject I | | | |
|---|---|---|---|
| | Mild | Moderate | Severe |
| Periodontal bacteria | | * | |
| Cariogenic bacterium | * | | |
| | | | |

| Subject J | | | |
|---|---|---|---|
| | Mild | Moderate | Severe |
| Periodontal bacteria | * | | |
| Cariogenic bacterium | * | | |

### [Example 2-1]

### Assessments of severity and risk of exacerbation of periodontal disease

### Detection of intraoral bacteria in subgingival plaque specimen

### <Preparation of subgingival plaque specimen>

Subgingival plaques were collected from 220 male and female subjects in their 20s to 70s at the Osaka University Dental Hospital before receiving a periodontal disease treatment. The subgingival plaque was collected by inserting two Absorbent paper points (ISO Color-Coded) #40 (from DENTSPLY MAILLEFER) into periodontal pocket for 30 seconds. Thereafter, the paper points were placed in a microtube containing 0.15 mL of sterilized distilled water and vortexed for 20 seconds. The paper points were removed with sterilized tweezers and the resultant was frozen and stored at -20°C until detection.

### <Acquirement of clinical information>

For all specimens, clinical information was scored according to the following criteria. The following four indices are widely utilized in the field of dentistry.
(i) Periodontal pocket depth (Pd): indicates the distance from the gingival margin to the tip of the probe upon inserting a periodontal probe into the pocket. The index was scored in a millimeter unit.
(ii) Bleeding on probing (BOP): indicates the presence or the absence of bleeding upon inserting a periodontal probe into the pocket. Absence of bleeding was scored 0 while presence of bleeding was scored 1.
(iii) Gingival Index (GI): indicates the degree of inflammation of the gum. No inflammation was scored 0, mild inflammation was scored 1, moderate inflammation was scored 2 and severe inflammation was scored 3.
(iv) Plaque Index (PlI): indicates the amount of plaque deposit on the tooth surface adjacent to the gum. 0 when no plaque was observed, 1 when plaque cannot be seen with the naked eye but observed when scratching with a probe, 2 when plaque can be seen with the naked eye, and 3 when abundance of plaque can be seen.

### <PCR>

All of the above-described stored frozen specimens were melted to be used as PCR templates. PCR was conducted with the following reaction liquid composition and reaction conditions to amplify the sequences of the regions of 16S rRNA targeted for detection of the intraoral bacteria in the specimen. PCR was conducted with GeneAmp 9700 (Applied Biosystems) using Premix Ex Taq™ Hot Start Version (from Takara) as a PCR kit. Primers having the following sequences were used. The 5'-terminal of the forward primer was labeled with Cy5.

Forward primer (for bacterial amplification):
5'-Cy5-TCCTACGGGAGGCAGCAGT-3' (SEQ ID NO:192)

Reverse primer (for bacterial amplification):
5'-CAGGGTATCTAATCCTGTTTGCTACC-3' (SEQ ID NO:200)

Forward primer (for amplification of absolute level index):
5'-Cy5-GAGAAGCCTACACAAACGTAACGTC-3' (SEQ ID NO:190)

Reverse primer (for amplification of absolute level index):
5'-CTCTAAAGACCGCTCTATCTCGG-3' (SEQ ID NO:191)

### <Reaction liquid composition>

2 x Premix Ex Taq (registered trademark)

| | |
|---|---|
| Hot Start Version | 10 µL |
| 4 µM forward primer (for bacterial amplification) | 1 µL |
| 4 µM reverse primer (for bacterial amplification) | 1 µL |
| 4 µM forward primer (for amplification of absolute level index) | 1 µL |
| 4 µM reverse primer (for amplification of absolute level index) | 1 µL |
| Template DNA | 5 µL |
| Absolute level index | 1 µL |
| Total | 20 µL |

### <Reaction conditions>

Following heating at 95°C for a minute, a total of 40 cycles of "dissociation at 98°C (10 sec), annealing at 60°C (30 sec) and synthesis at 72°C (20 sec)", and cooling at 4°C were conducted to obtain an amplified product.

### <DNA chip: Production of DNA chip for detecting intraoral bacteria>

A through-hole-type DNA chip was produced in the same manner as the method described in Example 2-1 of Japanese Unexamined Patent Application Publication No. 2007-74950 (method for detecting methylated DNA and/or unmethylated DNA).

Here, the mounted oligonucleotide probes were probes having the sequence information shown in Table 2-4.

**[Table 2-4]**

| SEQ ID NO | Name of probe | Sequence (5' to 3') |
|---|---|---|
| 93 | *Porphyromonas gingivalis* probe 1 | TTCAATGCAATACTCGTATC |
| 95 | *Tannerella forsythia* probe 1 | CACGTATCTCATTTTATTCC |
| 99 | *Treponema denticola* probe 2 | CCTCTTCTTCTTATTCTTCAT |
| 101 | *Campylobacter gracilis* probe 1 | GCCTTCGCAATAGGTATT |
| 104 | *Campylobacter* spp. probe 2 | GTCATAATTCTTTCCCAAGA |
| 105 | *Campylobacter* spp. probe 3 | CAATGGGTATTCTTCTTGAT |
| 106 | *Fusobacterium nucleatum* probe 1 | TAGTTATACAGTTTCCAACG |
| 109 | *Fusobacterium nucleatum* probe 4 | CCAGTACTCTAGTTACACA |
| 110 | *Fusobacterium nucleatum* probe 5 | TTTCTTTCTTCCCAACTGAA |
| 112 | *Fusobacterium nucleatum* probe 7 | TACATTCCGAAAAACGTCAT |
| 114 | *Fusobacterium nucleatum* probe 9 | TATGCAGTTTCCAACGCAA |
| 116 | *Parvimonas micra* probe 1 | AAGTGCTTAATGAGGTTAAG |
| 118 | *Prevotella intermedia* probe 1 | GGGTAAATGCAAAAAGGCA |
| 123 | *Prevotella nigrescens* probe 1 | CTTTATTCCCACATAAAAGC |
| 125 | *Streptococcus* spp. probe 1 | AAGTACCGTCACTGTGTG |
| 127 | *Aggregatibacter actinomycetemcomitans* probe 1 | GTCAATTTGGCATGCTATTA |
| 131 | *Campylobacter concisus* probe 1 | CCCAAGCAGTTCTATGGT |
| 133 | *Capnocytophaga* spp. probe 1 | TACACGTACACCTTATTCTT |
| 136 | *Capnocytophaga* spp. probe 4 | CAACCATTCAAGACCAACA |
| 140 | *Eikenella corrodens* probe 2 | CTCTAGCTATCCAGTTCAG |
| 141 | *Streptococcus* spp. probe 3 | CACCCGTTCTTCTCTTACA |
| 144 | *Streptococcus intermedius* probe 2 | ACAGTATGAACTTTCCATTCT |
| 146 | *Streptococcus* spp. probe 6 | TCTCCCCTCTTGCACTCA |
| 147 | *Streptococcus* spp. probe 7 | TCCCCTCTTGCACTCAAGT |
| 148 | *Actinomyces odontolyticus* probe 1 | AAGTCAGCCCGTACCCA |
| 151 | *Veillonella parvula* probe 2 | TATTCGCAAGAAGGCCTT |
| 154 | *Actinomyces naeslundii* II probe 2 | CCACCCACAAGGAGCAG |
| 156 | *Selenomonas noxia* probe 1 | TTCGCATTAGGCACGTTC |

### <Hybridization with DNA chip>

The following solutions were mixed to prepare a hybridization solution.

| | |
|---|---|
| Amplified DNA product obtained by PCR | 20 µL |
| 1M Tris-HCl | 48 µL |
| 1M NaCl | 48 µL |
| 0.5% Tween 20 | 20 µL |
| Water | 64 µL |
| Total | 200 µL |

200 µL of the hybridization solution was allowed to make contact with the above-described DNA chip and allowed to hybridize therewith at 50°C for 2 hours.

Following hybridization, the DNA chip was washed under the following conditions. Washing with 1000 µL of a 0.24M Tris-HCl/0.24M NaCl/0.05% Tween-20 solution for 220 seconds was repeated for 12 times, followed by washing with 1000 µL of 0.24M Tris-HCl/0.24M NaCl for 220 seconds for 4 times.

At the end of washing, each chip was transferred into a 0.24M Tris-HCl/0.24M NaCl mixed solution at room temperature.

### <Detection>

Following washing, the fluorescence intensity of each spot on the DNA chip was measured under the following conditions using Genopal Reader (from Mitsubishi Rayon).

### <Detection conditions>

Center excitation wavelength: 633 nm
Exposure time: 0.1, 1, 4 and 40 seconds

### <Results>

The fluorescence intensity of the spot mounted with a probe for detecting a targeted bacterium was divided by the background value (median of fluorescence intensities of spots having no probe mounted) to calculate a signal-to-noise ratio of the fluorescence intensity resulting from hybridization (hereinafter, referred to as a signal intensity). The signal-to-noise ratio was expressed in a logarithmic function Log2. For all 220 specimens, signal-to-noise ratio data from 0 to a maximum of 10 was acquired for each bacterium targeted for detection. Due to the characteristic of the logarithmic function, detection was judged at 1 or higher while data less than 1 was judged to be below the detection limit.

### <Analysis of correlation between clinical information and bacterial levels: severity of periodontal disease>

First, for each specimen, the clinical information was correlated with the signal-to-noise ratio data indicating the bacterial levels. Thereafter, the clinical information and the bacterial levels of the specimen were subjected to a cluster analysis to classify them by similarity. The 4 parameters of clinical information (Pd, BOP, GI and PlI) and the values of the 28 kinds of bacterial levels acquired from the probes shown in Table 2-4 were subjected to the cluster analysis to generate a heatmap for each cluster. For the cluster analysis, statistic software "R" (R Development Core Team) was used. The analysis conditions were those employed for general statistical analyses in biological researches. Pearson correlation was employed as the distance function and Ward's method was employed as a method for merging the clusters.

### <Results>

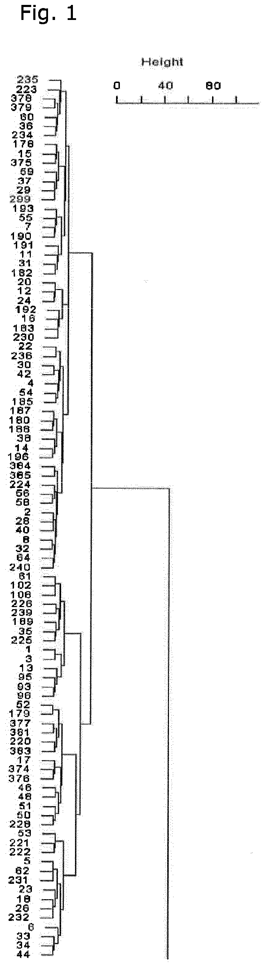

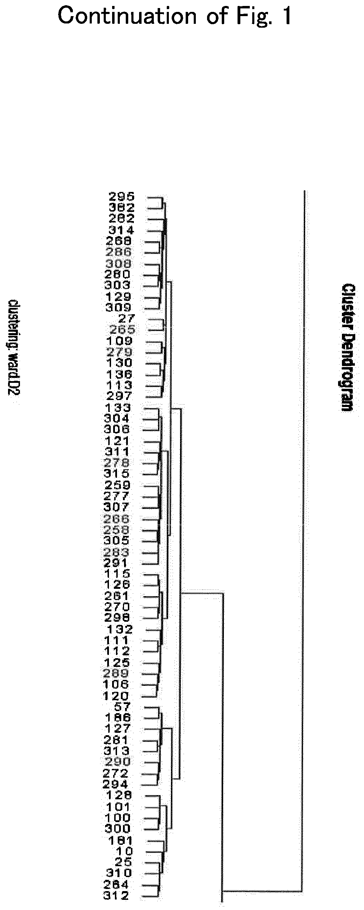

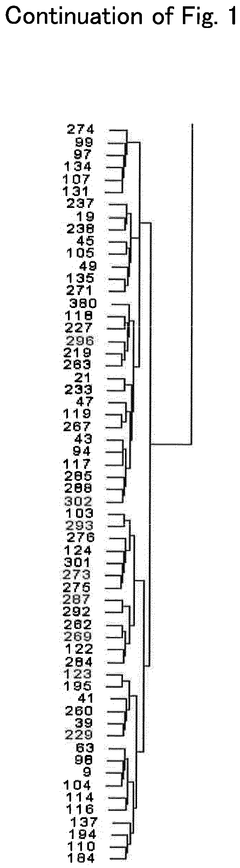

The specimens were classified roughly into three clusters as shown in Figure 1. In order to clarify the characteristics of the clinical information of the three clusters, the clusters were indicated as Groups 1, 2 and 3, and average values and standard deviations of Pd, BOP, GI, PlI and bacterial levels were calculated for each group (Table 2-5).

**[Table 2-5]**

| Parameter | All (n=220) | | Group 1 (n=96) | | Group 2 (n=60) | | Group (n=64) | |
|---|---|---|---|---|---|---|---|---|
| | Average value | Standard deviation | Average value | Standard deviation | Average value | Standard deviation | Average value | Standard deviation |
| Pd | 4.4 | 2.3 | 6.3 | 2.2 | 3.2 | 0.9 | 2.6 | 0.8 |
| BOP | 0.4 | 0.5 | 0.8 | 0.4 | 0.2 | 0.4 | 0.0 | 0.1 |
| GI | 0.8 | 0.8 | 1.4 | 0.6 | 0.4 | 0.6 | 0.1 | 0.3 |
| pII | 0.8 | 0.7 | 1.3 | 0.6 | 0.8 | 0.7 | 0.2 | 0.4 |
| *Porphyromonas gingivalis* probe 1 | 2.1 | 2.9 | 4.6 | 2.8 | 1> | - | 1> | - |
| *Tannerella forsythia* probe 1 | 2.6 | 2.9 | 5.5 | 1.8 | 1> | - | 1> | - |
| *Treponema denticola* probe 2 | 1.7 | 2.2 | 3.7 | 1.9 | 1> | - | 1> | - |
| *Campylobacter gracilis* probe 1 | 1> | - | 1> | - | 1> | - | 1> | - |
| *Campylobacter* spp. probe 2 | 2.3 | 2.2 | 4.1 | 1.8 | 1.5 | 1.3 | 1> | - |
| *Campylobacter* spp. probe 3 | 1> | - | 1> | - | 1> | - | 1> | - |
| *Fusobacterium nucleatum* probe 1 | 1> | - | 1.1 | 0.6 | 1> | - | 1> | - |
| *Fusobacterium nucleatum* probe 4 | 1> | - | 1> | - | 1> | - | 1> | - |
| *Fusobacterium nucleatum* probe 5 | 3.7 | 2.6 | 5.8 | 1.7 | 3.8 | 1.8 | 1> | - |
| *Fusobacterium nucleatum* probe 7 | 4.7 | 2.6 | 6.7 | 1.5 | 4.9 | 1.7 | 1.5 | 1.0 |
| *Fusobacterium nucleatum* probe 9 | 1> | - | 1.0 | 0.7 | 1> | - | 1> | - |
| *Parvimonas micra* probe 1 | 1> | - | 1> | - | 1> | - | 1> | - |
| *Prevotella intermedia* probe 1 | 1> | - | 1> | - | 1.2 | 1.3 | 1> | - |
| *Prevotella nigrescens* probe 1 | 1> | - | 1> | - | 1> | - | 1> | - |
| *Streptococcus* spp. probe 1 | 1> | - | 1> | - | 1> | - | 1> | - |
| *Aggregatibacter actinomycetemcomitans* probe 1 | 1> | - | 1> | - | 1> | - | 1> | - |
| *Campylobacter concisus* probe 1 | 1.5 | 1.6 | 1.4 | 1.5 | 2.8 | 1.7 | 1> | - |
| *Capnocytophaga* spp. probe 1 | 1> | - | 1> | - | 1> | - | 1> | - |
| *Capnocytophaga* spp. probe 4 | 1> | - | 1> | - | 1> | - | 1> | - |
| *Eikenella corrodens* probe 2 | 1> | - | 1> | - | 1> | - | 1> | - |
| *Streptococcus* spp. probe 3 | 2.9 | 1.9 | 1.6 | 1.4 | 4.2 | 1.5 | 3.5 | 1.7 |
| *Streptococcus intermedius* probe 2 | 1.1 | 1.3 | 1> | - | 2.1 | 1.7 | 1> | - |
| *Streptococcus* spp. probe 6 | 1> | 1> | 1> | - | 1> | - | 1> | - |
| *Streptococcus* spp. probe 7 | 1> | - | 1> | - | 1> | - | 1> | - |
| *Actinomyces odontolyticus* probe 1 | 1> | - | 1> | - | 1> | - | 1> | - |
| *Veil*/*onella parvula* probe 2 | 1.7 | 1.7 | 1.1 | 1.4 | 3.4 | 1.6 | 1.2 | 1.3 |
| *Actinomyces naeslundii* II probe 2 | 1.4 | 1.4 | 1> | - | 2.3 | 1.5 | 1.4 | 1.4 |
| *Selenomonas noxia* probe 1 | 1> | 1.4 | 1.1 | 1.6 | 1.4 | 1.4 | 1> | - |

According to "Guideline for examination, diagnosis and treatment plan of periodontal disease, 2008" edited by the Japanese Society of Periodontology, p.16, the treatment plan of the periodontal disease differs depending on the condition of Pd, i.e., whether 6 mm or deeper, 4-5 mm, or less than that. Therefore, Group 1 was considered to correspond to severe periodontal disease specimens, Group 2 was considered to correspond to early periodontal disease specimens, and Group 3 was considered to correspond to normal specimens.

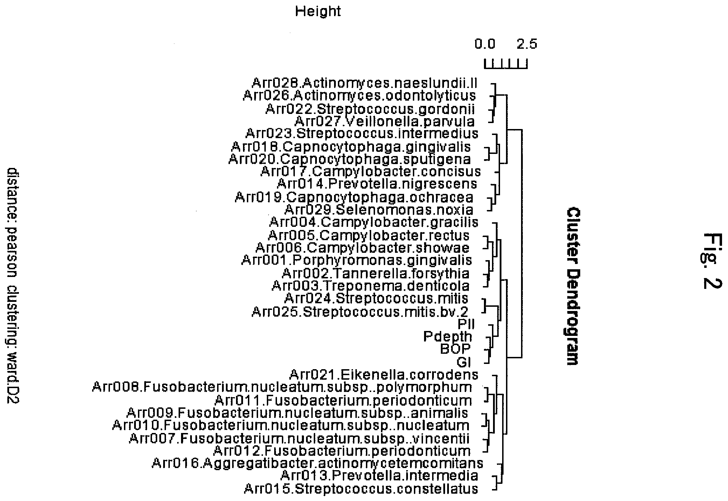

Meanwhile, the clinical information and the detection parameters of the bacteria were classified roughly into three clusters as shown in Figure 2.

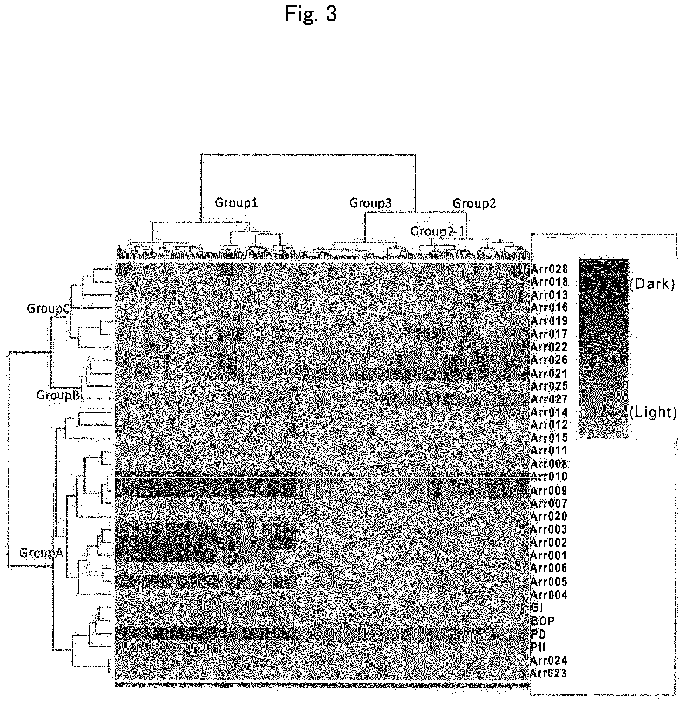

Furthermore, from the results shown in Figure 3, the indices for indicating the severity of periodontal disease were found to represent as follows. In the heatmap shown in Figure 3, the numerical values of each parameter were represented from green to red (light to dark) from lower values.

Group A consisted of clinical information, and "Red Complex", namely, *Porphyromonas gingivalis, Tannerella forsythia* and *Treponema denticola,* as well as *Campylobacter rectus, Campylobacter gracilis, Campylobacter showae, Fusobacterium nucleatum* and the like that belonged to the group with the second highest malignancy to "Red Complex". The parameters of Group A were detected to be the highest for the specimens of Group 1, where the detected level became lower in Group 2 and much lower in Group 3. Since the levels of the above-mentioned seven bacteria were information that had a strong correlation or inverse correlation with the clinical information, they can be indices for indicating severity of periodontal disease.

Subgroup 2-1 was found among the specimens of Group 2 where the detected level of *Fusobacterium nucleatum* was as high as that of Group 1 but its values of clinical information PD and "Red Complex" were low. This subgroup indicates a status where *Fusobacterium nucleatum* is increasing before an increase in the "Red Complex", which is highly likely to lead to exacerbation of periodontal disease in the future as represented by an increase in the "Red Complex", an increase in Pd, and the like. In other words, the bacterial level of *Fusobacterium nucleatum* to the total bacterial level of *Porphyromonas gingivalis, Tannerella forsythia* and *Treponema denticola* could be an index for assessing the risk of exacerbation.

Group B consisted of bacteria with relatively low malignancy. Three bacteria, namely, *Streptococcus* spp.3, *Streptococcus intermedius* and *Veillonella parvula* were detected to be particularly low in the specimens of Group 1 whereas their detected levels were high in Groups 2 and 3. In other words, the bacterial levels of the three bacteria, namely, *Streptococcus* spp., *Streptococcus intermedius* and *Veillonella parvula* increased with the decreases in the values of the clinical information, and thus they could be indices for indicating severity of periodontal disease.

Then, regression analysis was performed using the information of each bacterial level as an independent variable and the severity grouping as objective variables. As a result, the coefficient of determination became closer to 1 with three, six and further with eight bacterial species, as compared to information from a single bacterial species. Similar tendency was seen when regression analysis was performed using information of each bacterial level as an independent variable and the periodontal pocket values as objective variables. Accordingly, increasing the number of measured bacterial species enhanced the prediction accuracy of the clinical information such as the severity group and the periodontal pocket.

**[Table 2-6]**

| Number of bacteria (Targeted bacteria) | Coefficient of determination (Contribution rate) | |
|---|---|---|
| | Prediction of severity groups (normal, early-stage periodontal disease, periodontal disease) | Prediction of periodontal pocket (mm) |
| 1 (Pg) | 0.47 | 0.39 |
| 3 (Pg, Td, Tf) | 0.69 | 0.56 |
| 6 (Pg, Td, Tf, Cr, Fn, Ss) | 0.79 | 0.57 |
| 8 (Pg, Td, Tf, Cr, Fn, Ss) | 0.81 | 0.57 |

### [Example 2-2]

### Assessment of effect of treating periodontal disease

### Bacterial detection in plaque specimens before and after treatment

### <Preparation of plaque specimen>

In order to compare the bacterial levels of plaque specimens before and after a treatment, subgingival plaques were collected from 65 cases of male and female subjects in their 20s to 70s at the Osaka University Dental Hospital before and after the periodontal disease treatment. As the treatment, a basic periodontal therapy, namely, removal of dental calculus (scaling and root planing) was performed.

The subgingival plaque was collected by inserting two Absorbent paper points (ISO Color-Coded) #40 (from DENTSPLY MAILLEFER) into periodontal pocket for 30 seconds. Thereafter, the paper points were placed in a microtube containing 0.15 mL of sterilized distilled water and vortexed for 20 seconds. The paper points were removed with sterilized tweezers and the resultant was frozen and stored at -20°C until detection.

### <DNA chip: Production of DNA chip for detecting intraoral bacteria>

A through-hole-type DNA chip was produced in the same manner as the method described in Example 2-1 of Japanese Unexamined Patent Application Publication No. 2007-74950 (method for detecting methylated DNA and/or unmethylated DNA).

Here, the mounted oligonucleotide probes were probes having the sequence information shown in Table 2-7.

PCR, hybridization with the DNA chip and detection were carried out in the same manner as Example 2-1.

**[Table 2-7]**

| SEQ ID NO | Name of probe | Sequence (5' to 3') |
|---|---|---|
| 93 | *Porphyromonas gingivalis* probe 1 | TTCAATGCAATACTCGTATC |
| 95 | *Tannerella forsythia* probe 1 | CACGTATCTCATTTTATTCC |
| 98 | *Treponema denticola* probe 1 | CTCTTCTTCTTATTCTTCAT |
| 103 | *Campylobacter* spp. probe 1 | ATTCTTTCCCAAGAAAAGGA |
| 112 | *Fusobacterium nucleatum* probe 7 | TACATTCCGAAAAACGTCAT |
| 118 | *Prevotella intermedia* probe 1 | GGGTAAATGCAAAAAGGCA |
| 123 | *Prevotella nigrescens* probe 1 | CTTTATTCCCACATAAAAGC |
| 127 | *Aggregatibacter actinomycetemcomitans* probe 1 | GTCAATTTGGCATGCTATTA |
| 133 | *Capnocytophaga* spp. probe 1. | TACACGTACACCTTATTCTT |
| 142 | *Streptococcus* spp. probe 4 | CACCCGTTCTTCTCTTAC |
| 143 | *Streptococcus intermedius* probe 1 | CAGTATGAACTTTCCATTCT |
| 158 | *Streptococcus mutans* probe | CACACGTTCTTGACTTAC |
| 159 | Total level index probe | CGTATTACCGCGGCTGCTGGCAC |
| 174 | Absolute level index 15 probe | CTATTCGACCAGCGATATCACTACGTAGGC |

### <Results>

The background value (median and three standard deviations of fluorescence intensities of spots having no probe mounted) was subtracted from the fluorescence intensity of the spot mounted with a probe for detecting a targeted bacterium to calculate the signal intensity resulting from hybridization. Then, the signal intensities of the absolute level index probes were compared among multiple DNA chips to derive the correction coefficient for each DNA chip so that the signal intensities of the bacteria targeted for detection can be corrected and compared. The resultant was multiplied by a predetermined bacterial-level-calculating coefficient to calculate the bacterial level of each bacterium targeted for detection in a genome copy number. As each bacterial-level-calculating coefficient, a standard curve was generated by determining the signal intensities upon detecting genomic DNA derived from each bacterium so as to determine the coefficient to calculate each bacterial level backward from the signal intensity of each bacterium. Lastly, the resultant was multiplied by the dilution ratio of the detected specimen used as the PCR template to determine the bacterial count per paper point.

According to the above-described calculation, bacterial count data (from 0 to 10⁷ digits) of each bacterium targeted for detection was acquired for all 130 specimens. Subsequently, average values and standard deviations of each parameter before and after the treatment were calculated (Table 2-8).

**[Table 2-8]**

| | Pre-treatment | | Post-treatment | | Change between pre-treatment and post-treatment | p-Value |
|---|---|---|---|---|---|---|
| | Average value | Standard deviation | Average value | Standard deviation | | |
| Pd | 5.9 | 2.3 | 3.5 | 1.5 | 2.5 | <0.01 |
| BOP | 0.7 | 0.5 | 0.2 | 0.4 | 0.4 | N.A |
| GI | 1.4 | 0.6 | 0.3 | 0.5 | 1.1 | N.A |
| pll | 1.2 | 0.6 | 0.4 | 0.6 | 0.8 | N.A |
| Total microbe | 1.3 x 10⁷ | 5.0 x 10⁷ | 8.3 x 10⁵ | 13 x 10⁵ | 1.3 x 10⁷ | <0.05 |
| *Porphyromonas gingivalis* | 4.1 x 10⁵ | 6.8 x 10⁵ | 1.5 x 10⁵ | 6.0 x 10⁵ | 2.6 x 10⁵ | <0.01 |
| *Tannerella forsythia* | 4.4 x 10⁵ | 11 x 10⁵ | 3.2 x 10⁴ | 10 x 10⁴ | 4.1 x 10⁵ | <0.01 |
| *Treponema denticola* | 8.1 x 10⁵ | 22 x 10⁵ | 1.4 x 10⁴ | 6.4 x 10⁴ | 6.7 x 10⁴ | <0.05 |
| *Campylobacter* spp. | 6.9 x 10⁵ | 7.3 x 10⁵ | 2.3 x 10⁴ | 6.0 x 10⁴ | 4.6 x 10⁴ | <0.01 |
| *Fusobacterium nucleatum* | 5.0 x 10⁵ | 7.7 x 10⁵ | 1.1 x 10⁵ | 3.5 x 10⁵ | 3.9 x 10⁵ | <0.01 |
| *Prevotella intermedia* | 5.8 x 10⁵ | 21 x 10⁵ | 4.4 x 10⁴ | 2.5 x 10⁴ | 1.4 x 10⁴ | |
| *Prevotella nigrescens* | 4.1 x 10⁴ | 6.8 x 10⁴ | 1.1 x 10⁴ | 4.1 x 10⁴ | 5.6 x 10² | |
| *Aggregatibacter actinomycetemcomitans* | 4.3 x 10³ | 20 x 10³ | 1.6 x 10² | 13 x 10² | 4.1 x 10³ | |
| *Capnocytophaga* spp. | 1.1 x 10³ | 8.3 x 10³ | 1.4 x 10² | 8.0 x 10² | 9.1 x 10² | |
| *Streptococcus* spp. | 2.3 x 10⁴ | 3.8 x 10⁴ | 2.5 x 10⁴ | 5.9 x 10⁴ | -1.8 x 10³ | |
| *Streptococcus intermedius* | 1.7 x 10⁴ | 5.0 x 10⁴ | 1.5 x 10⁴ | 4.5 x 10⁴ | 2.4 x 10³ | |
| *Streptococcus mutans* | 1.1 x 10³ | 4.6 x 10³ | 1.5 x 10³ | 5.6 x 10³ | -4.4 x 10² | |

### <Comparison of bacterial data before and after treatment>

When the bacterial levels after the treatment were subtracted from the bacterial levels before the treatment, the bacterial levels of *Porphyromonas gingivalis, Tannerella forsythia, Treponema denticola, Fusobacterium nucleatum, Campylobacter rectus* and the total bacterial count were found to be decreased. All of the increases and decreases were found to be significant according to the significance level of 5% in the Student's t-test. Moreover, when the proportions of each bacterial level to the total bacterial count before and after the treatment were determined and compared, the proportion of *Streptococcus* spp. was found to be significantly increased.

From the above results, it was found that the effect of treating periodontal disease can be assessed by comparing the bacterial levels, particularly the bacterial levels of *Porphyromonas gingivalis, Tannerella forsythia, Treponema denticola, Fusobacterium nucleatum, Campylobacter rectus* and *Streptococcus* spp. before and after the treatment.

Furthermore, among the 65 cases, there were 7 cases where the bacterial levels of "Red Complex" did not decrease after the treatment. All of these teeth were molars, which coincides with the fact that dental calculus on molars are anatomically difficult to be removed. In addition, the 7 cases included the only case where the Pd value increased, which indicates exacerbation in terms of clinical information. This means that a case with no therapeutic effect was observed without being overlooked. Furthermore, for other 6 cases, Pd showed improvement or were nearly the same, while the bacterial levels of "Red Complex" were nearly the same or tended to increase. These results showed that the bacterial levels had a correlation with the observed clinical information, and thus can be suggested as indices for indicating disease activity.

J Health Care Dent. 2003; 5: 42-61 reports that the removal rates of the dental calculus on the molars by specialists were 91% when Pd was 1-3 mm, 61% when Pd was 4-6 mm and 37% when Pd was 7 mm or deeper. In this study, the bacteria levels of the "Red Complex" were found to be decreased in about 90% of the cases, showing that the study was a good examination for being consistent with the reports.

### [Example 2-3]

### Designing universal primers

The universal primers of the present invention include the following sequences.

According to the above-described procedure of Item 1, V3 and V4 were selected as the variable regions of 16S rRNA. Highly conserved universal primer design regions were selected to precede and succeed the V3 and V4 regions, thereby designing the sequences represented by SEQ ID NOS:192-204. The designed universal primer sequences were used while utilizing the function of Ribosomal Database Project (RDP) database ProbeMatch and selecting *Type* for Strain and *Isolates* for Source as the conditions to extract "Hits" for each score. The coverage was assessed by the "Hits" counts to the total count of the score.

With the combinations of Samples Nos. 1-5, homology of the genomic DNA of the periodontal disease bacteria with the primer sequences was 100%, whereas the homology was not 100% with the combinations of Samples Nos. 6-8.

**[Table 2-9]**

| SEQ ID NO | Sequence (5' to 3') | Primer name | Number of hits | Coverage (%) | Number of bases (Bp) | Tm | GC(%) | Secondary structure | Dimer |
|---|---|---|---|---|---|---|---|---|---|
| 192 | TCCTACGGGAGGCAGCAGT | Cy5-Universal16S-FWD | 11677 | 91 | 19 | 67.2 | 46% | Weak | no |
| 193 | TCCTACGGGAGGCAGCAG | Cy5-Universal16S-FWD1 | 11790 | 92 | 18 | 66.6 | 46% | Weak | no |
| 194 | TCCTACGGGAGGCAGCA | Cy5-Universal16S-FWD2 | 11796 | 92 | 17 | 65.9 | 43% | Weak | no |
| 195 | TCCTACGGGAGGCAGC | Cy5-Universal16S-FWD3 | 11797 | 92 | 16 | 62.8 | 42% | Weak | no |
| 196 | CCTACGGGAGGCAGC | Cy5-Universal16S-FWD4 | 11805 | 92 | 17 | 64.8 | 41% | Weak | no |
| 197 | CTACGGGAGGCAGCAG | Cy5-Universal16S-FWD5 | 11885 | 93 | 16 | 60.8 | 42% | none | no |
| 198 | TACGGGAGGCAGCAG | Cy5-Universal16S-FWD6 | 11930 | 93 | 15 | 59.6 | 63% | none | no |
| 199 | GGACTACCAGGGTATCTAATCCTGTT | Universal RVS | 10924 | 95 | 26 | 64.2 | 67% | Strong | no |
| 200 | CAGGGTATCTAATCCTGTTTGCTACC | Universal RVS2 2014 | 764 | 6 | 26 | 65.4 | 65% | Strong | no |
| 201 | CAGGGTATCTAATCCTGTTYG | Universal RVS1 2016 | 11020 | 86 | 21 | 54.7 | 69% | Strong | no |
| 202 | CAGGGTATCTAATCCTGTT | Universal RVS2 2016 | 11054 | 86 | 19 | 52.9 | 71% | Strong | no |
| 203 | GGGTATCTAATCCYGTT | Universal RVS3 2016 | 11475 | 89 | 17 | 50.5 | 70% | none | no |
| 204 | CRGGGTATCTAATCCYGTT | Universal RVS4 2016 | 11461 | 89 | 19 | 58 | 67% | none | no |

### [Example 2-4]

### Assessment of coverage of universal primers

### <Measurement target>

Genomic DNA derived from the respective bacteria purchased from ATCC were targeted for measurement.

### <PCR>

PCR was conducted with the following reaction liquid composition and reaction conditions to amplify the sequences of the regions of 16S rRNA targeted for detection of the bacteria-derived genomic DNA. Amplification reaction was conducted with GeneAmp 9700 (from Applied Biosystems) using Premix Ex Taq™ Hot Start Version (from Takara) as a PCR kit. Primer conditions shown in Table 2-10 were used. The 5'-terminal of the forward primer was labeled with Cy5 to label the terminal of the amplified product.

**[Table 2-10]**

| Sample No. | Genomic DNA | PCR PrimerF | PCR PrimerR |
|---|---|---|---|
| 1 | Porphyromonas gingivalis | Cy5-Universal16S-FWD6 | Universal RVS4 2016 |
| 2 | Prevotella intermedia | Cy5-Universal16S-FWD6 | Universal RVS4 2016 |
| 3 | Prevotella nigrescens | Cy5-Universal16S-FWD6 | Universal RVS4 2016 |
| 4 | Veillonella parvula | Cy5-Universal16S-FWD6 | Universal RVS4 2016 |
| 5 | Porphyromonas gingivalis | Cy5-Universal16S-FWD | Universal RVS2 2014 |
| 6 | Prevotella intermedia | Cy5-Universal16S-FWD | Universal RVS2 2014 |
| 7 | Prevotella nigrescens | Cy5-Universal16S-FWD | Universal RVS2 2014 |
| 8 | Veillonella parvula | Cy5-Universal16S-FWD | Universal RVS2 2014 |

### <Reaction liquid composition>

2 x Premix Ex Taq (registered trademark)

| | |
|---|---|
| Hot Start Version | 10 µL |
| 4 µM Forward primer (for bacterial amplification) | 1 µL |
| 4 µM Reverse primer (for bacterial amplification) | 1 µL |
| Template DNA | 5 µL |
| Absolute level index | 1 µL |
| Water | 2 µL |
| Total | 20 µL |

### <Reaction conditions>

Following heating at 95°C for a minute, a total of 40 cycles of "dissociation at 98°C (10 sec), annealing at 60°C (30 sec) and synthesis at 72°C (20 sec)", and cooling at 4°C were conducted to obtain an amplified product.

### <DNA chip: Production of DNA chip for detecting intraoral bacteria>

A through-hole-type DNA chip was produced in the same manner as the method described in Example 2-1 of Japanese Unexamined Patent Application Publication No. 2007-74950 (method for detecting methylated DNA and/or unmethylated DNA).

Here, the mounted oligonucleotide probes were probes having the sequence information shown in Table 2-11.

**[Table 2-11]**

| SEQ ID NO | Name of probe | Sequence (5' to 3') |
|---|---|---|
| 93 | *Porphyromonas gingivalis* probe 1 | TTCAATGCAATACTCGTATC |
| 95 | *Tannerella forsythia* probe 1 | CACGTATCTCATTTTATTCC |
| 99 | *Treponema denticola* probe 2 | CCTCTTCTTCTTATTCTTCAT |
| 114 | *Fusobacterium nucleatum* probe 9 | TATGCAGTTTCCAACGCAA |
| 116 | *Parvimonas micra* probe 1 | AAGTGCTTAATGAGGTTAAG |
| 118 | *Prevotella intermedia* probe 1 | GGGTAAATGCAAAAAGGCA |
| 123 | *Prevotella nigrescens* probe 1 | CTTTATTCCCACATAAAAGC |
| 127 | *Aggregatibacter actinomycetemcomitans* probe 1 | GTCAATTTGGCATGCTATTA |
| 131 | *Campylobacter concisus* probe 1 | CCCAAGCAGTTCTATGGT |
| 133 | *Capnocytophaga* spp. probe 1 | TACACGTACACCTTATTCTT |
| 141 | *Eikenella corrodens* probe 2 | CTCTAGCTATCCAGTTCAG |
| 142 | *Streptococcus* spp. probe 3 | CACCCGTTCTTCTCTTACA |
| 144 | *Streptococcus intermedius* probe 2 | ACAGTATGAACTTTCCATTCT |
| 148 | *Actinomyces odontolyticus* probe 1 | AAGTCAGCCCGTACCCA |
| 151 | *Veillonella parvula* probe 2 | TATTCGCAAGAAGGCCTT |
| 154 | *Actinomyces naeslundii* II probe 2 | CCACCCACAAGGAGCAG |
| 156 | *Selenomonas noxia* probe 1 | TTCGCATTAGGCACGTTC |
| 159 | Probe for total level index | CGTATTACCGCGGCTGCTGGCAC |

### <Hybridization with DNA chip>

The following solutions were mixed to prepare a hybridization solution.

| | |
|---|---|
| Amplified DNA product obtained by PCR | 20 µL |
| 1M Tris-HCl | 48 µL |
| 1M NaCl | 48 µL |
| 0.5% Tween 20 | 20 µL |
| Water | 64 µL |
| Total | 200 µL |

Using an automatic hybridization/washing machine (Mitsubishi Rayon), 200 µL of the hybridization solution was allowed to make contact with the above-described DNA chip and allowed to hybridize therewith at 50°C for 2 hours.

Following hybridization, the DNA chip was washed under the following conditions. Washing with 1000 µL of a 0.24M Tris-HCl/0.24M NaCl/0.05% Tween-20 solution for 220 seconds was repeated for 12 times, followed by washing with 1000 µL of 0.24M Tris-HCl/0.24M NaCl for 220 seconds for 4 times.

At the end of washing, each chip was transferred into a 0.24M Tris-HCl/0.24M NaCl mixed solution at room temperature.

### <Detection>

Following washing, the fluorescence intensity of each spot on the DNA chip was measured under the following conditions using Genopal Reader (from Mitsubishi Rayon).

### <Detection conditions>

Center excitation wavelength: 633 nm
Exposure time: 0.1, 1, 4 and 40 seconds

### <Results>

The background value (median of fluorescence intensities of spots having no probe mounted) was subtracted from the fluorescence intensity of the spot mounted with a probe for detecting a targeted bacterium to calculate the fluorescence intensity resulting from hybridization (hereinafter, referred to as a signal intensity). As can be appreciated from the results shown in Table 2-12, while the signal intensities of genomic DNA were as low as 3000 or less for Samples Nos. 6, 7 and 8, sufficient signal intensities of 30,000 or more were obtained on the probe spots of interest for Samples Nos. 2, 3 and 4. Moreover, the sequence homology was 100% for all primer pairs, and both Samples Nos. 1 and 5 as positive controls had sufficient signal intensities.

In Example 2-3, "Hits" counts of the scores of Cy5-Universal 16S-FWD6 and Universal RVS4 2016 to the total count were higher than those of Cy5-Universal 16S-FWD and Universal RVS2 2014, which was consistent with the results obtained in this example (Example 2-4) that showed enhancement of the capacity of Cy5-Universal 16S-FWD6 and Universal RVS4 2016 to detect bacteria-derived genomic DNA.

**[Table 2-12]**

| | Sample NO. | | | | | | | |
|---|---|---|---|---|---|---|---|---|
| | 1 | 2 | 3 | 4 | 5 | 6 | 7 | 8 |
| *Porphyromonas gingivalis* | 295291 | 0 | 0 | 0 | 485883 | 0 | 0 | 0 |
| *Tannerela for sythensis* | 0 | 0 | 0 | 0 | 0 | 0 | 0 | 0 |
| *Treponema denticola* | 0 | 0 | 0 | 0 | 0 | 0 | 0 | 0 |
| *Campylobacter rectus* | 0 | 0 | 0 | 0 | 0 | 0 | 0 | 0 |
| *Fusobacterium nucleatum* | 0 | 0 | 0 | 0 | 0 | 0 | 0 | 0 |
| *Parvimonas micra* | 0 | 0 | 0 | 0 | 0 | 0 | 0 | 0 |
| *Prevotella intermedia* | 0 | 46355 | 0 | 0 | 0 | 2059 | 0 | 0 |
| *Prevotella nigrescens* | 0 | 0 | 89338 | 0 | 0 | 0 | 1772 | 0 |
| *Aggregatibacter actinomycetemcomitans* | 0 | 0 | 0 | 0 | 3337 | 0 | 0 | 0 |
| *Campylobacter concisus* | 0 | 0 | 0 | 0 | 0 | 0 | 0 | 0 |
| *Capnocytophaga gingivalis* | 0 | 0 | 0 | 0 | 0 | 0 | 0 | 0 |
| *Streptococcus gordonii* | 0 | 0 | 0 | 0 | 0 | 0 | 0 | 0 |
| *Streptococcus htermedius* | 0 | 0 | 0 | 0 | 0 | 0 | 0 | 0 |
| *Actinomyces odontolyticus* | 0 | 0 | 0 | 0 | 0 | 0 | 0 | 0 |
| *Veillonella parvula* | 0 | 0 | 0 | 33214 | 0 | 0 | 0 | 506 |
| *Actinomyces naeslundii II* | 0 | 0 | 0 | 0 | 0 | 0 | 0 | 0 |
| *Streptococcus mutans* | 0 | 0 | 0 | 0 | 0 | 0 | 0 | 0 |
| Total bacteria | 519778 | 424242 | 230188 | 500044 | 344107 | 7243 | 1375 | 1678 |

### [Example 2-5]

### Designing DNA probes for detecting bacteria

DNA probes for bacteria detection of the present invention target the sequences represented by SEQ ID NOS:97, 100, 120, 121, 122, 129, 130, 152 and 155.

V3 and V4 were selected as the variable regions of 16S rRNA. Among the V3 and V4 regions, sequences specific to the respective bacteria were selected, or if specificity is already sufficient, sequences that give better Tm were selected. In addition, in a case where some bacterial strains have polymorphic sequences, multiple sequences were determined.

### [Comparative example 1]

### Assessment of DNA probe for bacteria detection

### <Measurement target>

Genomic DNA derived from the respective bacteria purchased from ATCC were targeted for measurement.

### <PCR>

PCR was conducted with the following reaction liquid composition and reaction conditions to amplify the sequences of the regions of 16S rRNA targeted for detection of the bacteria-derived genomic DNA. Amplification reaction was conducted with GeneAmp 9700 (from Applied Biosystems) using Premix Ex Taq™ Hot Start Version (from Takara) as a PCR kit. Primer conditions shown below were used. The 5'-terminal of the forward primer was labeled with Cy5 to label the terminal of the amplified product.

### <Primer sequences>

Forward primer:
5'Cy5-TACGGGAGGCAGCAG-3' (SEQ ID NO:205)

Reverse primer:
5'-CRGGGTATCTAATCCYGTT-3' (SEQ ID NO:206; where R represents A or G, and Y represents C or T)

### <Reaction liquid composition>

| | |
|---|---|
| 2 x Premix Ex Taq (registered trademark) | |
| Hot Start Version | 10 µL |
| 4 µM Forward primer (for bacterial amplification) | 1 µL |
| 4 µM Reverse primer (for bacterial amplification) | 1 µL |
| Template DNA | 5 µL |
| Absolute level index | 1 µL |
| Water | 2 µL |
| Total | 20 µL |

### <Reaction conditions>

Following heating at 95°C for a minute, a total of 40 cycles of "dissociation at 98°C (10 sec), annealing at 60°C (30 sec) and synthesis at 72°C (20 sec)", and cooling at 4°C were conducted to obtain an amplified product.

### <DNA chip: Production of DNA chip for detecting intraoral bacteria>

A through-hole-type DNA chip was produced in the same manner as the method described in Example 2-1 of Japanese Unexamined Patent Application Publication No. 2007-74950 (method for detecting methylated DNA and/or unmethylated DNA).

However, a DNA microarray including the sequences represented by SEQ ID NOS:95, 97, 99, 100,120, 122, 123, 124, 127, 129, 130, 151, 152, 154, 155 and 159 as DNA probes for detecting bacteria was prepared.

### <Hybridization with DNA chip>

The following solutions were mixed to prepare a hybridization solution.

| | |
|---|---|
| Amplified DNA product obtained by PCR | 20 µL |
| 1M Tris-HCl | 48 µL |
| 1M NaCl | 48 µL |
| 0.5% Tween 20 | 20 µL |
| Water | 64 µL |
| Total | 200 µL |

Using an automatic hybridization/washing machine (Mitsubishi Rayon), 200 µL of the hybridization solution was allowed to make contact with the above-described DNA chip and allowed to hybridize therewith at 50°C for 16 hours.

Following hybridization, the DNA chip was washed under the following conditions. Washing with 1000 µL of a 0.24M Tris-HCl/0.24M NaCl/0.05% Tween-20 solution for 220 seconds was repeated for 12 times, followed by washing with 1000 µL of 0.24M Tris-HCl/0.24M NaCl for 220 seconds for 4 times.

At the end of washing, each chip was transferred into a 0.24M Tris-HCl/0.24M NaCl mixed solution at room temperature.

### <Detection>

Following washing, the fluorescence intensity of each spot on the DNA chip was measured under the following conditions using Genopal Reader (from Mitsubishi Rayon).

### <Detection conditions>

Center excitation wavelength: 633 nm
Exposure time: 0.1, 1, 4 and 40 seconds

### <Results>

The background value (median of fluorescence intensities of spots having no probe mounted) was subtracted from the fluorescence intensity of the spot mounted with a probe for detecting a targeted bacterium to calculate the fluorescence intensity resulting from hybridization (hereinafter, referred to as a signal intensity). As can be appreciated from the results shown in Table 2-13, the signal intensities of the probes represented by SEQ ID NOS:97, 100, 120, 121, 122, 129, 130, 152 and 155 on the probe spots of interest of the present invention were twice to five times as high as the signal intensities of the existing probes represented by SEQ ID NOS:95, 99, 119, 127, 151 and 154.

From the above results, the probes of the present invention were all proven to be improved probes having high detection sensitivity.

**[Table 2-13]**

| | | | Genomic DNA | | | | | |
|---|---|---|---|---|---|---|---|---|
| | SEQ ID NO | Bacterium targeted by probe | Prevotella intermedia | Veillonella parvula | Actinomyces naeslundii. II | Tannerella forsythia | Treponema denticola | A. actinomyc etemcomit ans |
| Probe | 159 | Total Bacteria | 966419 | 1398030 | 1130725 | 579499 | 620176 | 1889965 |
| | 119 | Prevotella intermedia | 139532 | 0 | 0 | 0 | 0 | 0 |
| | 120 | Prevotella intermedia | 290051 | 0 | 0 | 0 | 0 | 0 |
| | 121 | Prevotella intermedia | 1242919 | 0 | 0 | 0 | 0 | 0 |
| | 122 | Prevotella intermedia | 1030481 | 0 | 0 | 0 | 0 | 0 |
| | 151 | Veillonella parvula | 0 | 139231 | 0 | 0 | 0 | 0 |
| | 152 | Veillonella parvula | 0 | 309013 | 0 | 0 | 0 | 0 |
| | 154 | Actinomyces naeslundii. II | 0 | 0 | 0 | 0 | 0 | 0 |
| | 155 | Actinomyces naeslundii. II | 0 | 0 | 1072153 | 0 | 0 | 0 |
| | 95 | Tannerella forsythia | 0 | 0 | 0 | 948227 | 0 | 0 |
| | 97 | Tannerella forsythia | 0 | 0 | 0 | 1900868 | 0 | 0 |
| | 99 | Treponema denticola | 0 | 0 | 0 | 0 | 1014797 | 0 |
| | 100 | Treponema denticola | 0 | 0 | 0 | 5909 | 2587139 | 0 |
| | 127 | Aggregatibacter actinomycetemcomitans | 0 | 0 | 0 | 0 | 0 | 1980828 |
| | 129 | Aggregatibacter actinomycetemcomitans | 0 | 0 | 0 | 0 | 0 | 3719353 |
| | 130 | Aggregatibacter actinomycetemcomitans | 0 | 0 | 0 | 0 | 0 | 3003901 |

SEQ ID NOS:1-206: Synthetic nucleic acids

SEQUENCE LISTING

<110>  MITSUBISHI RAYON CO.,LTD.

<120>  A method of intraoral inspection

<130>  G1553WO

<150>  JP 2016-136579
<151>  2016-07-11

<160>  206

<170>  PatentIn version 3.5

<210>  1
<211>  19
<212>  DNA
<213>  Artificial

<220>
<223>  Synthetic nucleic acid

<400>  1
tcctacggga ggcagcagt                                                    19


<210>  2
<211>  26
<212>  DNA
<213>  Artificial

<220>
<223>  Synthetic nucleic acid

<400>  2
cagggtatct aatcctgttt gctacc                                            26


<210>  3
<211>  20
<212>  DNA
<213>  Artificial

<220>
<223>  Synthetic nucleic acid

<400>  3
ttcaatgcaa tactcgtatc                                                   20


<210>  4
<211>  20
<212>  DNA
<213>  Artificial

<220>
<223>  Synthetic nucleic acid

<400>  4
gtacattcaa tgcaatactc                                                   20

<210>  5
<211>  20
<212>  DNA
<213>  Artificial

<220>
<223>  Synthetic nucleic acid

<400>  5
cacgtatctc attttattcc                                                    20


<210>  6
<211>  22
<212>  DNA
<213>  Artificial

<220>
<223>  Synthetic nucleic acid

<400>  6
aatacacgta tctcatttta tt                                                 22


<210>  7
<211>  20
<212>  DNA
<213>  Artificial

<220>
<223>  Synthetic nucleic acid

<400>  7
ctcttcttct tattcttcat                                                    20


<210>  8
<211>  21
<212>  DNA
<213>  Artificial

<220>
<223>  Synthetic nucleic acid

<400>  8
cctcttcttc ttattcttca t                                                  21


<210>  9
<211>  18
<212>  DNA
<213>  Artificial

<220>
<223>  Synthetic nucleic acid

<400>  9
gccttcgcaa taggtatt                                                      18


<210>  10
<211>  18
<212>  DNA

<213>  Artificial

<220>
<223>  Synthetic nucleic acid

<400>  10
cgccttcgca ataggtat                                                    18


<210>  11
<211>  20
<212>  DNA
<213>  Artificial

<220>
<223>  Synthetic nucleic acid

<400>  11
attctttccc aagaaaagga                                                  20


<210>  12
<211>  20
<212>  DNA
<213>  Artificial

<220>
<223>  Synthetic nucleic acid

<400>  12
gtcataattc tttcccaaga                                                  20


<210>  13
<211>  20
<212>  DNA
<213>  Artificial

<220>
<223>  Synthetic nucleic acid

<400>  13
caatgggtat tcttcttgat                                                  20


<210>  14
<211>  20
<212>  DNA
<213>  Artificial

<220>
<223>  Synthetic nucleic acid

<400>  14
tagttataca gtttccaacg                                                  20


<210>  15
<211>  20
<212>  DNA
<213>  Artificial

<220>

<223>  Synthetic nucleic acid

<400>  15
ctagttatac agtttccaac                                                    20


<210>  16
<211>  20
<212>  DNA
<213>  Artificial

<220>
<223>  Synthetic nucleic acid

<400>  16
tccagtactc tagttacaca                                                    20


<210>  17
<211>  19
<212>  DNA
<213>  Artificial

<220>
<223>  Synthetic nucleic acid

<400>  17
ccagtactct agttacaca                                                     19


<210>  18
<211>  20
<212>  DNA
<213>  Artificial

<220>
<223>  Synthetic nucleic acid

<400>  18
tttctttctt cccaactgaa                                                    20


<210>  19
<211>  20
<212>  DNA
<213>  Artificial

<220>
<223>  Synthetic nucleic acid

<400>  19
atttctttct tcccaactga                                                    20


<210>  20
<211>  20
<212>  DNA
<213>  Artificial

<220>
<223>  Synthetic nucleic acid

<400>  20

tacattccga aaaacgtcat                                                    20


<210>  21
<211>  20
<212>  DNA
<213>  Artificial

<220>
<223>  Synthetic nucleic acid

<400>  21
ttacattccg aaaaacgtca                                                    20


<210>  22
<211>  19
<212>  DNA
<213>  Artificial

<220>
<223>  Synthetic nucleic acid

<400>  22
tatgcagttt ccaacgcaa                                                     19


<210>  23
<211>  19
<212>  DNA
<213>  Artificial

<220>
<223>  Synthetic nucleic acid

<400>  23
ctctagttat gcagtttcc                                                     19


<210>  24
<211>  20
<212>  DNA
<213>  Artificial

<220>
<223>  Synthetic nucleic acid

<400>  24
aagtgcttaa tgaggttaag                                                    20


<210>  25
<211>  20
<212>  DNA
<213>  Artificial

<220>
<223>  Synthetic nucleic acid

<400>  25
tttcaagtgc ttaatgaggt                                                    20

<210>  26
<211>  19
<212>  DNA
<213>  Artificial

<220>
<223>  Synthetic nucleic acid

<400>  26
gggtaaatgc aaaaaggca                                                    19


<210>  27
<211>  19
<212>  DNA
<213>  Artificial

<220>
<223>  Synthetic nucleic acid

<400>  27
gcaaggtaga tgttgagca                                                    19


<210>  28
<211>  20
<212>  DNA
<213>  Artificial

<220>
<223>  Synthetic nucleic acid

<400>  28
ctttattccc acataaaagc                                                   20


<210>  29
<211>  20
<212>  DNA
<213>  Artificial

<220>
<223>  Synthetic nucleic acid

<400>  29
tccttattca tgaggtacat                                                   20


<210>  30
<211>  18
<212>  DNA
<213>  Artificial

<220>
<223>  Synthetic nucleic acid

<400>  30
aagtaccgtc actgtgtg                                                     18


<210>  31
<211>  19
<212>  DNA

<213>  Artificial

<220>
<223>  Synthetic nucleic acid

<400>  31
ttaagtaccg tcactgtgt                                                    19


<210>  32
<211>  20
<212>  DNA
<213>  Artificial

<220>
<223>  Synthetic nucleic acid

<400>  32
gtcaatttgg catgctatta                                                   20


<210>  33
<211>  20
<212>  DNA
<213>  Artificial

<220>
<223>  Synthetic nucleic acid

<400>  33
tttaacgtca atttggcatg                                                   20


<210>  34
<211>  18
<212>  DNA
<213>  Artificial

<220>
<223>  Synthetic nucleic acid

<400>  34
cccaagcagt tctatggt                                                     18


<210>  35
<211>  18
<212>  DNA
<213>  Artificial

<220>
<223>  Synthetic nucleic acid

<400>  35
tcccaagcag ttctatgg                                                     18


<210>  36
<211>  20
<212>  DNA
<213>  Artificial

<220>

<223>  Synthetic nucleic acid

<400>  36
tacacgtaca ccttattctt                                                    20


<210>  37
<211>  19
<212>  DNA
<213>  Artificial

<220>
<223>  Synthetic nucleic acid

<400>  37
catcaatgta cacgtacac                                                     19


<210>  38
<211>  19
<212>  DNA
<213>  Artificial

<220>
<223>  Synthetic nucleic acid

<400>  38
cattcaagac caacagttt                                                     19


<210>  39
<211>  19
<212>  DNA
<213>  Artificial

<220>
<223>  Synthetic nucleic acid

<400>  39
caaccattca agaccaaca                                                     19


<210>  40
<211>  18
<212>  DNA
<213>  Artificial

<220>
<223>  Synthetic nucleic acid

<400>  40
gcttagttga gctaagcg                                                      18


<210>  41
<211>  19
<212>  DNA
<213>  Artificial

<220>
<223>  Synthetic nucleic acid

<400>  41

tcaaaggcag ttgcttagt                                                    19

<210>  42
<211>  19
<212>  DNA
<213>  Artificial

<220>
<223>  Synthetic nucleic acid

<400>  42
ctagctatcc agttcagaa                                                    19

<210>  43
<211>  19
<212>  DNA
<213>  Artificial

<220>
<223>  Synthetic nucleic acid

<400>  43
ctctagctat ccagttcag                                                    19

<210>  44
<211>  19
<212>  DNA
<213>  Artificial

<220>
<223>  Synthetic nucleic acid

<400>  44
cacccgttct tctcttaca                                                    19

<210>  45
<211>  18
<212>  DNA
<213>  Artificial

<220>
<223>  Synthetic nucleic acid

<400>  45
cacccgttct tctcttac                                                     18

<210>  46
<211>  20
<212>  DNA
<213>  Artificial

<220>
<223>  Synthetic nucleic acid

<400>  46
cagtatgaac tttccattct                                                   20

<210>  47
<211>  21
<212>  DNA
<213>  Artificial

<220>
<223>  Synthetic nucleic acid

<400>  47
acagtatgaa ctttccattc t                                                21


<210>  48
<211>  18
<212>  DNA
<213>  Artificial

<220>
<223>  Synthetic nucleic acid

<400>  48
cccctcttgc actcaagt                                                    18


<210>  49
<211>  18
<212>  DNA
<213>  Artificial

<220>
<223>  Synthetic nucleic acid

<400>  49
tctcccctct tgcactca                                                    18


<210>  50
<211>  19
<212>  DNA
<213>  Artificial

<220>
<223>  Synthetic nucleic acid

<400>  50
tcccctcttg cactcaagt                                                   19


<210>  51
<211>  17
<212>  DNA
<213>  Artificial

<220>
<223>  Synthetic nucleic acid

<400>  51
aagtcagccc gtaccca                                                     17


<210>  52
<211>  17
<212>  DNA

<213>  Artificial

<220>
<223>  Synthetic nucleic acid

<400>  52
cgcactcaag tcagccc                                                    17


<210>  53
<211>  19
<212>  DNA
<213>  Artificial

<220>
<223>  Synthetic nucleic acid

<400>  53
ctattcgcaa gaaggcctt                                                  19


<210>  54
<211>  18
<212>  DNA
<213>  Artificial

<220>
<223>  Synthetic nucleic acid

<400>  54
tattcgcaag aaggcctt                                                   18


<210>  55
<211>  17
<212>  DNA
<213>  Artificial

<220>
<223>  Synthetic nucleic acid

<400>  55
cacaaggagc aggcctg                                                    17


<210>  56
<211>  17
<212>  DNA
<213>  Artificial

<220>
<223>  Synthetic nucleic acid

<400>  56
ccacccacaa ggagcag                                                    17


<210>  57
<211>  18
<212>  DNA
<213>  Artificial

<220>

<223>  Synthetic nucleic acid

<400>  57
ttcgcattag gcacgttc                                                    18


<210>  58
<211>  19
<212>  DNA
<213>  Artificial

<220>
<223>  Synthetic nucleic acid

<400>  58
ctattcgcat taggcacgt                                                   19


<210>  59
<211>  18
<212>  DNA
<213>  Artificial

<220>
<223>  Synthetic nucleic acid

<400>  59
cacacgttct tgacttac                                                    18


<210>  60
<211>  23
<212>  DNA
<213>  Artificial

<220>
<223>  Synthetic nucleic acid

<400>  60
cgtattaccg cggctgctgg cac                                              23


<210>  61
<211>  30
<212>  DNA
<213>  Artificial

<220>
<223>  Synthetic nucleic acid

<400>  61
cgtgcattgt cgtgtaggtt cgaccctaat                                       30


<210>  62
<211>  30
<212>  DNA
<213>  Artificial

<220>
<223>  Synthetic nucleic acid

<400>  62

gcagctacgt tcatacctac gcaaggcatt                                    30

<210>  63
<211>  30
<212>  DNA
<213>  Artificial

<220>
<223>  Synthetic nucleic acid

<400>  63
gaggagatac cgaatcggtc gacgacattt                                    30

<210>  64
<211>  30
<212>  DNA
<213>  Artificial

<220>
<223>  Synthetic nucleic acid

<400>  64
tgttgcgtga aggtcgtgaa cgattggcaa                                    30

<210>  65
<211>  30
<212>  DNA
<213>  Artificial

<220>
<223>  Synthetic nucleic acid

<400>  65
cccctactga gcaaacgttg cactaatgga                                    30

<210>  66
<211>  30
<212>  DNA
<213>  Artificial

<220>
<223>  Synthetic nucleic acid

<400>  66
aacaacgacc gagtgcatag tcacgtacga                                    30

<210>  67
<211>  30
<212>  DNA
<213>  Artificial

<220>
<223>  Synthetic nucleic acid

<400>  67
aggagcccta aggtattggc gagaaaagtc                                    30

<210>  68
<211>  30
<212>  DNA
<213>  Artificial

<220>
<223>  Synthetic nucleic acid

<400>  68
ctgagtatcc gcatatcttc cgaggttgca                                        30


<210>  69
<211>  30
<212>  DNA
<213>  Artificial

<220>
<223>  Synthetic nucleic acid

<400>  69
acttagctga ccgaaggacc ataacgctgt                                        30


<210>  70
<211>  30
<212>  DNA
<213>  Artificial

<220>
<223>  Synthetic nucleic acid

<400>  70
tggaagggat ccgtagtcaa ccgttgactt                                        30


<210>  71
<211>  30
<212>  DNA
<213>  Artificial

<220>
<223>  Synthetic nucleic acid

<400>  71
cggatcgaca tacgacgcct acagaatgtt                                        30


<210>  72
<211>  30
<212>  DNA
<213>  Artificial

<220>
<223>  Synthetic nucleic acid

<400>  72
taaacgtcta ggcgagacta tgagtgctcc                                        30


<210>  73
<211>  30
<212>  DNA

<213>  Artificial

<220>
<223>  Synthetic nucleic acid

<400>  73
cgtatggatc gatccgacgt accacattag                                        30


<210>  74
<211>  30
<212>  DNA
<213>  Artificial

<220>
<223>  Synthetic nucleic acid

<400>  74
actgcgtatg atcgacacgg ctaatcgtag                                        30


<210>  75
<211>  30
<212>  DNA
<213>  Artificial

<220>
<223>  Synthetic nucleic acid

<400>  75
ctattcgacc agcgatatca ctacgtaggc                                        30


<210>  76
<211>  459
<212>  DNA
<213>  Artificial

<220>
<223>  Synthetic nucleic acid

<400>  76
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgaatggta agggtcgtat tagggtcgaa cctacacgac     240

aatgcacgtc gaagcggttg ctaatgcaca ttggctaagg cccacggaac acgaatcacg     300

tgagatcact tactattcga cggaactact atacgcaccg ggacatgcaa gtagcgtccc     360

acaagcataa ggaactctat actcgccatc tacgcagcta caggggatac acgtatgagc     420

ggttacgaag taaagccgag atagagcggt ctttagaga                            459


<210>  77
<211>  459
<212>  DNA
<213>  Artificial

<220>
<223>  Synthetic nucleic acid

<400>  77
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtggtagccg attgaactaa tgccttgcgt aggtatgaac     240

gtagctgcta gtcgaggcct tgtatgcaca ttggctaagg cccacggaac acgaatcacg     300

tgagatcact tactattcga cggaactact atacgcaccg ggacatgcaa gtagcgtccc     360

acaagcataa ggaactctat actcgccatc tacgcagcta caggggatac acgtatgagc     420

ggttacgaag taaagccgag atagagcggt ctttagaga                            459


<210>  78
<211>  459
<212>  DNA
<213>  Artificial

<220>
<223>  Synthetic nucleic acid

<400>  78
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgtcctcga tacatacgaa atgtcgtcga ccgattcggt     240

atctcctctg gcaccgaaga caaatgcaca ttggctaagg cccacggaac acgaatcacg     300

tgagatcact tactattcga cggaactact atacgcaccg ggacatgcaa gtagcgtccc     360

acaagcataa ggaactctat actcgccatc tacgcagcta caggggatac acgtatgagc     420

ggttacgaag taaagccgag atagagcggt ctttagaga                            459


<210>  79
<211>  459
<212>  DNA
<213>  Artificial

<220>
<223>  Synthetic nucleic acid

<400>  79
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgtgctacg ctttacgctt gccaatcgtt cacgaccttc 240

acgcaacact tgatcgaacc gaaatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 80
<211> 459
<212> DNA
<213> Artificial

<220>
<223> Synthetic nucleic acid

<400> 80
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtgacaacag acggtacgtc cattagtgca acgtttgctc 240

agtagggggt ctaagcgtca cttatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 81
<211> 459
<212> DNA
<213> Artificial

<220>
<223> Synthetic nucleic acid

<400> 81
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtgggagcag ttttcttctc gtacgtgact atgcactcgg 240

tcgttgttgg aataccggtc gtaatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210>  82
<211>  459
<212>  DNA
<213>  Artificial

<220>
<223>  Synthetic nucleic acid

<400>  82
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgaacgcaa tacggtggga cttttctcgc caatacctta     240

gggctcctgt gtacctaagc gaaatgcaca ttggctaagg cccacggaac acgaatcacg     300

tgagatcact tactattcga cggaactact atacgcaccg ggacatgcaa gtagcgtccc     360

acaagcataa ggaactctat actcgccatc tacgcagcta caggggatac acgtatgagc     420

ggttacgaag taaagccgag atagagcggt ctttagaga                            459


<210>  83
<211>  459
<212>  DNA
<213>  Artificial

<220>
<223>  Synthetic nucleic acid

<400>  83
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgggatgac ggataagttg caacctcgga agatatgcgg     240

atactcagac gtgatatgcg cagatgcaca ttggctaagg cccacggaac acgaatcacg     300

tgagatcact tactattcga cggaactact atacgcaccg ggacatgcaa gtagcgtccc     360

acaagcataa ggaactctat actcgccatc tacgcagcta caggggatac acgtatgagc     420

ggttacgaag taaagccgag atagagcggt ctttagaga                            459


<210>  84
<211>  459
<212>  DNA
<213>  Artificial

<220>
<223>  Synthetic nucleic acid

<400>  84
gtgagaagcc tacacaaacg taacgtcagg gctaagacaa acgctaacgg tacaccctag      60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtgggtgtac atcggatgac agcgttatgg tccttcggtc 240

agctaagtaa gtccgttttc cacatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 85
<211> 459
<212> DNA
<213> Artificial

<220>
<223> Synthetic nucleic acid

<400> 85
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtgttcagtc aaccggagaa gtcaacggtt gactacggat 240

cccttccatg tagagctctc acaatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 86
<211> 459
<212> DNA
<213> Artificial

<220>
<223> Synthetic nucleic acid

<400> 86
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtggcgattg tcgcgttaaa cattctgtag gcgtcgtatg 240

tcgatccggg acttcgcttc ataatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 87
<211> 459
<212> DNA
<213> Artificial

<220>
<223> Synthetic nucleic acid

<400> 87
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtgtcgtgtg tcgtgagagg agcactcata gtctcgccta 240

gacgtttatg acgagatatc acgatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 88
<211> 459
<212> DNA
<213> Artificial

<220>
<223> Synthetic nucleic acid

<400> 88
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtggtagcgt gtaacgcact aatgtggtac gtcggatcga 240

tccatacgca actttgtacc gagatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 89
<211> 459
<212> DNA
<213> Artificial

<220>
<223> Synthetic nucleic acid

<400>  89
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgagcaacc atcttgagct acgattagcc gtgtcgatca     240

tacgcagtcc gaggcattac tgaatgcaca ttggctaagg cccacggaac acgaatcacg     300

tgagatcact tactattcga cggaactact atacgcaccg ggacatgcaa gtagcgtccc     360

acaagcataa ggaactctat actcgccatc tacgcagcta caggggatac acgtatgagc     420

ggttacgaag taaagccgag atagagcggt ctttagaga                            459


<210>  90
<211>  459
<212>  DNA
<213>  Artificial

<220>
<223>  Synthetic nucleic acid

<400>  90
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgggccttc tccgaatagc ctacgtagtg atatcgctgg     240

tcgaataggc ggattgctca taaatgcaca ttggctaagg cccacggaac acgaatcacg     300

tgagatcact tactattcga cggaactact atacgcaccg ggacatgcaa gtagcgtccc     360

acaagcataa ggaactctat actcgccatc tacgcagcta caggggatac acgtatgagc     420

ggttacgaag taaagccgag atagagcggt ctttagaga                            459


<210>  91
<211>  25
<212>  DNA
<213>  Artificial

<220>
<223>  Synthetic nucleic acid

<400>  91
gagaagccta cacaaacgta acgtc                                            25


<210>  92
<211>  23
<212>  DNA
<213>  Artificial

<220>

<223>  Synthetic nucleic acid

<400>  92
ctctaaagac cgctctatct cgg                                              23


<210>  93
<211>  20
<212>  DNA
<213>  Artificial

<220>
<223>  Synthetic nucleic acid

<400>  93
ttcaatgcaa tactcgtatc                                                  20


<210>  94
<211>  20
<212>  DNA
<213>  Artificial

<220>
<223>  Synthetic nucleic acid

<400>  94
gtacattcaa tgcaatactc                                                  20


<210>  95
<211>  20
<212>  DNA
<213>  Artificial

<220>
<223>  Synthetic nucleic acid

<400>  95
cacgtatctc attttattcc                                                  20


<210>  96
<211>  22
<212>  DNA
<213>  Artificial

<220>
<223>  Synthetic nucleic acid

<400>  96
aatacacgta tctcatttta tt                                               22


<210>  97
<211>  25
<212>  DNA
<213>  Artificial

<220>
<223>  Synthetic nucleic acid

<400>  97

cacgtatctc attttattcc cctgt                                                25

<210>  98
<211>  20
<212>  DNA
<213>  Artificial

<220>
<223>  Synthetic nucleic acid

<400>  98
ctcttcttct tattcttcat                                                      20

<210>  99
<211>  21
<212>  DNA
<213>  Artificial

<220>
<223>  Synthetic nucleic acid

<400>  99
cctcttcttc ttattcttca t                                                    21

<210>  100
<211>  25
<212>  DNA
<213>  Artificial

<220>
<223>  Synthetic nucleic acid

<400>  100
cctcttcttc ttattcttca tctgc                                                25

<210>  101
<211>  18
<212>  DNA
<213>  Artificial

<220>
<223>  Synthetic nucleic acid

<400>  101
gccttcgcaa taggtatt                                                        18

<210>  102
<211>  18
<212>  DNA
<213>  Artificial

<220>
<223>  Synthetic nucleic acid

<400>  102
cgccttcgca ataggtat                                                        18

<210>  103
<211>  20
<212>  DNA
<213>  Artificial

<220>
<223>  Synthetic nucleic acid

<400>  103
attctttccc aagaaaagga                                                    20


<210>  104
<211>  20
<212>  DNA
<213>  Artificial

<220>
<223>  Synthetic nucleic acid

<400>  104
gtcataattc tttcccaaga                                                    20


<210>  105
<211>  20
<212>  DNA
<213>  Artificial

<220>
<223>  Synthetic nucleic acid

<400>  105
caatgggtat tcttcttgat                                                    20


<210>  106
<211>  20
<212>  DNA
<213>  Artificial

<220>
<223>  Synthetic nucleic acid

<400>  106
tagttataca gtttccaacg                                                    20


<210>  107
<211>  20
<212>  DNA
<213>  Artificial

<220>
<223>  Synthetic nucleic acid

<400>  107
ctagttatac agtttccaac                                                    20


<210>  108
<211>  20
<212>  DNA

<213>  Artificial

<220>
<223>  Synthetic nucleic acid

<400>  108
tccagtactc tagttacaca                                                    20


<210>  109
<211>  19
<212>  DNA
<213>  Artificial

<220>
<223>  Synthetic nucleic acid

<400>  109
ccagtactct agttacaca                                                     19


<210>  110
<211>  20
<212>  DNA
<213>  Artificial

<220>
<223>  Synthetic nucleic acid

<400>  110
tttctttctt cccaactgaa                                                    20


<210>  111
<211>  20
<212>  DNA
<213>  Artificial

<220>
<223>  Synthetic nucleic acid

<400>  111
atttctttct tcccaactga                                                    20


<210>  112
<211>  20
<212>  DNA
<213>  Artificial

<220>
<223>  Synthetic nucleic acid

<400>  112
tacattccga aaaacgtcat                                                    20


<210>  113
<211>  20
<212>  DNA
<213>  Artificial

<220>

<223>  Synthetic nucleic acid

<400>  113
ttacattccg aaaaacgtca                                                    20


<210>  114
<211>  19
<212>  DNA
<213>  Artificial

<220>
<223>  Synthetic nucleic acid

<400>  114
tatgcagttt ccaacgcaa                                                     19


<210>  115
<211>  19
<212>  DNA
<213>  Artificial

<220>
<223>  Synthetic nucleic acid

<400>  115
ctctagttat gcagtttcc                                                     19


<210>  116
<211>  20
<212>  DNA
<213>  Artificial

<220>
<223>  Synthetic nucleic acid

<400>  116
aagtgcttaa tgaggttaag                                                    20


<210>  117
<211>  20
<212>  DNA
<213>  Artificial

<220>
<223>  Synthetic nucleic acid

<400>  117
tttcaagtgc ttaatgaggt                                                    20


<210>  118
<211>  19
<212>  DNA
<213>  Artificial

<220>
<223>  Synthetic nucleic acid

<400>  118

gggtaaatgc aaaaaggca                                                    19

<210>  119
<211>  19
<212>  DNA
<213>  Artificial

<220>
<223>  Synthetic nucleic acid

<400>  119
gcaaggtaga tgttgagca                                                    19

<210>  120
<211>  17
<212>  DNA
<213>  Artificial

<220>
<223>  Synthetic nucleic acid

<400>  120
cgaagggtaa atgcaaa                                                      17

<210>  121
<211>  22
<212>  DNA
<213>  Artificial

<220>
<223>  Synthetic nucleic acid

<400>  121
cgaagggtaa atgcaaaggg gc                                                22

<210>  122
<211>  22
<212>  DNA
<213>  Artificial

<220>
<223>  Synthetic nucleic acid

<400>  122
cgaagggtaa atgcaaaaag gc                                                22

<210>  123
<211>  20
<212>  DNA
<213>  Artificial

<220>
<223>  Synthetic nucleic acid

<400>  123
ctttattccc acataaaagc                                                   20

<210>  124
<211>  20
<212>  DNA
<213>  Artificial

<220>
<223>  Synthetic nucleic acid

<400>  124
tccttattca tgaggtacat                                                    20


<210>  125
<211>  18
<212>  DNA
<213>  Artificial

<220>
<223>  Synthetic nucleic acid

<400>  125
aagtaccgtc actgtgtg                                                      18


<210>  126
<211>  19
<212>  DNA
<213>  Artificial

<220>
<223>  Synthetic nucleic acid

<400>  126
ttaagtaccg tcactgtgt                                                     19


<210>  127
<211>  20
<212>  DNA
<213>  Artificial

<220>
<223>  Synthetic nucleic acid

<400>  127
gtcaatttgg catgctatta                                                    20


<210>  128
<211>  20
<212>  DNA
<213>  Artificial

<220>
<223>  Synthetic nucleic acid

<400>  128
tttaacgtca atttggcatg                                                    20


<210>  129
<211>  27
<212>  DNA

<213>  Artificial

<220>
<223>  Synthetic nucleic acid

<400>  129
gtcaatttgg catgctatta acacacc                                          27


<210>  130
<211>  27
<212>  DNA
<213>  Artificial

<220>
<223>  Synthetic nucleic acid

<400>  130
gtcaagttgg catgctatta acacacc                                          27


<210>  131
<211>  18
<212>  DNA
<213>  Artificial

<220>
<223>  Synthetic nucleic acid

<400>  131
cccaagcagt tctatggt                                                    18


<210>  132
<211>  18
<212>  DNA
<213>  Artificial

<220>
<223>  Synthetic nucleic acid

<400>  132
tcccaagcag ttctatgg                                                    18


<210>  133
<211>  20
<212>  DNA
<213>  Artificial

<220>
<223>  Synthetic nucleic acid

<400>  133
tacacgtaca ccttattctt                                                  20


<210>  134
<211>  19
<212>  DNA
<213>  Artificial

<220>

<223>  Synthetic nucleic acid

<400>  134
catcaatgta cacgtacac                                                    19


<210>  135
<211>  19
<212>  DNA
<213>  Artificial

<220>
<223>  Synthetic nucleic acid

<400>  135
cattcaagac caacagttt                                                    19


<210>  136
<211>  19
<212>  DNA
<213>  Artificial

<220>
<223>  Synthetic nucleic acid

<400>  136
caaccattca agaccaaca                                                    19


<210>  137
<211>  18
<212>  DNA
<213>  Artificial

<220>
<223>  Synthetic nucleic acid

<400>  137
gcttagttga gctaagcg                                                     18


<210>  138
<211>  19
<212>  DNA
<213>  Artificial

<220>
<223>  Synthetic nucleic acid

<400>  138
tcaaaggcag ttgcttagt                                                    19


<210>  139
<211>  19
<212>  DNA
<213>  Artificial

<220>
<223>  Synthetic nucleic acid

<400>  139

ctagctatcc agttcagaa                                                    19

<210>  140
<211>  19
<212>  DNA
<213>  Artificial

<220>
<223>  Synthetic nucleic acid

<400>  140
ctctagctat ccagttcag                                                    19

<210>  141
<211>  19
<212>  DNA
<213>  Artificial

<220>
<223>  Synthetic nucleic acid

<400>  141
cacccgttct tctcttaca                                                    19

<210>  142
<211>  18
<212>  DNA
<213>  Artificial

<220>
<223>  Synthetic nucleic acid

<400>  142
cacccgttct tctcttac                                                     18

<210>  143
<211>  20
<212>  DNA
<213>  Artificial

<220>
<223>  Synthetic nucleic acid

<400>  143
cagtatgaac tttccattct                                                   20

<210>  144
<211>  21
<212>  DNA
<213>  Artificial

<220>
<223>  Synthetic nucleic acid

<400>  144
acagtatgaa ctttccattc t                                                 21

<210>  145
<211>  18
<212>  DNA
<213>  Artificial

<220>
<223>  Synthetic nucleic acid

<400>  145
cccctcttgc actcaagt                                                    18


<210>  146
<211>  18
<212>  DNA
<213>  Artificial

<220>
<223>  Synthetic nucleic acid

<400>  146
tctcccctct tgcactca                                                    18


<210>  147
<211>  19
<212>  DNA
<213>  Artificial

<220>
<223>  Synthetic nucleic acid

<400>  147
tcccctcttg cactcaagt                                                   19


<210>  148
<211>  17
<212>  DNA
<213>  Artificial

<220>
<223>  Synthetic nucleic acid

<400>  148
aagtcagccc gtaccca                                                     17


<210>  149
<211>  17
<212>  DNA
<213>  Artificial

<220>
<223>  Synthetic nucleic acid

<400>  149
cgcactcaag tcagccc                                                     17


<210>  150
<211>  19
<212>  DNA

<213>  Artificial

<220>
<223>  Synthetic nucleic acid

<400>  150
ctattcgcaa gaaggcctt                                                    19


<210>  151
<211>  18
<212>  DNA
<213>  Artificial

<220>
<223>  Synthetic nucleic acid

<400>  151
tattcgcaag aaggcctt                                                     18


<210>  152
<211>  17
<212>  DNA
<213>  Artificial

<220>
<223>  Synthetic nucleic acid

<400>  152
tccttctaac tgttcgc                                                      17


<210>  153
<211>  17
<212>  DNA
<213>  Artificial

<220>
<223>  Synthetic nucleic acid

<400>  153
cacaaggagc aggcctg                                                      17


<210>  154
<211>  17
<212>  DNA
<213>  Artificial

<220>
<223>  Synthetic nucleic acid

<400>  154
ccacccacaa ggagcag                                                      17


<210>  155
<211>  17
<212>  DNA
<213>  Artificial

<220>

<223>  Synthetic nucleic acid

<400>  155
aacccaccca caaacga                                                    17


<210>  156
<211>  18
<212>  DNA
<213>  Artificial

<220>
<223>  Synthetic nucleic acid

<400>  156
ttcgcattag gcacgttc                                                   18


<210>  157
<211>  19
<212>  DNA
<213>  Artificial

<220>
<223>  Synthetic nucleic acid

<400>  157
ctattcgcat taggcacgt                                                  19


<210>  158
<211>  18
<212>  DNA
<213>  Artificial

<220>
<223>  Synthetic nucleic acid

<400>  158
cacacgttct tgacttac                                                   18


<210>  159
<211>  23
<212>  DNA
<213>  Artificial

<220>
<223>  Synthetic nucleic acid

<400>  159
cgtattaccg cggctgctgg cac                                             23


<210>  160
<211>  30
<212>  DNA
<213>  Artificial

<220>
<223>  Synthetic nucleic acid

<400>  160

cgtgcattgt cgtgtaggtt cgaccctaat                                        30

<210>  161
<211>  30
<212>  DNA
<213>  Artificial

<220>
<223>  Synthetic nucleic acid

<400>  161
gcagctacgt tcatacctac gcaaggcatt                                        30

<210>  162
<211>  30
<212>  DNA
<213>  Artificial

<220>
<223>  Synthetic nucleic acid

<400>  162
gaggagatac cgaatcggtc gacgacattt                                        30

<210>  163
<211>  30
<212>  DNA
<213>  Artificial

<220>
<223>  Synthetic nucleic acid

<400>  163
tgttgcgtga aggtcgtgaa cgattggcaa                                        30

<210>  164
<211>  30
<212>  DNA
<213>  Artificial

<220>
<223>  Synthetic nucleic acid

<400>  164
cccctactga gcaaacgttg cactaatgga                                        30

<210>  165
<211>  30
<212>  DNA
<213>  Artificial

<220>
<223>  Synthetic nucleic acid

<400>  165
aacaacgacc gagtgcatag tcacgtacga                                        30

<210>  166
<211>  30
<212>  DNA
<213>  Artificial

<220>
<223>  Synthetic nucleic acid

<400>  166
aggagcccta aggtattggc gagaaaagtc                                        30


<210>  167
<211>  30
<212>  DNA
<213>  Artificial

<220>
<223>  Synthetic nucleic acid

<400>  167
ctgagtatcc gcatatcttc cgaggttgca                                        30


<210>  168
<211>  30
<212>  DNA
<213>  Artificial

<220>
<223>  Synthetic nucleic acid

<400>  168
acttagctga ccgaaggacc ataacgctgt                                        30


<210>  169
<211>  30
<212>  DNA
<213>  Artificial

<220>
<223>  Synthetic nucleic acid

<400>  169
tggaagggat ccgtagtcaa ccgttgactt                                        30


<210>  170
<211>  30
<212>  DNA
<213>  Artificial

<220>
<223>  Synthetic nucleic acid

<400>  170
cggatcgaca tacgacgcct acagaatgtt                                        30


<210>  171
<211>  30
<212>  DNA

<213>  Artificial

<220>
<223>  Synthetic nucleic acid

<400>  171
taaacgtcta ggcgagacta tgagtgctcc                                        30


<210>  172
<211>  30
<212>  DNA
<213>  Artificial

<220>
<223>  Synthetic nucleic acid

<400>  172
cgtatggatc gatccgacgt accacattag                                        30


<210>  173
<211>  30
<212>  DNA
<213>  Artificial

<220>
<223>  Synthetic nucleic acid

<400>  173
actgcgtatg atcgacacgg ctaatcgtag                                        30


<210>  174
<211>  30
<212>  DNA
<213>  Artificial

<220>
<223>  Synthetic nucleic acid

<400>  174
ctattcgacc agcgatatca ctacgtaggc                                        30


<210>  175
<211>  459
<212>  DNA
<213>  Artificial

<220>
<223>  Synthetic nucleic acid

<400>  175
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgaatggta agggtcgtat tagggtcgaa cctacacgac     240

aatgcacgtc gaagcggttg ctaatgcaca ttggctaagg cccacggaac acgaatcacg     300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 176
<211> 459
<212> DNA
<213> Artificial

<220>
<223> Synthetic nucleic acid

<400> 176
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtggtagccg attgaactaa tgccttgcgt aggtatgaac 240

gtagctgcta gtcgaggcct tgtatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 177
<211> 459
<212> DNA
<213> Artificial

<220>
<223> Synthetic nucleic acid

<400> 177
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtgtcctcga tacatacgaa atgtcgtcga ccgattcggt 240

atctcctctg gcaccgaaga caaatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 178
<211> 459
<212> DNA

<213>  Artificial

<220>
<223>  Synthetic nucleic acid

<400>  178
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgtgctacg ctttacgctt gccaatcgtt cacgaccttc     240

acgcaacact tgatcgaacc gaaatgcaca ttggctaagg cccacggaac acgaatcacg     300

tgagatcact tactattcga cggaactact atacgcaccg ggacatgcaa gtagcgtccc     360

acaagcataa ggaactctat actcgccatc tacgcagcta caggggatac acgtatgagc     420

ggttacgaag taaagccgag atagagcggt ctttagaga                            459


<210>  179
<211>  459
<212>  DNA
<213>  Artificial

<220>
<223>  Synthetic nucleic acid

<400>  179
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgacaacag acggtacgtc cattagtgca acgtttgctc     240

agtagggggt ctaagcgtca cttatgcaca ttggctaagg cccacggaac acgaatcacg     300

tgagatcact tactattcga cggaactact atacgcaccg ggacatgcaa gtagcgtccc     360

acaagcataa ggaactctat actcgccatc tacgcagcta caggggatac acgtatgagc     420

ggttacgaag taaagccgag atagagcggt ctttagaga                            459


<210>  180
<211>  459
<212>  DNA
<213>  Artificial

<220>
<223>  Synthetic nucleic acid

<400>  180
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgggagcag ttttcttctc gtacgtgact atgcactcgg 240

tcgttgttgg aataccggtc gtaatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 181
<211> 459
<212> DNA
<213> Artificial

<220>
<223> Synthetic nucleic acid

<400> 181
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtgaacgcaa tacggtggga cttttctcgc caatacctta 240

gggctcctgt gtacctaagc gaaatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 182
<211> 459
<212> DNA
<213> Artificial

<220>
<223> Synthetic nucleic acid

<400> 182
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtgggatgac ggataagttg caacctcgga agatatgcgg 240

atactcagac gtgatatgcg cagatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210>  183
<211>  459
<212>  DNA
<213>  Artificial

<220>
<223>  Synthetic nucleic acid

<400>  183
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgggtgtac atcggatgac agcgttatgg tccttcggtc     240

agctaagtaa gtccgttttc cacatgcaca ttggctaagg cccacggaac acgaatcacg     300

tgagatcact tactattcga cggaactact atacgcaccg ggacatgcaa gtagcgtccc     360

acaagcataa ggaactctat actcgccatc tacgcagcta caggggatac acgtatgagc     420

ggttacgaag taaagccgag atagagcggt ctttagaga                            459

<210>  184
<211>  459
<212>  DNA
<213>  Artificial

<220>
<223>  Synthetic nucleic acid

<400>  184
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgttcagtc aaccggagaa gtcaacggtt gactacggat     240

cccttccatg tagagctctc acaatgcaca ttggctaagg cccacggaac acgaatcacg     300

tgagatcact tactattcga cggaactact atacgcaccg ggacatgcaa gtagcgtccc     360

acaagcataa ggaactctat actcgccatc tacgcagcta caggggatac acgtatgagc     420

ggttacgaag taaagccgag atagagcggt ctttagaga                            459

<210>  185
<211>  459
<212>  DNA
<213>  Artificial

<220>
<223>  Synthetic nucleic acid

<400>  185
gtgagaagcc tacacaaacg taacgtcagg gctaagacaa acgctaacgg tacaccctag      60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtggcgattg tcgcgttaaa cattctgtag gcgtcgtatg 240

tcgatccggg acttcgcttc ataatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 186
<211> 459
<212> DNA
<213> Artificial

<220>
<223> Synthetic nucleic acid

<400> 186
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtgtcgtgtg tcgtgagagg agcactcata gtctcgccta 240

gacgtttatg acgagatatc acgatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 187
<211> 459
<212> DNA
<213> Artificial

<220>
<223> Synthetic nucleic acid

<400> 187
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtggtagcgt gtaacgcact aatgtggtac gtcggatcga 240

tccatacgca actttgtacc gagatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 188
<211> 459
<212> DNA
<213> Artificial

<220>
<223> Synthetic nucleic acid

<400> 188
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtgagcaacc atcttgagct acgattagcc gtgtcgatca 240

tacgcagtcc gaggcattac tgaatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 189
<211> 459
<212> DNA
<213> Artificial

<220>
<223> Synthetic nucleic acid

<400> 189
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtgggccttc tccgaatagc ctacgtagtg atatcgctgg 240

tcgaataggc ggattgctca taaatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagaga 459

<210> 190
<211> 25
<212> DNA
<213> Artificial

<220>

<223>  Synthetic nucleic acid

<400>  190
gagaagccta cacaaacgta acgtc                                          25


<210>  191
<211>  23
<212>  DNA
<213>  Artificial

<220>
<223>  Synthetic nucleic acid

<400>  191
ctctaaagac cgctctatct cgg                                            23


<210>  192
<211>  19
<212>  DNA
<213>  Artificial

<220>
<223>  Synthetic nucleic acid

<400>  192
tcctacggga ggcagcagt                                                 19


<210>  193
<211>  18
<212>  DNA
<213>  Artificial

<220>
<223>  Synthetic nucleic acid

<400>  193
tcctacggga ggcagcag                                                  18


<210>  194
<211>  17
<212>  DNA
<213>  Artificial

<220>
<223>  Synthetic nucleic acid

<400>  194
tcctacggga ggcagca                                                   17


<210>  195
<211>  16
<212>  DNA
<213>  Artificial

<220>
<223>  Synthetic nucleic acid

<400>  195

tcctacggga ggcagc                                                        16


<210>  196
<211>  15
<212>  DNA
<213>  Artificial

<220>
<223>  Synthetic nucleic acid

<400>  196
cctacgggag gcagc                                                         15


<210>  197
<211>  16
<212>  DNA
<213>  Artificial

<220>
<223>  Synthetic nucleic acid

<400>  197
ctacgggagg cagcag                                                        16


<210>  198
<211>  15
<212>  DNA
<213>  Artificial

<220>
<223>  Synthetic nucleic acid

<400>  198
tacgggaggc agcag                                                         15


<210>  199
<211>  26
<212>  DNA
<213>  Artificial

<220>
<223>  Synthetic nucleic acid

<400>  199
ggactaccag ggtatctaat cctgtt                                             26


<210>  200
<211>  26
<212>  DNA
<213>  Artificial

<220>
<223>  Synthetic nucleic acid

<400>  200
cagggtatct aatcctgttt gctacc                                             26

<210>  201
<211>  21
<212>  DNA
<213>  Artificial

<220>
<223>  Synthetic nucleic acid

<400>  201
cagggtatct aatcctgtty g                                                21


<210>  202
<211>  19
<212>  DNA
<213>  Artificial

<220>
<223>  Synthetic nucleic acid

<400>  202
cagggtatct aatcctgtt                                                   19


<210>  203
<211>  17
<212>  DNA
<213>  Artificial

<220>
<223>  Synthetic nucleic acid

<400>  203
gggtatctaa tccygtt                                                     17


<210>  204
<211>  19
<212>  DNA
<213>  Artificial

<220>
<223>  Synthetic nucleic acid

<400>  204
crgggtatct aatccygtt                                                   19


<210>  205
<211>  15
<212>  DNA
<213>  Artificial

<220>
<223>  Synthetic nucleic acid

<400>  205
tacgggaggc agcag                                                       15


<210>  206
<211>  19
<212>  DNA

<213>  Artificial

<220>
<223>  Synthetic nucleic acid

<400>  206
crgggtatct aatccygtt                                                    19

## Claims

1. An intraoral examination method, comprising measuring levels of bacteria present in an intraoral sample collected from a subject to assess the status of periodontal disease and/or dental caries.

2. The method according to Claim 1, comprising determining a proportion of a bacterial count of a specific bacterium with respect to the total count of the bacteria present in the intraoral sample to assess the status of periodontal disease and/or dental caries.

3. The method according to Claim 2, which uses saliva as the intraoral sample, wherein the sum of bacterial counts of at least one bacterium selected from the group consisting of *Porphyromonas gingivalis, Tannerella forsythensis* and *Treponema denticola* among the bacteria present in the saliva is:
less than 0.01% to the total bacterial count, then the symptom of periodontal disease is assessed to be "mild";
0.01% or more but less than 0.5% to the total bacterial count, then the symptom of periodontal disease is assessed to be "moderate"; and
0.5% or more to the total bacterial count, then the symptom of periodontal disease is assessed to be "severe".

4. The method according to Claim 2, which uses saliva as the intraoral sample, wherein if the bacterial count of *Streptococcus mutans* among the bacteria present in the saliva is:
less than 0.05% to the total bacterial count, then the symptom of dental caries is assessed to be "mild";
0.05% or more but less than 2.5% to the total bacterial count, then the symptom of dental caries is assessed to be "moderate"; and
2.5% or more to the total bacterial count, then the symptom of dental caries is assessed to be "severe".

5. The method according to Claim 2, which uses plaque as the intraoral sample, wherein if the sum of bacterial counts of at least one bacterium selected from the group consisting of *Porphyromonas gingivalis, Tannerella forsythensis* and *Treponema denticola* among the bacteria present in the plaque is:
less than 0.1% to the total bacterial count, then the symptom of periodontal disease is assessed to be "mild";
0.1% or more but less than 5% to the total bacterial count, then the symptom of periodontal disease is assessed to be "moderate"; and
5% or more to the total bacterial count, then the symptom of periodontal disease is assessed to be "severe".

6. The method according to Claim 1, comprising the steps of:
measuring a bacterial level of a specific bacterium in the intraoral sample collected from the subject, a total level of the bacteria present in the intraoral sample, or both of a bacterial level of a specific bacterium and a total level of the bacteria present in the intraoral sample; and
statistically analyzing the acquired measurement results to assess the severity of periodontal disease.

7. The method according to Claim 1, comprising the steps of:
measuring a bacterial level of a specific bacterium in the intraoral sample collected from the subject, a total level of the bacteria present in the intraoral sample, or both of a bacterial level of a specific bacterium and a total level of the bacteria present in the intraoral sample, before and after a treatment of periodontal disease; and
assessing the effect of treating periodontal disease based on the results from comparison between the bacterial level before the treatment and the bacterial level after the treatment.

8. The method according to Claim 1, comprising the steps of:
measuring a bacterial level of a specific bacterium in the intraoral sample collected from the subject, a total level of the bacteria present in the intraoral sample, or both of a bacterial level of a specific bacterium and a total level of the bacteria present in the intraoral sample; and
assessing the risk of exacerbation of periodontal disease based on the ratio of the acquired bacterial levels.

9. The method according to either one of Claims 6 and 7, wherein the specific bacterium in the intraoral sample is at least one selected from the group consisting of bacteria belonging to genus *Porphyromonas,* genus *Tannerella,* genus *Treponema,* genus *Prevotella,* genus *Campylobacter,* genus *Fusobacterium,* genus *Parvimonas,* genus *Streptococcus,* genus *Aggregatibacter,* genus *Capnocytophaga,* genus *Eikenella,* genus *Actinomyces,* genus *Veillonella,* genus *Selenomonas,* genus *Lactobacillus,* genus *Pseudomonas,* genus *Haemophilus,* genus *Klebsiella,* genus *Serratia,* genus *Moraxella* and genus *Candida.*

10. The method according to either one of Claims 6 and 7, wherein the specific bacterium in the intraoral sample is at least one selected from the group consisting of *Porphyromonas gingivalis, Tannerella forsythia, Treponema denticola, Prevotella intermedia, Aggregatibacter actinomycetemcomitans, Fusobacterium nucleatum* subsp. *vincentii, Fusobacterium nucleatum* subsp. *polymorphum, Fusobacterium nucleatum* subsp. *animalis, Fusobacterium nucleatum* subsp. *nucleatum, Streptococcus sanguis, Streptococcus mitis, Actinomyces viscosus, Streptococcus aureus, Pseudomonas aeruginosa, Streptococcus pyogenes, Streptococcus pneumoniae, Campylobacter gracilis, Campylobacter rectus, Campylobacter showae, Fusobacterium periodonticum, Parvimonas micra, Prevotella nigrescens, Streptococcus constellatus, Campylobacter concisus, Capnocytophaga gingivalis, Capnocytophaga ochracea, Capnocytophaga sputigena, Eikenella corrodens, Streptococcus gordonii, Streptococcus intermedius, Streptococcus mitis* bv. 2, *Actinomyces odontolyticus, Veillonella parvula, Actinomyces naeslundii II* and *Selenomonas noxia.*

11. The method according to either one of Claims 6 and 7, wherein the specific bacterium in the intraoral sample is at least one selected from the group consisting of *Porphyromonas gingivalis, Tannerella forsythia, Treponema denticola, Fusobacterium nucleatum, Campylobacter rectus, Streptococcus constellatus, Streptococcus gordonii, Streptococcus mitis, Streptococcus oralis, Streptococcus sanguinis* and *Veillonella parvula.*

12. The method according to Claim 8, wherein the specific bacterium in the intraoral sample is at least one selected from the group consisting of *Porphyromonas gingivalis, Tannerella forsythia* and *Treponema denticola,* and *Fusobacterium nucleatum.*

13. The method according to Claim 1, wherein the count of bacterium present in the intraoral sample is determined by a method of calculating the bacterial count which comprises the steps (1)-(3) below:
(1) comparing data acquired from a test sample with a signal intensity of an absolute level index probe to correct the data;
(2) for each of pre-isolated bacteria targeted for detection, determining a bacterial count calculation formula from a signal intensity ratio of a probe for detecting the bacterium targeted for detection; and
(3) by using the calculation formula obtained in step (2) above, determining a bacterial count of each bacterial species targeted for detection from the signal intensity corrected in step (1) above.

14. A primer or a primer set comprising one or more of DNAs having the nucleotide sequences represented by SEQ ID NOS:193-198 and 201-204 or nucleotide sequences complementary to said nucleotide sequences.

15. An oligonucleotide probe comprising:
DNA having the nucleotide sequence represented by SEQ ID NO:97, 100, 120, 121, 122, 129, 130, 152 or 155 or a nucleotide sequence complementary to said nucleotide sequence; or
DNA that hybridizes with DNA having a nucleotide sequence complementary to said DNA under stringent conditions, and that has a function of detecting at least a part of a nucleotide sequence of nucleic acids derived from an intraoral bacterium.
